# EUROPEAN PATENT APPLICATION

(11) **EP 2 085 484 A1**
(43) Date of publication of application: **05.08.2009**
(21) Application number: 07831051.3
(22) Date of filing: 01.11.2007
(51) Int. Cl.: C12Q 1/68, C12N 15/09, C12Q 1/02, A01K 67/027, A61P 25/22

(54) **METHOD FOR IDENTIFICATION OF COMPOUND HAVING ANTIANXIETY EFFECT**

(30) Priority: 02.11.2006 JP 2006298814
(71) Applicant: Daiichi Sankyo Company, Limited, Chuo-ku Tokyo 103-0023 (JP); Kazusa DNA Research Institute Foundation, Kisarazu-shi, Chiba 292-0818 (JP)
(72) Inventor: OKAJIMA, Daisuke, Tokyo 134-8630 (JP); YOKOTA, Hiroshi, Tokyo 134-8630 (JP); OHARA, Osamu, Kisarazu-shi Chiba 292-0818 (JP); NAGASE, Takahiro, Kisarazu-shi Chiba 292-0818 (JP); OHISHI, Michio, Kisarazu-shi Chiba 292-0818 (JP)
(74) Representative: Zwicker, Jörk
(86) International application number: PCT/JP2007/071317
(87) International publication number: WO 2008/053962

(57) **Abstract**

There are provided a method for identifying a compound having an anti-anxiety effect, an inhibitor of a function of a protein having a function as a G-protein coupled receptor, **characterized by** finding the protein and a gene encoding the protein and using at least one of a DNA represented by the nucleotide sequence of this gene or a complementary strand thereof, a homologue of the DNA, a protein encoded by the DNA, and a cell expressing the protein encoded by the DNA, an agent and a method for improving an anxiety condition, a method and an agent for prophylactic and/or therapeutic treatment of an anxiety disorder, and a measurement method for diagnosis and a diagnostic method of an anxiety disorder.

## Description

### Technical Field

The present invention relates to a method for identifying a compound having an anti-anxiety effect. More specifically, the present invention relates to a method for identifying a compound having an anti-anxiety effect which is an inhibitor of a function of a protein encoded by a DNA represented by the nucleotide sequence of SEQ ID NO: 1 in the sequence listing or a splicing variant of the protein. Furthermore, the present invention relates to an agent for improving an anxiety condition which contains a compound inhibiting a function and/or the expression of a protein encoded by a DNA represented by the nucleotide sequence of SEQ ID NO: 1 in the sequence listing or a splicing variant of the protein. Furthermore, the present invention relates to a method for improving an anxiety condition, comprising inhibiting a function and/or the expression of a protein encoded by a DNA represented by the nucleotide sequence of SEQ ID NO: 1 in the sequence listing or a splicing variant of the protein. Furthermore, the present invention relates to a prophylactic and/or therapeutic agent of an anxiety disorder containing an effective amount of the above-mentioned agent for improving an anxiety condition. Furthermore, the present invention relates to a method for prophylactic and/or therapeutic treatment of an anxiety disorder, using the above-mentioned agent for improving an anxiety condition or the above-mentioned method for improving an anxiety condition. Furthermore, the present invention relates to a measurement method used for the diagnosis of an anxiety disorder or a method for diagnosing an anxiety disorder, comprising analyzing at least one DNA selected from a DNA represented by the nucleotide sequence of SEQ ID NO: 1 in the sequence listing and a splicing variant of the DNA and/or a protein encoded by the DNA.

### Background Art

Membrane protein receptors existing in the cell membrane serve as important sensor for the cell in an organism and are important target molecules in the development of therapeutic agents for various diseases.

A membrane protein receptor has a domain that passes through the lipid bilayer of a biomembrane, exists in the cell membrane, recognizes specifically various bioactive substances, and transmits and exhibits its action. A bioactive substance that specifically binds to a membrane protein receptor is generally referred to as a ligand. Examples of ligands include peptide hormones, neurotransmitters, growth factors, and so forth. When a ligand binds to a membrane protein receptor, a cellular response is induced via formation of second messengers, changes in intracellular ion concentrations, phosphorylation of proteins, and the like. A series of reactions, in which changes are caused by the binding of a ligand to a membrane protein receptor in the cell, such as formation of second messengers, changes in intracellular ion concentrations, and phosphorylation of proteins, are generally referred to as signal transduction, and the process of the series of reactions is referred to as a signal transduction pathway.

G-protein coupled receptors (hereinafter, may be referred to as GPCRs) are glycoproteins existing in the cell membrane which are one type of seven-transmembrane receptors with a structural characteristic of having seven transmembrane domains and constitute a superfamily having a large number of family members. More than 1000 types of GPCR genes have already been identified, and the three dimensional structures of GPCRs, ligands for GPCRs, intracellular signal transduction pathways via GPCRs, functions of GPCRs, and the like are being studied.

GPCRs are receptors of light, odor, and taste as well as receptors of hormones and neurotransmitters, and serve as major sensors for cells in organisms from yeast to human.

When a GPCR is stimulated by a ligand, it binds to a G-protein existing in the cell. A G-protein is a protein that couples with a GPCR and functions as a signal transduction factor. G-proteins are roughly classified into several types of families depending on the differences in functions against various factors involved in signal transduction in intracellular signal transduction pathways (hereinafter, referred to as effectors) and the gene encoding the protein. A G-protein belonging to any family is a trimer consisting of three subunits called α, β, and γ. Usually, guanosine 5'-diphosphate (GDP) specifically binds to the α-subunit. A GDP-binding G-protein is in the inactive form and does not act on an effector. When a GPCR is stimulated by a ligand, an exchange reaction occurs between GDP that binds to a G-protein and guanosine 5'-triphosphate (GTP) present in the cell, by which GDP is released from the G-protein, then GTP binds to the G-protein, and thus a GTP-binding G-protein is formed. The GTP-binding G-protein is referred to as an active form and rapidly dissociates into the α-subunit bound with GTP (αGTP) and a dimer consisting of the β- and γ-subunits (βγ). αGTP and βγ directly act on effectors (for example, calcium ion channels, potassium ion channels, etc.) to activate intracellular signal transduction pathways, resulting in induction of various cellular responses.

The gene of human brain angiogenesis inhibitor 2 (hereinafter, referred to as hBAI2), which is classified into class B (secretin-like) of the GPCR superfamily, is registered in GenBank under an accession number AB005298.

The sequence information and the expression distributions of hBAI2 have been reported (Non-Patent Document 1). There has been no report on the association between the functions of hBAI2 and diseases, except that the association between the proteins encoded by the hBAI2 gene and genes having high homology with this gene (Patent Document 1) and depression has been reported by the inventors of the present invention (Patent Document 2). Based on comparison with BAI1, a homologue of BAI2, in their structures, it is thought that a thrombospondin type I domain (hereinafter, referred to as TSP-I domain) exists in the extracellular domain of the proteins (Non-Patent Document 2). The TSP-I domain is a characteristic domain observed in a region comprising the 385th to the 522nd amino acids in the amino acid sequence of thrombospondin, and is known to be a functional domain which is important for the involvement of thrombospondin in the extracellular matrix and the neovascularization inhibiting ability thereof.

It has been reported that hBAI1 is highly expressed specifically in human brain tissues and has an extracellular domain having a neovascularization inhibiting ability, the expression thereof may be regulated by p53, and so forth, suggesting that this gene is involved in a mechanism associated with neovascularization in the brain in a certain manner (Non-Patent Documents 1 to 4).

It has been reported that the expression levels of mouse BAI2, an hBAI2-related gene, and a vascular endothelial growth factor (VEGF) negatively correlate to each other in brain tissues of a cerebral ischemia model rat, and it is thought that BAI2 may also be involved in neovascularization as with hBAI1. Specifically, after a hypoxic condition occurred, the expression of BAI2 decreased, and then the expression of VEGF increased. Furthermore, existence of splicing variants of mouse BAI2 has been reported (Non-Patent Document 3).

Meanwhile, cholecystokinin (hereinafter, referred to as CCK) is known to be a gastrointestinal hormone released from endocrine cells in the duodenal mucous membrane. CCK is secreted after the ingestion of fats and promotes gallbladder contraction and pancreatic enzyme secretion. CCK exhibits effects on the digestive organs, such as promotion of gallbladder contraction, pancreas enzyme secretion and stimulation of intestinal motility. Furthermore, CCK is considered to be a signal substance that transmits the feeling of fullness to neurons in the brain.

It is known that the seventh tyrosine residue from the C terminus of CCK is sulfated. Posttranslational processing of CCK produces CCK fragments in several kinds of lengths with different cleavage sites on the N-terminus side, such as CCK-4, CCK-8, CCK-12, CCK-33, and CCK-58. It has been confirmed that these fragments differ in the bioactivity and the abundance. Furthermore, cerulein extracted from a frog skin has been reported as a compound that has a chemical structure similar to that of CCK and exhibits an identical biological activity.

Furthermore, CCK is extensively distributed in brain tissues and is abundant in, for example, the cerebral cortex, the hippocampus, the amygdala, and the hypothalamus. Its actions in the central nervous system, such as involvement in anxiety, analgesia, sedation, suppression of food intake, memory, and learning, have also been reported. It has also been reported that CCK molecules partly coexist with dopamine (DA) and γ-aminobutyric acid (GABA) and interact with serotonin (5-hydroxytryptamine [5HT]) nerve system and the like. Furthermore, it has also been reported that release of CCK is regulated by GABA. CCK-8 and CCK-4 have been reported as the major CCKs which exhibit bioactivity in the brain.
Furthermore, CCK-8 existing in the brain is a cholecystokinin octapeptide sulfated form (CCK-8S), in which the seventh tyrosine residue from the C terminus is sulfated.

In recent years, it has been reported that CCK is essential for memory retention. For example, it has been revealed that it is difficult without CCK-8S to bring a memory back to the consciousness level and take action, that CCK-4 (a tetrapeptide at the C terminus of CCK-33) inhibits memory recall, and so forth.

The CCK-A receptor and the CCK-B receptor have been reported as receptors of CCK. Both of them are G-protein coupled receptors. Expression of the CCK-A receptor has been detected in tissues and cells derived from the gastrointestinal tracts, leukocytes in blood, and the like. The CCK-A receptor is involved in regulation of digestion via promotion of effectors such as, for example, phospholipase C and adenyl cyclase in intracellular signal transduction pathways. Meanwhile, expression of the CCK-B receptor has been detected in tissues and cells derived from the brain and the gastrointestinal tracts, leukocytes in blood, and the like. The CCK-B receptor is also referred to as a gastrin receptor and is involved in regulation of digestion and cell growth via promotion of effectors such as, for example, phospholipase C and intracellular calcium ion influx in intracellular signal transduction pathways. In recent years, the association between the CCK-B receptor and anxiety has been drawing attention.
Patent Document 1: International Publication No. WO2005/49833
Patent Document 2: U.S. Patent Application Publication No. 20060234305
Non-Patent Document 1: Shiratsuchi, T. et al., "Cytogenetics and Cell Genetics," 1997, Vol. 79, p. 103-108
Non-Patent Document 2: Nishimori, H. et al., "Oncogene," 1997, Vol. 15, p. 2145-2150
Non-Patent Document 3: Kee, H. J. et al., "Journal of Cerebral Blood Flow and Metabolism," 2002, Vol. 22, p. 1054-1067
Non-Patent Document 4: Kaur, B. et al., "American Journal of Pathology," 2003, Vol. 162, p. 19-27

### Disclosure of the Invention

### Problems to be Solved by the Invention

An object of the present invention is to find association between a protein having a function as a GPCR and the gene encoding the protein and a disease and provide effective means for the prophylactic and/or therapeutic treatment of the disease. Furthermore, another object of the present invention is to provide a method for identifying a compound to be used in the above-mentioned prophylactic and/or therapeutic treatment of a disease.

### Means for Solving the Problems

The inventors of the present invention conducted various researches to achieve the foregoing objects and found that a protein that appeared to have a function as a GPCR found by the inventors of the present invention, the gene encoding this protein, and splicing variants thereof were associated with an anxiety disorder. Specifically, the inventors of the present invention demonstrated that a knockout mouse deficient in a homologue gene of this gene showed an anti-anxiety phenotype and considered that the gene was also associated with an anxiety disorder in humans. This gene is strongly expressed in brain tissues, in particular, the cerebral cortex, the hippocampus, and the amygdala.

When this gene or a splicing variant thereof is expressed in an animal cell, the protein encoded by the gene was expressed on the cell membrane, cellular responses were induced by a stimulus of a ligand via intracellular signal transduction.

The inventors of the present invention demonstrated that a compound that inhibits a function of the protein encoded by the gene, that is, a cellular response could be identified by using an experimental system in which an animal cell expressing this gene was stimulated by a ligand to induce a cellular response.

The present invention was accomplished on the basis of these findings.

The present invention relates to a method for identifying a compound having an anti-anxiety effect, an inhibitor of a function of a protein encoded by a DNA represented by the nucleotide sequence of SEQ ID NO: 1 in the sequence listing or a homologue protein thereof, characterized by using at least one selected from the group consisting of the DNA represented by the nucleotide sequence of SEQ ID NO: 1 in the sequence listing or a homologue DNA thereof, a protein encoded by the DNA, and a cell containing the DNA.

Furthermore, the present invention relates to a method for identifying a compound having an anti-anxiety effect, an inhibitor of a function of a protein encoded by a DNA represented by the nucleotide sequence of SEQ ID NO: 1 in the sequence listing or a splicing variant of the protein, characterized by using at least one selected from the group consisting of a DNA represented by any one nucleotide sequence of the DNA represented by the nucleotide sequence of SEQ ID NO: 1 in the sequence listing and a splicing variant thereof, a protein encoded by the DNA, and a cell containing the DNA.

Furthermore, the present invention relates to a method for identifying a compound having an anti-anxiety effect, an inhibitor of a function of a protein encoded by a DNA represented by the nucleotide sequence of SEQ ID NO: 1 in the sequence listing or a splicing variant of the protein, comprising bringing a cell containing a DNA represented by any one nucleotide sequence of the nucleotide sequences of SEQ ID NOS: 1, 15, 17, 19, and 21 in the sequence listing into contact with a test compound, measuring a function of a protein expressed on the cell membrane of the cell, and determining a test compound that decreases or eliminates the function of the protein as compared with when the cell and the test compound are not brought into contact with each other as a compound having an anti-anxiety effect.

Furthermore, the present invention relates to the method for identification, wherein the function of the protein expressed on the cell membrane of the cell is a function of inducing an increase in the intracellular calcium concentration by addition of a ligand of the protein.

Furthermore, the present invention relates to a method for identification, wherein the function of the protein expressed on the cell membrane of the cell is a function of inducing a change in the membrane potential by addition of a ligand of the protein.

Furthermore, the present invention relates to a method for identification, wherein the ligand used is a peptide selected from the group of the following:
(i) cholecystokinin octapeptide sulfated form (SEQ ID NO: 14, hereinafter, referred to as CCK-8S);
(ii) a peptide having a mutation such as deletion, substitution, or addition of one or several amino acids in the amino acid sequence of CCK-8S and having a function identical to that of CCK-8S; and
(iii) a peptide comprising the amino acid sequence of the peptide of the (i) or (ii) and having a function identical to that of CCK-8S.

Furthermore, the present invention relates to any one of the above-mentioned methods for the identification, further comprising evaluating an anti-anxiety effect of a compound in an experimental system using a nonhuman animal for measuring the anxiety condition.

Furthermore, the present invention relates to any one of the above-mentioned methods for identification, further comprising evaluating an anti-anxiety effect of a compound using a nonhuman animal in an experimental system selected from the group consisting of fear conditioning tests, Vogel anti-conflict tests, Geller-Seifter anti-conflict tests, elevated maze tests, hole-board tests, light-dark tests, social interaction tests, and marble-burying tests.

Furthermore, the present invention relates to a method for improving an anxiety condition, comprising inhibiting a function and/or the expression of a protein encoded by a DNA represented by the nucleotide sequence of SEQ ID NO: 1 in the sequence listing or a splicing variant of the protein.

Furthermore, the present invention relates to a method for prophylactic and/or therapeutic treatment of an anxiety disorder, comprising using the above-mentioned method for improving an anxiety condition.

Furthermore, the present invention relates to a method for measurement used for diagnosis of an anxiety disorder, comprising a quantitative or qualitative analysis using at least one DNA selected from a DNA represented by the nucleotide sequence of SEQ ID NO: 1 in the sequence listing and a splicing variant of the DNA and/or a protein encoded by the DNA as a marker.

Furthermore, the present invention relates to a method for diagnosing an anxiety disorder, comprising a quantitative or qualitative analysis using at least one DNA selected from a DNA represented by the nucleotide sequence of SEQ ID NO: 1 in the sequence listing and a splicing variant of the DNA and/or a protein encoded by the DNA as a marker.

### Advantages of the Invention

According to the present invention, a method for identifying a compound having an anti-anxiety effect, an inhibitor of a function of a protein acting as a functional membrane protein receptor having a seven-transmembrane domain that appears to be a GPCR, can be provided. Furthermore, according to the present invention, an agent and a method for improving an anxiety condition and a method and an agent for prophylactic and/or therapeutic treatment of an anxiety disorder can be provided. Furthermore, according to the present invention, a method for the measurement to diagnose an anxiety disorder and a diagnostic method can be provided.

### Brief Description of the Drawings

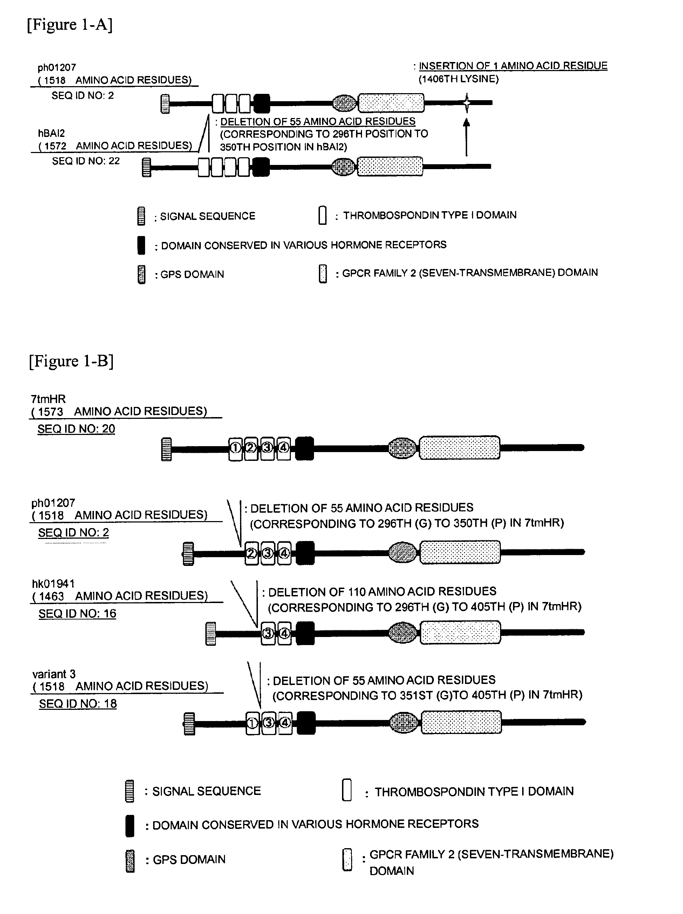

Figure 1-A is a schematic view showing a comparison of the functional domain of a protein represented by the amino acid sequence of SEQ ID NO: 2 with that of the protein encoded by hBAI2 (Example 1);
Figure 1-B is a schematic view showing a comparison of a structural characteristic of a protein represented by the amino acid sequence of SEQ ID NO: 2 with those of splicing variants thereof. The ph01207 represents the protein represented by the amino acid sequence of SEQ ID NO: 2. The 7tmHR, hk01941, and variant 3 are all splicing variants of the protein represented by the amino acid sequence of SEQ ID NO: 2 (Example 6);

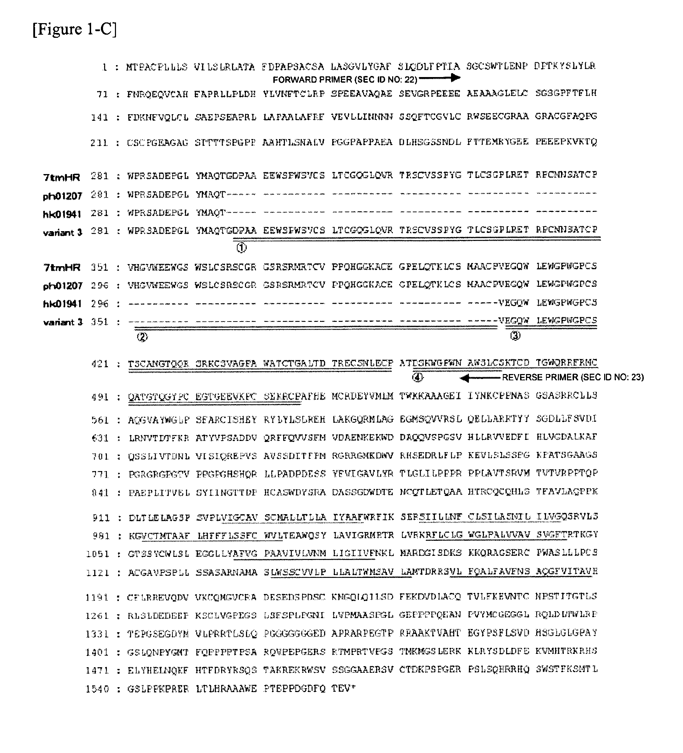

Figure 1-C shows a comparison of the amino acid sequence of the protein represented by the amino acid sequence of SEQ ID NO: 2 with those of splicing variants thereof. In the figure, the double underline represents a thrombospondin type I domain (TSP-I domain), and the underline represents a transmembrane domain. The ph01207 represents the protein represented by the amino acid sequence of SEQ ID NO: 2. The 7tmHR, hk01941, and variant 3 all represent splicing variants of the protein represented by the amino acid sequence of SEQ ID NO: 2 (Example 6);

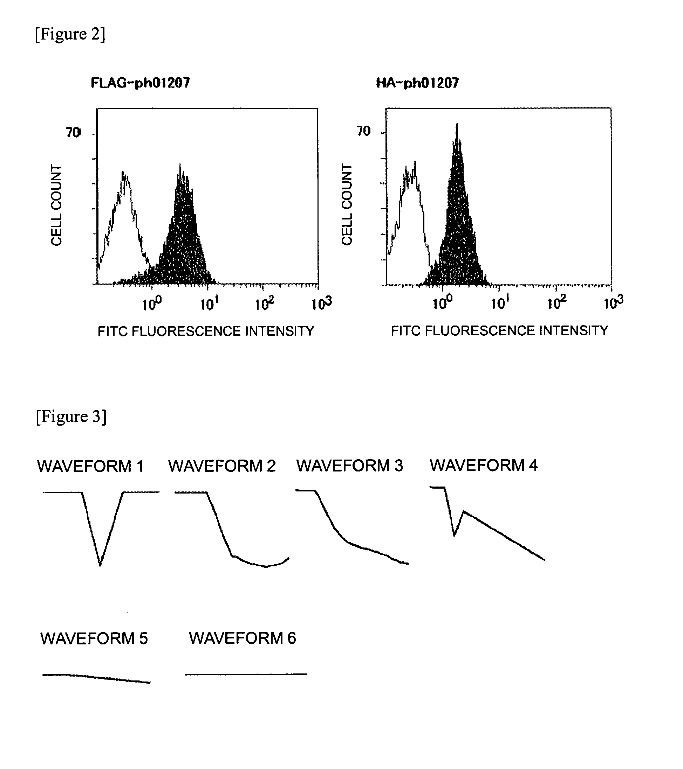

Figure 2 shows that, when cDNA clone ph01207 was expressed in the CHO-K1 cell, an animal cell strain, as a FLAG-tag fusion protein and an HA-tag fusion protein, these proteins were expressed on the cell membrane (a left panel and a right panel, respectively). Detection of the proteins on the cell membrane was analyzed by flow cytometry using an anti-FLAG-tag antibody, an anti-HA-tag antibody, and an FITC-labeled secondary antibody (FITC-anti-mouse IgG antibody). In the figure, the region filled in black represents a cell expressing each tagged fusion protein, and the region open in white represents a control cell in which the protein was not expressed (Example 2);
Figure 3 shows waveforms that can be observed when a response of a GPCR to a ligand is measured by changes in the membrane potential of the cell (a waveform generated by a GPCR-specific response [waveform 1], waveforms generated by an artificial factor or the like [waveforms 2 to 4], and waveforms observed when there is no response [waveforms 5 and 6]) (Example 3);

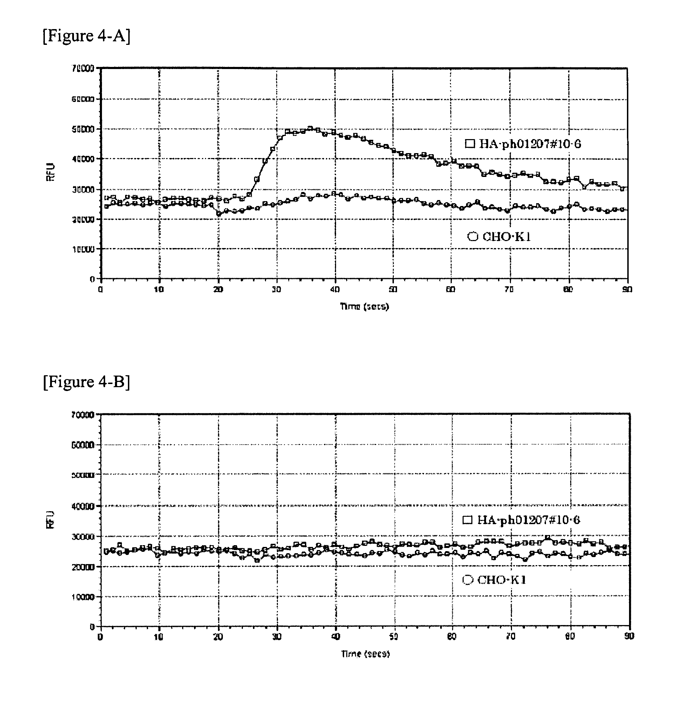

Figure 4-A shows that, in a CHO-K1 cell strain in which cDNA clone ph01207 was stably expressed as an HA-tag fusion protein (HA-ph01207#10-6), the intracellular Ca²⁺ concentration was increased by 1 nM CCK-8S (SEQ ID NO: 14). On the other hand, an increase in the intracellular Ca²⁺ concentration by CCK-8S (SEQ ID NO: 14) was not observed in a CHO-K1 cell strain not transfected with this cDNA. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 5);
Figure 4-B shows that, in a CHO-K1 cell strain in which cDNA clone ph01207 was stably expressed as an HA-tag fusion protein (HA-ph01207#10-6), the intracellular Ca²⁺ concentration was not increased by 1 nM CCK-8NS (CCK-8 nonsulfated form). CCK-8NS is a CCK octapeptide represented by the same amino acid sequence as that of CCK-8S, but the seventh tyrosine residue from the C terminus is not sulfated in this peptide. In a CHO-K1 cell strain not transfected with cDNA clone ph01207, the intracellular Ca²⁺ concentration was not increased by CCK-8NS either. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 5);

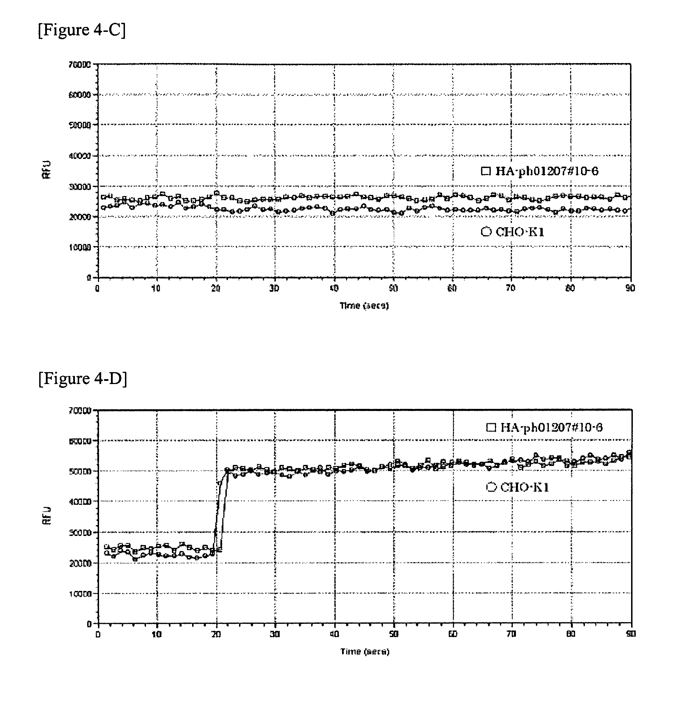

Figure 4-C shows that, in a CHO-K1 cell strain stably expressing cDNA clone ph01207 as an HA-tag fusion protein (HA-ph01207#10-6), the intracellular Ca²⁺ concentration was not increased by 1 nM CCK-4. In a CHO-K1 cell strain not transfected with the cDNA, the intracellular Ca²⁺ concentration was not increased by CCK-4 either. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 5);
Figure 4-D shows that, in a CHO-K1 cell strain stably expressing cDNA clone ph01207 as an HA-tag fusion protein (HA-ph01207#10-6), the intracellular Ca²⁺ concentration was increased by 10 µM calcium ionophore A23187. The intracellular Ca²⁺ concentration was also increased by A23187 in the CHO-K1 cell strain not transfected with the cDNA. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 5);

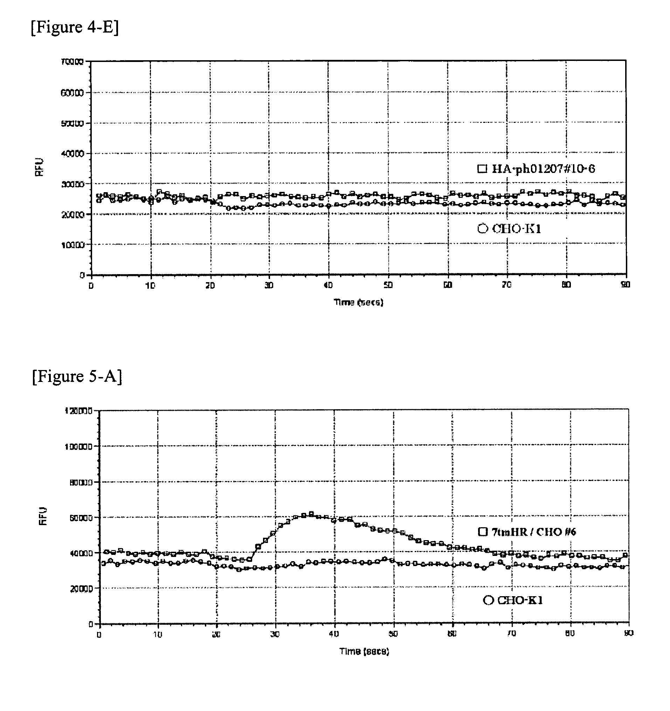

Figure 4-E shows the intracellular Ca²⁺ concentrations after a buffer was added in a CHO-K1 cell strain stably expressing cDNA clone ph01207 as an HA-tag fusion protein (HA-ph01207#10-6) and a CHO-K1 cell strain not transfected with the cDNA. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 5);
Figure 5-A shows representative results where the intracellular Ca²⁺ concentration was increased by 1 nM CCK-8S (SEQ ID NO: 14) in a strain stably expressing 7tmHR. In a host cell not transfected with a 7tmHR expressing vector, the intracellular Ca²⁺ concentration was not increased by CCK-8S (SEQ ID NO: 14). In the figure, 7tmHR/CHO#6 means a clone of the strain stably expressing 7tmHR, and CHO-K1 means a host cell. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 8);

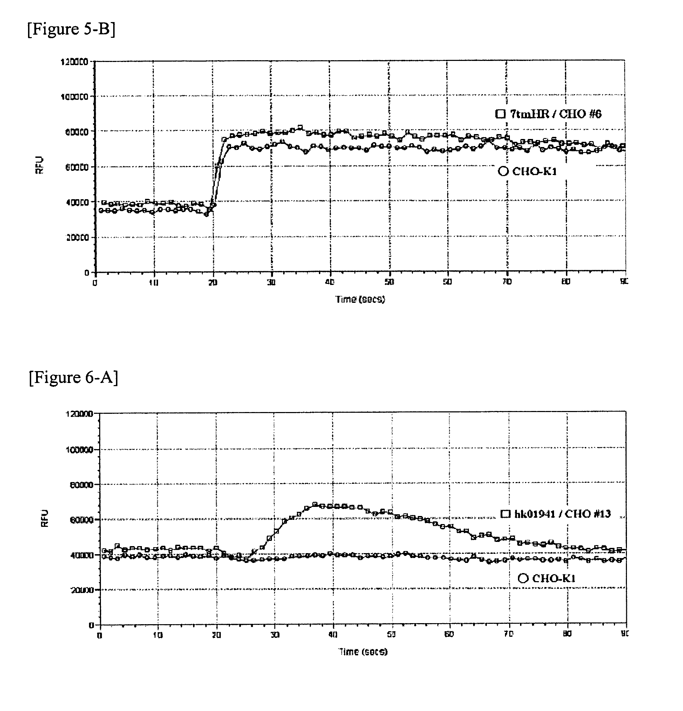

Figure 5-B shows that the intracellular Ca²⁺ concentration was increased by 20 µM calcium ionophore A23187 in a strain stably expressing 7tmHR. In a host cell not transfected with a 7tmHR expressing vector, the intracellular Ca²⁺ concentration was also increased by A23187. In the figure, 7tmHR/CHO#6 means a clone of the strain stably expressing 7tmHR, and CHO-K1 means a host cell. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 8);
Figure 6-A shows representative results where the intracellular Ca²⁺ concentration was increased by 1 nM CCK-8S (SEQ ID NO: 14) in a strain stably expressing hk01941. In a host cell not transfected with an hk01941 expressing vector, the intracellular Ca²⁺ concentration was not increased by CCK-8S (SEQ ID NO: 14). In the figure, hk01941/CHO#13 means a clone of the strain stably expressing hk01941, and CHO-K1 means a host cell. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 8);

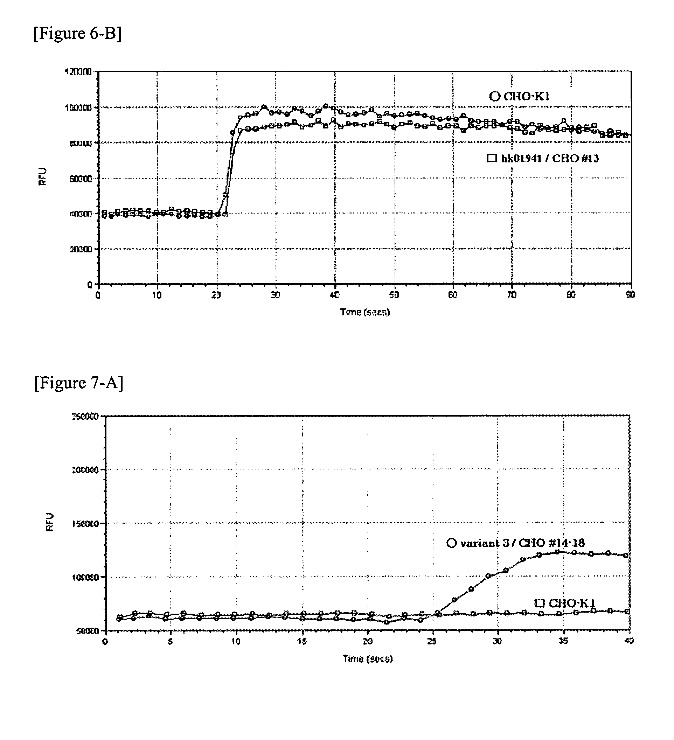

Figure 6-B shows that the intracellular Ca²⁺ concentration was increased by 20 µM calcium ionophore A23187 in a strain stably expressing hk01941. In a host cell not transfected with an hk01941 expressing vector, the intracellular Ca²⁺ concentration was also increased by A23187. In the figure, hk01941/CHO#13 means a clone of the strain stably expressing hk01941, and CHO-K1 means a host cell. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 8);
Figure 7-A shows representative results where the intracellular Ca²⁺ concentration was increased by 1 nM CCK-8S (SEQ ID NO: 14) in a strain stably expressing variant 3. In a host cell not transfected with a variant 3 expressing vector, the intracellular Ca²⁺ concentration was not increased by CCK-8S (SEQ ID NO: 14). In the figure, variant3/CHO#14-18 means a clone of the strain stably expressing variant 3, and CHO-K1 means a host cell. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 9);

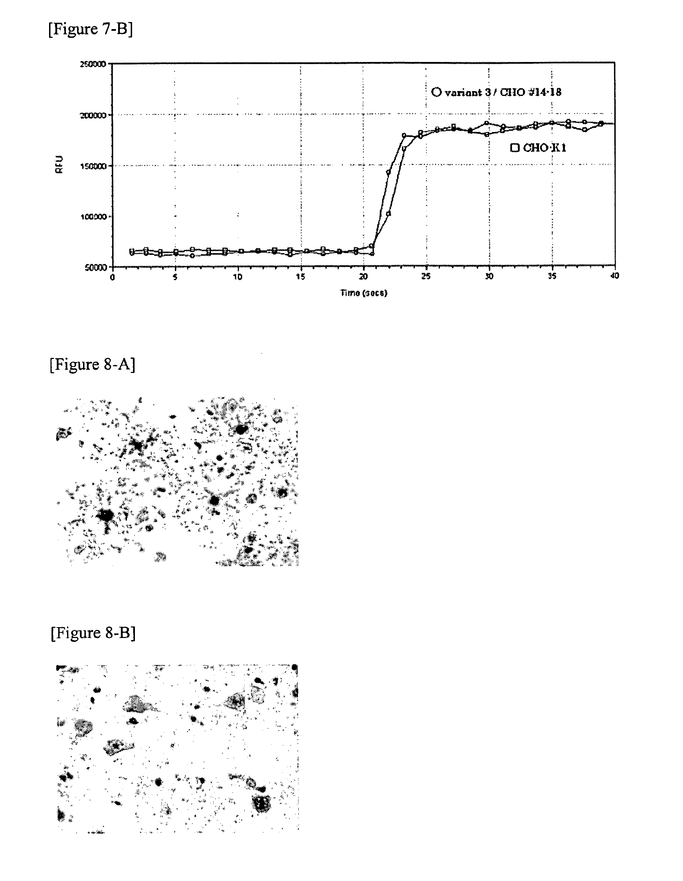

Figure 7-B shows that the intracellular Ca²⁺ concentration was increased by 20 µM calcium ionophore A23187 in a strain stably expressing variant 3. In a host cell not transfected with a variant 3 expressing vector, the intracellular Ca²⁺ concentration was also increased by A23187. In the figure, variant3/CHO#14-18 means a clone of the strain stably expressing variant 3, and CHO-K1 means a host cell. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 9);
Figure 8-A is data of an analysis by tissue immunostaining using an anti-human BAI2 polyclonal antibody showing that the ph01207 gene was strongly expressed in a protoplasmic astrocyte of the human amygdala (Example 10);
Figure 8-B is data of an analysis by tissue immunostaining using an anti-human BAI2 polyclonal antibody showing that the ph01207 gene was strongly expressed in neurons and glia of the human amygdala (Example 10);

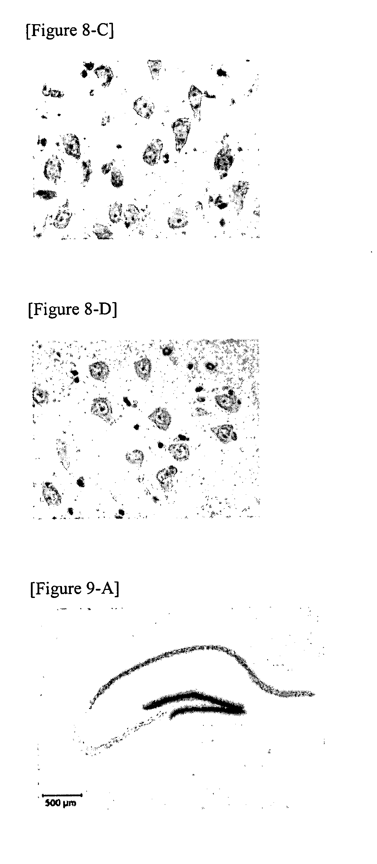

Figure 8-C is data of an analysis by tissue immunostaining using an anti-human BAI2 polyclonal antibody showing that the ph01207 gene was strongly expressed in neurons of the hippocampus CA2 region (Example 10);
Figure 8-D is data of an analysis by tissue immunostaining using an anti-human BAI2 polyclonal antibody showing that the ph01207 gene was strongly expressed in neurons of the hippocampus CA1 region (Example 10);
Figure 9-A shows the result of detection by a LacZ expression analysis showing that the LacZ-Neo gene contained in a targeting vector was strongly expressed in the hippocampus of an F1 heterozygous mutant mouse prepared by using ES cells into which the vector for targeting the mouse BAI2 gene was introduced. This result shows that the LacZ-Neo fragment was inserted at the target site of the mouse BAI2 gene, that is, the gene was disrupted, and the mouse BAI2 gene was expressed in the hippocampus (Example 11);

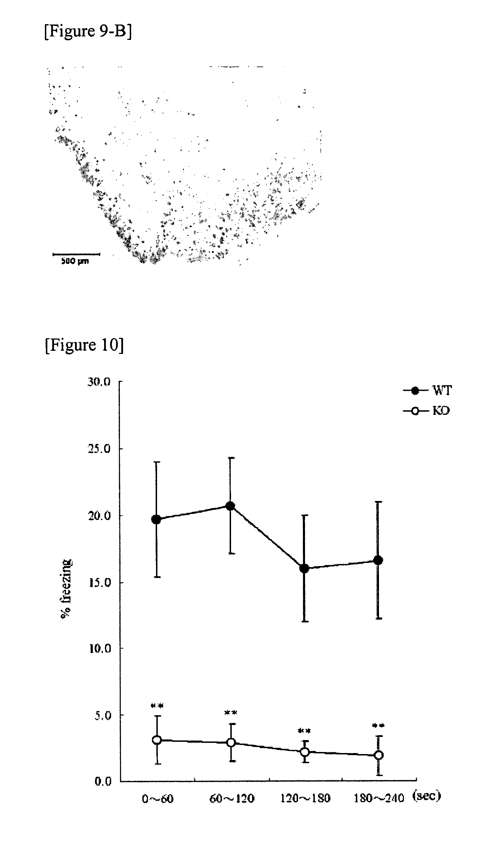

Figure 9-B shows the result of detection by a LacZ expression analysis showing that the LacZ-Neo gene contained in a targeting vector was strongly expressed in the amygdala of an F1 heterozygous mutant mouse prepared by using ES cells into which the vector for targeting the mouse BAI2 gene was introduced. This result shows that the LacZ-Neo fragment was inserted at the target site of the mouse BAI2 gene, that is, the gene was disrupted, and the mouse BAI2 gene was expressed in the amygdala (Example 11);
Figure 10 shows immobility time (% freezing) in a fear conditioning test using BAI2-knockout mice and wild-type mice. In the figure, black circles represent the results of the wild-type mice (expressed as WT), and white circles represent those of the BAI2-knockout mice (expressed as KO). The fear conditioning test was performed using 10 each of wild-type mice and BAI2-knockout mice. The results are shown with proportion (%) of immobility time in a certain measurement time (horizontal axis) ± standard error. In the figure, an asterisk shows that statistical processing by t-test showed a significant difference (P < 0.01) (Example 11);

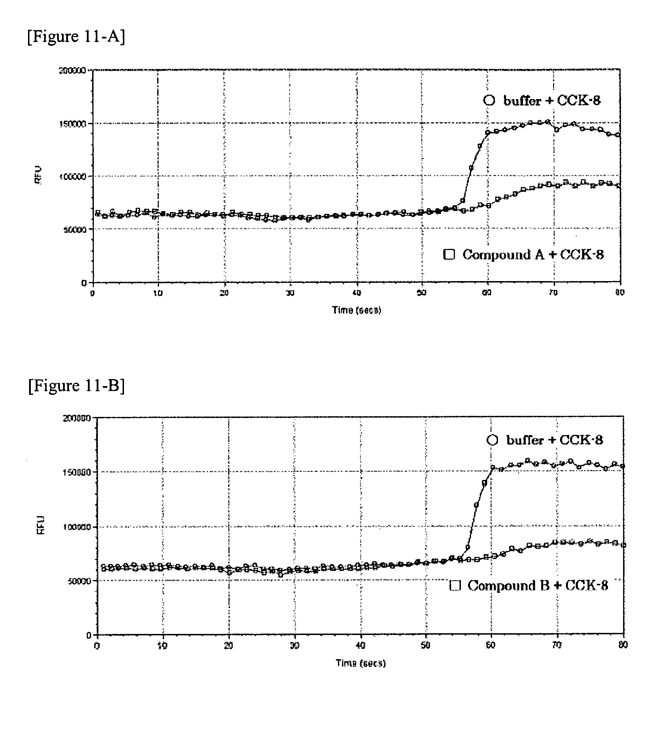

Figure 11-A shows that an increase in the intracellular Ca²⁺ concentration by 10 nM CCK-8S was inhibited by the addition of 10 µg/mL compound A in a CHO-K1 cell strain stably expressing cDNA clone ph01207. The buffer was added instead of compound A as a control. Compound A or the buffer was added at 15 sec after the start of measurement, and CCK-8S was added at 35 sec after the addition of compound A or the buffer. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 12);
Figure 11-B shows that an increase in the intracellular Ca²⁺ concentration by 10 nM CCK-8S was inhibited by the addition of 10 µg/mL compound B in a CHO-K1 cell strain stably expressing cDNA clone ph01207. The buffer was added instead of compound B as a control. Compound B or the buffer was added at 15 sec after the start of measurement, and CCK-8S was added at 35 sec after the addition of compound B or the buffer. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 12); and

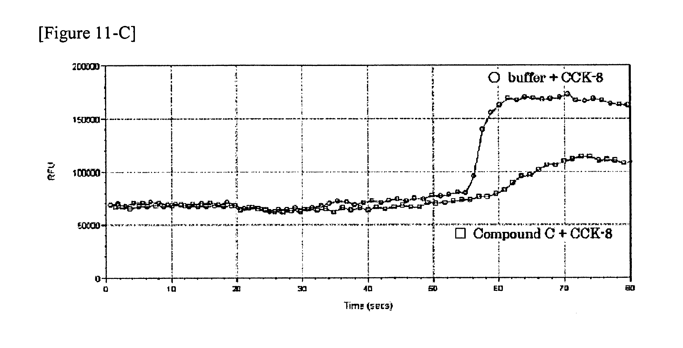

Figure 11-C shows that an increase in the intracellular Ca²⁺ concentration by 10 nM CCK-8S was inhibited by the addition of 10 µg/mL compound C in a CHO-K1 cell strain stably expressing cDNA clone ph01207. The buffer was added instead of compound C as a control. Compound C or the buffer was added at 15 sec after the start of measurement, and CCK-8S was added at 35 sec after the addition of compound C or the buffer. The horizontal axis represents time after the start of measurement of the intracellular Ca²⁺ concentration (Time [sec]). The vertical axis represents the fluorescence intensity (RFU) of a fluorescent dye reflecting the Ca²⁺ concentration (Example 12).

### Best Mode for Carrying Out the Invention

In the present specification, a term "protein" may be used as a generic term meaning an isolated or synthesized full-length protein, an isolated or synthesized full-length polypeptide, and an isolated or synthesized full-length oligopeptide. Here, the minimum size of a protein, a polypeptide, or an oligopeptide is two amino acids. Hereinafter, an amino acid may be expressed with one or three letters.

Proteins functioning as a functional membrane protein receptor and having a seven-transmembrane domain that appears to be a G-protein coupled receptor are proteins encoded by the DNA and splicing variants thereof found by the inventors of the present invention (Patent Documents 1 and 2). The proteins encoded by this DNA and splicing variants thereof were expressed on the cell membrane in an animal cell and induced cellular responses by a ligand stimulus via intracellular signal transduction.

A knockout mouse deficient in a homologue gene of the gene encoding this protein showed an anti-anxiety phenotype. This finding suggests that the gene product of this homologue gene is associated with an anxiety condition in mice.

The inventors of the present invention consider that the gene encoding this protein and splicing variants thereof are associated with an anxiety condition and an anxiety disorder in humans. Specifically, it can be considered that increases in the expression and a function of this gene are associated with an anxiety condition and an anxiety disorder. Furthermore, strong expression of this gene is observed in brain tissues, in particular, the cerebral cortex, the hippocampus, and the amygdala.

Since it can be considered that increases in the expression and stimulations in a function of this gene are associated with an anxiety condition and an anxiety disorder, the inventors of the present invention consider that an inhibitor of a function of a protein encoded by this gene has an anti-anxiety effect.

In the present specification, the "inhibitor of a function of a protein" means a compound having an effect of inhibiting a function of the protein. Here, the compound is not particularly limited so long as it inhibits the function of the protein, and examples thereof include low-molecular compounds and nucleic acid molecules such as siRNA. Since this protein functions as a membrane protein receptor, examples of functions of this protein include binding with a ligand, activation of an intracellular signal transduction mechanism, and induction of a cellular response. Therefore, the "inhibitor of a function of this protein" includes compounds having an effect of inhibiting a function of this membrane protein receptor. Specific examples of the "inhibitor of a function of this protein " include antagonists, compounds acting on a domain of a receptor protein to inhibit the activation of an intracellular signal transduction mechanism, inhibitors of protein expression, and so forth. The "antagonists" are not particularly limited so long as they are compounds that inhibit a function of a receptor protein, and examples thereof include compounds that bind to a receptor to inhibit an effect of an agonist but cannot exhibit an effect by itself that an agonist can do by binding to the receptor, compounds that inhibit the binding of a ligand to a receptor, and compounds acting as an inverse agonist on a receptor. In recent years, it has been known that receptors such as GPCRs are converted from the active form to the inactive form or from the inactive form to the active form irrespective of the action of a ligand. Here, a substance inhibiting a process in which receptors such as GPCRs are converted from the inactive form to the active form irrespective of the action of a ligand is referred to as an inverse agonist. The inverse agonist of this protein is also considered to inhibit a function of this protein. The "compounds acting on a domain of a receptor protein to inhibit the activation of an intracellular signal transduction mechanism" means, for example, compounds that act on any of domains such as transmembrane domains, intracellular domains, and MAGUK interaction sites to inhibit downstream intracellular signal transduction, thus inhibiting a function of the protein. The "inhibitors of protein expression" means compounds that inhibit at least one of various reactions that occur in a process in which the genetic information of a DNA encoding the protein transcribed to mRNA or a process in which the genetic information is transcribed to mRNA and translated as an amino acid sequence of a protein to inhibit the production of the protein by transcription and translation of the gene encoding the protein. Since compounds that inhibit expression of a protein can inhibit a function of the protein by inhibiting the expression thereof, they are included in the inhibitors of a function of a protein in the present specification.

The "anti-anxiety effect" means an effect of improving an anxiety condition.

The "anxiety condition" means a condition in which anxiety occurs in excess. The "anxiety" in psychiatric medicine refers to a "state of mind feeling discomfort with no apparent identifiable cause." The condition in which anxiety occurs in excess means a condition in which various physiological, physical, and/or mental symptoms induced by anxiety causes problems in social life and daily life. Examples of physical symptoms include a number of symptoms such as palpitations, shortness of breath, dizziness, finger tremor, diaphoresis, nausea, vomiting, chest pain or discomfort, chills or hot flash, numbness or twinge, and diarrhea. Furthermore, examples of psychiatric symptoms include terror, tension, anxiousness, fear, unrest, impatience, anguish, excitement, and so forth.

The "improving an anxiety condition" means that an anxiety condition is relieved or disappears as compared with a condition before a method for improving an anxiety condition is implemented. Examples thereof include relief or disappearance of a number of physical symptoms such as palpitations, shortness of breath, dizziness, finger tremor, diaphoresis, nausea, vomiting, chest pain or discomfort, chills or hot flash, numbness or twinge, and diarrhea and psychiatric symptoms such as phobia, tension, anxiousness, fear, unrest, impatience, anguish, and excitement.

A medicament having an effect of improving an anxiety condition may be referred to as "agent for improving an anxiety condition" or "anxiolytic agent" in the present specification.

An "anxiety disorder" is a neurosis with anxiety as the principal symptom, and overactivity of the autonomic nerve system due to excessive anxiety occurs and causes a variety of physiological, physical, and mental symptoms. An anxiety disorder presents with physical symptoms including a plurality of symptoms such as palpitations, shortness of breath, dizziness, finger tremor, diaphoresis, nausea, vomiting, chest pain or discomfort, chills or hot flash, numbness or twinge, and diarrhea. Furthermore, an anxiety disorder presents with psychiatric symptoms such as phobia, tension, anxiousness, fear, unrest, impatience, anguish, and excitement.

Anxiety disorders are classified into generalized anxiety disorder, panic disorder, obsessive-compulsive disorder, posttraumatic stress disorder, social fear, and so forth according to the symptoms and the characteristics.

Generalized anxiety disorder presents with excessive and extremely unrealistic anxiousness that persists for at least six months and is associated with symptoms such as mytonia, fatigue, difficulty in concentrating, restlessness, feeling irritated, irritability, and sleep disorder. Symptoms observed in generalized anxiety disorder may be observed in other anxiety disorders such as, for example, panic disorder, obsessive-compulsive disorder, posttraumatic stress disorder, and social anxiety disorder.

Panic disorder presents with strong fear and discomfort which can be clearly distinguished from other conditions, for example, derealization and fear of death (referred to as panic attack) paraxysmally, repeatedly, and suddenly. The bout is associated with palpitations, tachycardia, diaphoresis, shortness of breath, sensation of suffocation, chest pain, nausea, dizziness, paresthesia, sensory paralysis, twinge, and the like. The bout suddenly occurs, lasts for approx. 10 to 20 min, and disappears in approx. 30 min to 1 h. However, the frequency and the severity of bouts vary depending on the individual. Furthermore, anticipatory anxiety, that is, anxiety that a similar bout may occur again is formed, and it may become difficult for a patient to go out. Such a case is referred to as agoraphobia. Diagnosis is made based on criteria such as the bout not being due to a substance or a physical disease such as, for example, hyperthyroidism, and not accompanying other psychiatric disorders.

Obsessive-compulsive disorder is characterized by presenting with either obsession or a compulsive act. Obsession is a recurrent thought or impulse. A compulsive act is an act in concert with obsession, which is a recurrent behavior or a mental activity that cannot be resisted to do so.

Posttraumatic stress disorder develops by experiencing a traumatic incidence such as a physical injury or a phobia for physical injury, and presents with the following symptoms: persistent re-experience by memory, dream, flashback, and the like of the traumatic incidence; persistent avoidance of a stimulus associated with the traumatic incidence, for example, forgetting of the incidence itself and avoidance of a specific activity; paralysis of overall responsiveness; increased arousal such as insomnia, irritability, and loss of concentration; and persistence of the disorder for at least one month.

Social anxiety disorder presents with physical symptoms such as blush, diaphoresis, shakes, chills, muscular tension, quivering voice, thirst, pollakiuria, and palpitations resulting from having excessive fear about social situations such as being stared at by others, being evaluated by others, meeting a stranger, and taking any action.

Anxiety disorders may coexist with other psychiatric disorders. For example, there are cases where depression, somatoform disorder (autonomic dystonia), or schizophrenia and an anxiety disorder coexist, the disorder progresses from an anxiety disorder to depression, and so forth. Furthermore, alcohol withdrawal may accompany an anxiety condition.

Anxiety disorders are clinically distinguished from other psychiatric disorders such as depression. For example, in the Diagnostic and Statistical Manual of Mental Disorders (DMS) published by the American Psychiatric Association, the International Statistical Classification of Diseases and Related Health Problems (IDC), and the like, anxiety disorders are classified into a category separately from other psychiatric disorders such as depression. In fact, while anxiety disorders show some symptoms similar to those of depression, many symptoms are different.

"Depression" is also referred to as depressive disorder, and is an emotional psychiatric disorder presenting with emotional disorders such as grief, thought disorders such as inhibition of thought, hypobulia, behavioral suppression, sleep disorder, circadian change of a depressed state, and the like as prominent symptoms. Furthermore, it is considered that its onset involves biological factors, psychological factors, and social and environmental factors. The term "depressed state" means presence of symptoms commonly noted in depression such as, for example, emotional disorders such as grief, thought disorders such as inhibition of thought, hypobulia, behavioral suppression, and sleep disorder.

Depression is classified into "bipolar disorder" and "depressive disorder." Bipolar disorder is characterized by repeated episodes with markedly disturbed mood and active level, including mood elevation and increased energy and activity (manic state) and mood depression and decreased energy and activity (depressed state). It is rare that only the manic state is repeated, and the manic state and the depressed state are present. In female patients, however, the depressed state is often predominant. Furthermore, bipolar disorder is a highly recurrent disease, and not only pathological outcome but also social outcome become poor with recurrence. In particular, the depressed state may be associated with suicide attempt. As etiology of depressive disorder, deficiency in serotonin and noradrenalin, which are neurotransmitters in the brain, has been a promising hypothesis. Recently, however, depression is considered to occur due to inadequate neuron junction in the brain regions responsible for emotions. Life events such as loss of employment and bereavement of a close family and chronic social stress are thought to be depression inducing factors.

Therapeutic agents used for anxiety disorders are generally different from therapeutic agents for depression. For example, anxiolytic agents such as benzodiazepine anxiolytic agents, thienodiazepine anxiolytic agents, piperazine anxiolytic agents, and serotonergic anxiolytic agents are used for the treatment of anxiety disorders. In addition to treatment of anxiety disorders, the anxiolytic agents may be used for prophylactic treatment of conditions associated with an anxiety condition, such as somatoform disorder (autonomic dystonia), schizophrenia, depression, and alcohol withdrawal (abstinence) symptoms, to resolve the anxiety condition. On the other hand, drugs promoting actions of neurotransmitters are used for the treatment of depression, and examples thereof include tricyclic antidepressants, tetracyclic antidepressants, triazolopyridine antidepressants, selective serotonin reuptake inhibitors (SSRI), and serotonin noradrenalin reuptake inhibitors (SNRI). SSRI and SNRI may be used for the treatment of an anxiety disorder.

The "membrane protein receptor" means a protein comprising a protein having domains that pass through the lipid bilayer of a biomembrane, exists in the cell membrane, specifically recognizes various bioactive substances, and transmits and exhibits their actions. Here, the protein includes a glycoprotein. The "functional membrane protein receptor" means a membrane protein receptor having a function of inducing cellular responses via intracellular signal transduction by the action of a ligand. The membrane protein receptor has an extracellular domain that interacts with a ligand, a domain that passes through the lipid bilayer of a biomembrane, and an intracellular domain that mediates intracellular signal transduction.

The "G-protein coupled receptor (GPCR)" means a membrane protein receptor that receives a stimulus from a ligand, and then binds to a G-protein existed in the cell, and activates the G-protein. The "G-protein" means a protein that is coupled with a GPCR and converted from a GDP-binding G-protein to a GTP-binding G-protein by a GDP/GTP exchange reaction, and induces various cellular responses as an intracellular signal transduction factor. To "activate a G-protein" means that conversion from the GDP-binding G-protein to the GTP-binding G-protein is induced and/or promoted by inducing and/or promoting a GDP/GTP exchange reaction, resulting in an induction and/or promotion of various cellular responses involving the GPCR coupled with G-protein.

The "ligand" means a bioactive substance that interacts specifically with a membrane protein receptor.

The "interaction" means, for example, that two identical or different proteins specifically act on each other, resulting in a change, for example, an increase or a decrease, of a function of one or both of the proteins. The "to specifically act" means to act more selectively as compared with proteins other than the proteins involved in the action. Examples of interactions include binding of two different types of proteins, activation of one protein by the other protein, and so forth.

The "intracellular signal transduction" means a series of reactions induced by an action of a ligand on a receptor, including formation of second messengers in a cell, changes in intracellular ion concentrations, phosphorylation of proteins, and so forth, and the "intracellular signal transduction pathway" means the process of the series of reactions.

The method for identification according to the present invention can be implemented by using at least one selected from the group consisting of the DNA found by the inventors of the present invention or a homologue DNA thereof, a protein encoded by the DNA, and a cell containing the DNA.

The DNA used in the present invention is more specifically the DNA represented by the nucleotide sequence of SEQ ID NO: 1 or a homologue DNA thereof. In the present specification, the "homologue DNA" means a DNA having sequence homology with the target DNA and encoding a protein having similarity in structural characteristics and biological functions, and the like to those of the protein encoded by the DNA.

The protein used in the present invention is preferably a protein encoded by the DNA represented by the nucleotide sequence of SEQ ID NO: 1 or a homologue protein thereof. In the present specification, the "homologue protein" means a protein having sequence homology with the target protein and similarity in structural characteristics and biological functions and the like.

The cell used in the present invention is more specifically a cell containing the DNA represented by the nucleotide sequence of SEQ ID NO: 1 or a homologue DNA thereof. It is preferable to use a cell in which the expression of the DNA represented by the nucleotide sequence of SEQ ID NO: 1 or a homologue DNA thereof and/or a protein encoded by the DNA has been confirmed. As such a cell, a eukaryotic cell or a cultured cell strain in which the expression of this DNA and/or this protein has been confirmed can be used. Furthermore, a cell in which the protein is expressed by transfecting the gene encoding this protein with a suitable eukaryotic cell or cultured cell can also be used.

In the present invention, DNAs and proteins are preferably derived from humans, but can be mammal-derived DNAs and proteins having functions identical to those of the human-derived DNAs and proteins as well as structural homology, for example, DNAs and proteins of mouse, horse, sheep, bovine, dog, monkey, cat, bear, rat, rabbit, and the like.

The DNA represented by the nucleotide sequence of SEQ ID NO: 1 is a DNA encoding a protein that functions as a functional membrane protein receptor having a seven-transmembrane domain. More specifically, a preferred example of the protein encoded by the DNA represented by the nucleotide sequence of SEQ ID NO: 1 is a protein represented by the amino acid sequence of SEQ ID NO: 2 in the sequence listing.

The protein encoded by DNA represented by the nucleotide sequence of SEQ ID NO: 1 has three TSP-I domains, one GPCR proteolytic site (GPS) domain, and one seven-transmembrane domain as structural characteristics (see Figures 1-A and 1-B). The seven-transmembrane domain is also referred to as the GPCR family 2 domain and has a structure characteristic to a G-protein coupled receptor as with the GPS domain. The TSP-I domain is a characteristic domain observed in thrombospondin and is known to be a functional domain that is important for involvement of thrombospondin in the extracellular matrix and an angiogenesis inhibiting ability thereof.

In fact, the products of the gene of the DNA represented by the nucleotide sequence of SEQ ID NO: 1 exhibited a function as a membrane protein receptor. Specifically, it was observed in an animal cell into which the DNA represented by the nucleotide sequence of SEQ ID NO: 1 has been introduced that the protein encoded by the DNA was expressed on the cell membrane, and cellular responses were induced by a stimulus of a ligand, for example, a stimulus of CCK-8S (SEQ ID NO: 14) via intracellular signal transduction.

Furthermore, it was observed that the products of the gene of the DNA represented by the nucleotide sequence of SEQ ID NO: 1 interacted with DLG2, DLG3, DLG4, AIP1, and MAGI3, which are MAGUK family-related proteins, and the like in the C-terminal region thereof. MAGUK family-related proteins have the PDZ domain, which recognizes the amino acid sequence closest to the C terminus of a target protein in interactions between proteins, and is considered to be involved in signal transduction from these membrane proteins and contribute to cell adhesion and the like by being localized in the cell membrane and interacting with membrane proteins such as receptors and ion channels. Furthermore, interactions between the gene product of the hBAI2 gene having sequence homology with the DNA represented by the nucleotide sequence of SEQ ID NO: 1 and MAGUK family-related proteins have also been similarly observed. Meanwhile, it has been reported that hBAII, a homologue of hBAI2, binds to BAI1-associated protein 1 (BAP1), one of the MAGUK family-related proteins, via a partial sequence of the terminal region of hBAI1 (QTEV, SEQ ID NO: 3) (Shiratsuchi, T. et al., "Biochemical and Biophysical Research Communications," 1998, Vol. 247, p. 597-604).

The inventors of the present invention consider that, since the amino acid sequence of SEQ ID NO: 3 (QTEV) is conserved at the C-terminal region in both the protein encoded by the DNA represented by the nucleotide sequence of SEQ ID NO: 1 and the protein encoded by the hBAI2 gene (SEQ ID NO: 21) having sequence homology with this gene, these proteins interact with a protein having the PDZ domain in this sequence region.

A homologue DNA of the DNA represented by the nucleotide sequence of SEQ ID NO: 1 preferably has sequence homology with the DNA represented by the nucleotide sequence of SEQ ID NO: 1, and is a DNA encoding a protein exhibiting functions identical to those of a GPCR by the action of a ligand.

Preferred examples of the homologue DNA of the DNA represented by the nucleotide sequence of SEQ ID NO: 1 are splicing variants of the DNA represented by the nucleotide sequence of SEQ ID NO: 1.

Preferred examples of the protein having similarity in structural characteristics, biological functions, and the like to those of the protein encoded by the DNA represented by the nucleotide sequence of SEQ ID NO: 1 are splicing variants of the protein encoded by the DNA represented by the nucleotide sequence of SEQ ID NO: 1.

The "splicing variant" means two or more types of mature mRNAs generated by selective splicing of the mRNA precursor of a gene transcribed from the genome, DNA complementary to the mature mRNA, or a protein translated from this mature mRNA in gene expression in eukaryotes. Genes are expressed in eukaryotes by forming a mature mRNA by splicing from an mRNA precursor transcribed from a region comprising exons dispersed on the genome and introns existing between exons. Further, a protein is translated from the mature mRNA. Splicing means a process in which introns are excised from an mRNA precursor at splice sites (borders between introns and exons) to form a mature mRNA. At the time of splicing, positions and combinations of splice sites may be changed, resulting in generation of two or more types of mature mRNAs, so-called selective splicing. As a result of selective splicing, two or more types of proteins are often generated from one gene.

Splicing variants of the DNA represented by the nucleotide sequence of SEQ ID NO: 1 can be two more types of mature mRNAs generated by selective splicing of mRNA precursor transcribed from the genome of the gene comprising the DNA or a DNA complementary to the mature mRNA.

A preferred example of the splicing variants of the DNA represented by the nucleotide sequence of SEQ ID NO: 1 is a DNA represented by any one nucleotide sequence of the nucleotide sequences of SEQ ID NOS: 15, 17, 19, and 21.

The DNA represented by the nucleotide sequence of SEQ ID NO: 19 encode a protein having the longest amino acid sequence among the proteins encoded by any one of DNAs represented by the nucleotide sequences of SEQ ID NOS: 1, 15, 17, 19, and 21.

Splicing variants of the DNA represented by the nucleotide sequence of SEQ ID NO: 1 are not limited to the above-mentioned splicing variants, and any DNA can be included so long as it is a DNA encoding a protein having sequence homology with the DNA, structural characteristics of the DNA, and, further, biological functions identical to those of the protein encoded by the DNA.

Splicing variants of the protein encoded by the DNA represented by the nucleotide sequence of SEQ ID NO: 1 are proteins translated from two or more types of mature mRNAs generated by selective splicing of the mRNA precursor of the gene encoding the protein transcribed from the genome.

A preferred example of splicing variants of the protein encoded by the DNA represented by the nucleotide sequence of SEQ ID NO: 1 is a protein encoded by a DNA represented by any one nucleotide sequence of the nucleotide sequences of SEQ ID NOS: 15, 17, 19, and 21.

A preferred example of the protein encoded by a DNA represented by any one nucleotide sequence of the nucleotide sequences of SEQ ID NOS: 15, 17, 19, and 21 is a protein represented by any one amino acid sequence of the amino acid sequences of SEQ ID NOS: 16, 18, 20, and 22.

The protein represented by the amino acid sequence of SEQ ID NO: 20 is a protein represented by the longest amino acid sequence among the proteins having the amino acid sequences of SEQ ID NO: 2, 16, 18, 20, and 22.

The splicing variants of the protein encoded by the DNA represented by the nucleotide sequence of SEQ ID NO: 1 are not limited to the above-mentioned splicing variants, and any protein is included so long as it is a protein having sequence homology with the protein, structural characteristics of the protein, and, further, biological functions identical to those of the protein encoded by the DNA.

In other words, the splicing variants of the protein encoded by the DNA represented by the nucleotide sequence of SEQ ID NO: 1 include any protein so long as it is a protein having sequence homology with the protein represented by the amino acid sequence of SEQ ID NO: 2, structural characteristics of the protein, and, further, biological functions identical to those of the protein.

In the present specification, it is usually appropriate to have "sequence homology" of 50% or more, preferably at least 70% of the entire nucleotide sequence or amino acid sequence. More preferably, it is appropriate to have sequence homology of 70% or more, 75% or more, 80% or more, 85% or more, 90% or more, or 95% or more.

DNAs having sequence homology with the DNA represented by the nucleotide sequence of SEQ ID NO: 1 include a DNA having a nucleotide sequence comprising a mutation such as deletion, substitution, addition, or insertion of one or more, for example, one to 100, preferably one to 30, more preferably one to 20, yet more preferably one to 10, yet more preferably one to several nucleotides in the nucleotide sequence of the DNA represented by the nucleotide sequence of SEQ ID NO: 1. Preferably, such a DNA is a DNA encoding a protein having functions identical to biological functions of the DNA represented by the nucleotide sequence of SEQ ID NO: 1. The extent, the position, and the like of a mutation are not particularly limited so long as the DNA including the mutation has structural characteristics similar to those of the DNA represented by the nucleotide sequence of SEQ ID NO: 1 and biological functions identical to those of the protein encoded by the DNA represented by the nucleotide sequence of SEQ ID NO: 1.

A DNA comprising a mutation may be naturally occurring or obtained by introducing a mutation based on the naturally occurring gene. Means for introducing a mutation are known, and, for example, site specific mutagenesis methods, homologous gene recombination methods, primer elongation methods, polymerase chain reaction (hereinafter, referred to as PCR), and the like can be used solely or suitably in combination. For example, the methods described in books (Sambrook et al. ed., "Molecular Cloning, A Laboratory Manual 2nd ed.," 1989, Cold Spring Harbor Laboratory; Muramatsu, M. ed., "Genetic Engineering Lab Manual," 1988, Maruzen Co., Ltd.) or modified methods thereof can be used, and Ulmer's technique (Ulmer, K. M., "Science," 1983, Vol. 219, p. 666-671) can also be used.

The DNAs used in the present invention include a DNA hybridizable with the DNA represented by the nucleotide sequence of SEQ ID NO: 1 or a splicing variant of the DNA under stringent conditions. As the hybridization conditions, for example, the methods described in books (Sambrook et al. ed., "Molecular Cloning, A Laboratory Manual 2nd ed.," 1989, Cold Spring Harbor Laboratory) and the like can be employed. Specifically, the expression "under stringent conditions" means, for example, a condition of heating in 6 x SSC, 0.5% SDS, and 50% formamide solution at 42°C and then washing in 0.1 x SSC, 0.5% SDS solution at 68°C. These DNAs do not have to be a DNA having a complementary sequence so long as they are DNAs hybridizable with the DNA represented by the nucleotide sequence of SEQ ID NO: 1 or a splicing variant of the DNA. DNAs encoding proteins having functions identical to biological functions of the protein encoded by the DNA represented by the nucleotide sequence of SEQ ID NO: 1 are preferred.

An example of a protein having sequence homology with the protein encoded by the DNA represented by the nucleotide sequence of SEQ ID NO: 1 is a protein comprising an amino acid sequence comprising a mutation such as deletion, substitution, addition, or insertion of one or more, for example, one to 100, preferably one to 30, more preferably one to 20, yet more preferably one to 10, yet more preferably one to several amino acids in the amino acid sequence of SEQ ID NO: 2 and having functions identical to biological functions of the protein encoded by the DNA represented by the nucleotide sequence of SEQ ID NO: 1. The extent, the position, and the like of a mutation of amino acids are not particularly limited so long as the protein comprising the mutation has functions identical to those of the protein represented by the amino acid sequence of SEQ ID NO: 2.

The protein having a mutation may be naturally occurring, be generated by, for example, mutation, posttranslational modification, or the like, or be obtained by introducing a mutation based on the naturally occurring gene. Methods for introducing a mutation are known. For example, site-specific mutagenesis methods, homologous gene recombination methods, primer elongation methods, PCR, and the like can be used solely or suitably in combination. For example, the methods described in books (Sambrook et al. ed., "Molecular Cloning, A Laboratory Manual 2nd ed.," 1989, Cold Spring Harbor Laboratory; Muramatsu, M. ed., "Genetic Engineering Lab Manual,"1988, Maruzen Co., Ltd.) or modified methods thereof can be used. Ulmer's technique (Ulmer, K. M., "Science," 1983, Vol. 219, p. 666-671) can also be used. In view of not changing basic properties (physical properties, functions, bioactivity, immunological activity, etc.) of this protein in introduction of a mutation, for example, substitution between amino acids in the same class (polar amino acids, nonpolar amino acids, hydrophobic amino acids, hydrophilic amino acids, positively charged amino acids, negatively charged amino acids, aromatic amino acids, etc.) can be easily assumed.

Examples of structural characteristics of the DNA include a seven-transmembrane domain-encoding region and a TSP-I domain-encoding region. Furthermore, examples of structural characteristics of the protein encoded by the DNA include a seven-transmembrane domain and a TSP-I domain. DNAs or proteins having sequence homology in such domains and regions of preferably at least 70%, more preferably 70% or more, 75% or more, 80% or more, 85% or more, 90% or more, or 95% or more are preferred. Furthermore, it is more preferable that these domains have the function, for example, a function of localizing a protein containing the domain in the membrane or a function of inhibiting angiogenesis.

Furthermore, as a structural characteristic of the DNA, a region encoding the amino acid sequence of SEQ ID NO: 3 (QTEV) is conserved at the 3' end region. As a structural characteristic of the protein encoded by the DNA, the amino acid sequence of SEQ ID NO: 3 (QTEV) is conserved at the C-terminal region.

Examples of functions as a membrane protein receptor include functions identical to biological functions of the protein encoded by the DNA represented by the nucleotide sequence of SEQ ID NO: 1.

The "functions as a membrane protein receptor" means functions of being expressed as a membrane protein in an animal cell, promoting intracellular signal transduction by an action of a ligand, and inducing cellular responses. Examples thereof include functions identical to those of a GPCR.

The "functions identical to those of a GPCR" means functions of binding to a G-protein by an action of a ligand and activating it, promoting intracellular signal transduction, and inducing cellular responses. The G-protein bound with a GPCR is a trimer consisting of three subunits α, β, and γ, and is roughly classified into four families (Gq, Gs, Gi, and G12) depending on the difference in the α-subunit. When an intracellular signal transduction pathway involving the Gq family is activated, an increase in the calcium concentration is induced as a downstream signal. Meanwhile, when an intracellular signal transduction pathway involving the Gs family is activated, an increase in the level of cyclic AMP (hereinafter, referred to as cAMP) is induced as a downstream signal. When an intracellular signal transduction pathway involving the Gi family is activated, a decrease in cAMP is induced as a downstream signal. It has been reported that, when an intracellular signal transduction pathway involving the G12 family is activated, changes in cytoskeleton and the like are induced as downstream signals, but the actions thereof remain unknown.

Specific examples of cellular responses include changes in the intracellular calcium concentration, changes in the level of intracellular cAMP, and changes in the cell membrane potential. These cellular responses can be measured by known methods. Changes in the intracellular calcium concentration can be detected by, for example, allowing a fluorescent dye that can bind to a calcium ion to be taken up into the cell, inducing a fluorescence phenomenon by an excitation light in the presence of or in the absence of a ligand, and measuring and comparing the amounts of fluorescence. The amount of fluorescence can be measured by using known fluorometers, for example, fluorescence plate readers such as the FLIPR, the FLEX station (Molecular Devices), and the like. Changes in the level of intracellular cAMP can be detected by preparing cell lysates from a cell in which a ligand is allowed to act and a cell in which a ligand is not allowed to act by a known method and measuring and comparing the concentrations of cAMP contained in the cell lysates. The cAMP concentration can be measured by competitive enzyme immunoassay (competitive ELISA: Brezillon, S. et al., "The Journal of Biological Chemistry," 2003, Vol. 278, p. 776-783; Hosoi, T. et al., "The Journal of Biological Chemistry," 2002, Vol. 277, p. 31459-31465) and a measurement method using Homogenous Time Resolved Fluorescence (HTRF). The measurement method using HTRF can be implemented by using, for example, a commercially available cAMP measurement kit (Cis Bio International). Changes in the cell membrane potential can be detected by, for example, expressing the DNA represented by the nucleotide sequence of SEQ ID NO: 1 or a splicing variant of the DNA in an African Xenopus laevis oocyte and in the presence of or in the absence of a stimulus of a ligand and measuring and comparing the amounts of an electrical current generated specifically in a membrane protein receptor.

The ligand is not limited so long as it can act on the gene products of this DNA expressed in the cell to induce cellular responses and can be identified and obtained from an appropriate ligand supply source and used. As ligand supply sources, various samples prepared from cells or tissues, for example, samples prepared from cells or tissues expressing the gene represented by the nucleotide sequence of SEQ ID NO: 1, a peptide library, a protein library, a compound library, and the like can be used.

A ligand can be identified by detecting the binding of a protein encoded by this DNA and a test substance by a known method for analyzing binding. Alternatively, in an method for identification using a cell expressing this protein, a target ligand can be obtained by measuring cellular responses induced in the cell when a test substance is brought into contact with the cell. The test substance means a substance to be examined and includes various samples prepared from cells or tissues, samples prepared from cells or tissues expressing the gene represented by the nucleotide sequence described, a peptide library, a protein library, a compound library, and so forth. When a change (promotion, occurrence, decrease, or elimination) occurs in a cellular response after a test substance is brought into contact with a cell as compared that when it is not brought into contact with a cell, it can be determined that the test substance is a ligand or contains a ligand. Specific examples of cellular responses include changes in the intracellular calcium concentration, changes in the level of intracellular cAMP, and changes in the cell membrane potential. These cellular responses can be measured by the above-mentioned known methods. Alternatively, in an method for identification using a cell expressing this protein, as an indicator of a cellular response, a target ligand can be obtained by measuring an interaction between this protein and a MAGUK family-related protein. When a change (promotion or occurrence) occurs in the interaction between this protein and the MAGUK family-related protein in the cell after the test substance is brought into contact with the protein as compared with when it is not brought into contact, it can be determined that the test substance is a ligand or contains a ligand. An interaction between this protein and a MAGUK family-related protein can be detected by known methods such as immunoblotting.

As ligands, samples prepared from various cells or tissues can be used. A sample can be prepared by, for example, a method of culturing cells or tissues by a known method and centrifuging or the like the conditioned medium or a method of crushing or dissolving cells or tissues by a known method. A ligand can also be purified from the above-mentioned sample by known methods for purifying protein such as, for example, gel filtration chromatography and used. Specific examples of ligands include a conditioned medium of the HeLa cell strain (see Example 3) and CCK8-S (see Examples 5 and 7 to 9).

Examples of functions identical to biological functions of the protein encoded by the DNA represented by the nucleotide sequence of SEQ ID NO: 1 include a function of interacting with a protein having a guanylate kinase activity and/or a cell adhesion function such as, for example, a MAGUK family-related protein. Specific examples of MAGUK family-related proteins include DLG2, DLG3, DLG4, AIP1, and MAGI3.

As described above, the DNA represented by the nucleotide sequence of SEQ ID NO: 1 and splicing variants of the DNA are DNAs encoding a protein functioning as a functional membrane protein receptor having a seven-transmembrane domain. As structural characteristics, the protein encoded by this DNA has several, preferably, two to four TSP-I domains, one GPS domain, and one seven-transmembrane domain (see Figures 1-A and 1-B).

The DNA represented by the nucleotide sequence of SEQ ID NO: 1 comprises a 4557-bp nucleotide sequence including an open reading frame (ORF) encoding 1518 amino acid residues (SEQ ID NO: 2) having a region predicted to be a signal sequence (20 amino acid residues from the N-terminus).

A preferred example of the protein encoded by the DNA represented by the nucleotide sequence of SEQ ID NO: 1 is the protein represented by the amino acid sequence of SEQ ID NO: 2.

The protein encoded by the DNA represented by the nucleotide sequence of SEQ ID NO: 1 comprises 1518 amino acid residues having a region predicted to be a signal sequence (20 amino acid residues from the N-terminus) and has three TSP-I domains as well as an GPS domain and a seven-transmembrane domain in the amino acid sequence thereof (see Figures 1-A and 1-B). The amino acid sequence of this protein is identical to the amino acid sequence of the protein encoded by the DNA represented by the nucleotide sequence of SEQ ID NO: 19, except that 55 amino acid residues including one TSP-I domain on the N-terminus side are deleted. The deleted 55 amino acid residues correspond to the 296th glycine (G) to the 350th proline (P) in the amino acid sequence of the protein represented by the amino acid sequence of SEQ ID NO: 20. The three TSP-I domains comprise a region from the 297th histidine (His) to the 350th proline (Pro), a region from the 352nd glutamic acid (Glu) to the 405th proline (Pro), and a region from the 408th aspartic acid (Asp) to the 461st proline (Pro) in the amino acid sequence of SEQ ID NO: 2. The seven transmembrane domains comprise a region from the 870th valine (Val) to the 890th phenylalanine (Phe), a region from the 899th serine (Ser) to the 919th glycine (Gly), a region from the 928th valine (Val) to the 948th leucine (Leu), a region from the 970th arginine (Arg) to the 990th threonine (Thr), a region from the 1012th alanine (Ala) to the 1032nd phenylalanine (Phe), a region from the 1087th leucine (Leu) to the 1107th alanine (Ala), and a region from the 1114th valine (Val) to the 1134th valine (Val) in the amino acid sequence of SEQ ID NO: 2.

The DNA represented by the nucleotide sequence of SEQ ID NO: 15 comprises a 4389-bp nucleotide sequence including an ORF encoding 1463 amino acid residues (SEQ ID NO: 16) having a region predicted to be a signal sequence (20 amino acid residues form the N-terminus).

An example of the protein encoded by the DNA represented by the nucleotide sequence of SEQ ID NO: 15 is the protein represented by the amino acid sequence of SEQ ID NO: 16.

The protein encoded by the DNA represented by the nucleotide sequence of SEQ ID NO: 15 comprises 1463 amino acid residues having a region predicted to be a signal sequence (20 amino acid residues from the N-terminus) and has a seven-transmembrane domain, two TSP-I domains, and one GPS domain in the amino acid sequence thereof (see Figure 1-B). The amino acid sequence of this protein is identical to the amino acid sequence of the protein encoded by the DNA represented by the nucleotide sequence of SEQ ID NO: 19, except that 110 amino acid residues including two TSP-I domains on the N-terminus side are deleted. The deleted 110 amino acid residues correspond to the 296th glycine (G) to the 405th proline (P) in the amino acid sequence of the protein represented by the amino acid sequence of SEQ ID NO: 20.

The nucleotide sequence of SEQ ID NO: 17 comprises a 4554-bp nucleotide sequence including an ORF encoding 1518 amino acid residues (SEQ ID NO: 18) having a region predicted to be a signal sequence (20 amino acid residues from the N-terminus).

An example of the protein encoded by the DNA represented by the nucleotide sequence of SEQ ID NO: 17 is the protein represented by the amino acid sequence of SEQ ID NO: 18.

The protein encoded by the DNA represented by the nucleotide sequence of SEQ ID NO: 17 comprises 1518 amino acid residues (SEQ ID NO: 18) having a region predicted to be a signal sequence (20 amino acid residues from the N-terminus), and has a seven-transmembrane domain, three TSP-I domains, and one GPS domain in the amino acid sequence thereof (see Figure 1-B). The amino acid sequence of this protein is identical to the amino acid sequence of the protein encoded by the DNA represented by the nucleotide sequence of SEQ ID NO: 19, except that 55 amino acid residues including one TSP-I domain, the 2nd from the N-terminus side, are deleted. The deleted 55 amino acid residues correspond to the 351 st valine (V) to the 405th proline (P) in the protein represented by the amino acid sequence of SEQ ID NO: 20.

The DNA represented by the nucleotide sequence of SEQ ID NO: 19 is referred to as 7tmHR (seven-transmembrane helix receptor) gene (GenBank accession number, AB065648). This DNA comprises a 4719-bp nucleotide sequence including an ORF encoding 1573 amino acid residues (SEQ ID NO: 20) having a region predicted to be a signal sequence (20 amino acid residues from the N-terminus).

An example of the protein encoded by the DNA represented by the nucleotide sequence of SEQ ID NO: 19 is the protein represented by the amino acid sequence of SEQ ID NO: 20.

The protein encoded by the DNA represented by the nucleotide sequence of SEQ ID NO: 19 is referred to as 7tmHR (GenBank accession number, AB065648). This protein comprises 1573 amino acid residues, and has a seven-transmembrane domain, four TSP-I domains, and one GPS domain in the amino acid sequence thereof (see Figure 1-B).

The DNA represented by the nucleotide sequence of SEQ ID NO: 21 is a known human DNA and is referred to as the hBAI2 gene (GenBank accession number, AB005298). This DNA comprises a 5399-bp nucleotide sequence including an ORF encoding 1572 amino acid residues (SEQ ID NO: 22) having a region predicted to be a signal sequence (20 amino acid residues from the N-terminus).

An example of the protein encoded by the DNA represented by the nucleotide sequence of SEQ ID NO: 21 is the protein represented by the amino acid sequence of SEQ ID NO: 22.

The protein encoded by the DNA represented by the nucleotide sequence of SEQ ID NO: 21 is a known human protein and is referred to as the hBAI2 (GenBank accession number, AB005298). This protein comprises 1572 amino acid residues, and has a seven-transmembrane domain, four TSP-I domains, and one GPS domain in the amino acid sequence thereof (see Figure 1-A). The amino acid sequence of this protein is identical to the amino acid sequence of the protein encoded by the DNA represented by the nucleotide sequence of SEQ ID NO: 19, except that one amino acid residue corresponding to the 1461 st lysine is deleted in the C-terminal region.

Thus, DNAs represented by any one nucleotide sequence of the nucleotide sequences of SEQ ID NOS: 1, 15, 17, 19, and 21 and the proteins encoded by the DNAs have homology with one another, and conserve the TSP-I domain, the GPS domain, and the seven-transmembrane domain.

The inventors of the present invention consider based on the similarity in sequence homology and structural characteristics that DNAs represented by any one nucleotide sequence of the nucleotide sequences of SEQ ID NOS: 1, 15, 17, 19, and 21 and the proteins encoded by the DNA are all splicing variants.

Proteins represented by any one amino acid sequence of the amino acid sequences of SEQ ID NOS: 2, 16, 18, 20, and 22 have homology with one another, and conserve the TSP-I domain, the GPS domain, and the seven-transmembrane domain. The inventors of the present invention consider based on the similarity in sequence homology and structural characteristics that these proteins are splicing variants.

The gene products of DNAs represented by any one nucleotide sequence of the nucleotide sequences of SEQ ID NOS: 15, 17, and 19 actually exhibited functions as a membrane protein receptor. Specifically, occurrence of cellular responses by a stimulus of CCK-8S (SEQ ID NO: 14) via intracellular signal transduction was observed in animal cells expressing the gene products as with an animal cell expressing a gene product of the DNA represented by the nucleotide sequence of SEQ ID NO: 1.

The DNA used in the present invention of the above-mentioned DNA can be, for example, a DNA represented by any one nucleotide sequence of the nucleotide sequences of SEQ ID NOS: 1, 15, 17, 19, and 21.

The DNA can be a DNA to which, for example, one or two or more genes of an enzyme such as glutathione S-transferase (GST), β-galactosidase (hereinafter, may be referred to as β-Gal), horseradish peroxidase (hereinafter, may be referred to as HRP), or alkaline phosphatase (hereinafter, may be referred to as ALP), and tag peptides such as His-tag, Myc-tag, or HA-tag, FLAG-tag or Xpress-tag are added on the 5'-end or 3'-end side so long as the functions thereof, for example, expression of an encoded protein or functions of an expressed protein are not inhibited. These genes can be added by commonly used genetic engineering techniques and are useful because they facilitate detection of genes and mRNA.

The DNA used in the present invention can be the DNA represented by the nucleotide sequence of SEQ ID NO: 1 or a DNA comprising a fragment represented by a partial nuceltoide sequence existing in a specified region of a splicing variant of the DNA. Since such a DNA fragment is useful because it can be used as a primer or a probe for detecting the DNA represented by the nucleotide sequence of SEQ ID NO: 1 or splicing variants of the DNA or as a primer for producing this DNA. A primer comprises preferably 15 to 30, more preferably 20 to 25 nucleotides. A probe comprises preferably eight to 50 nucleotides, more preferably 17 to 35 nucleotides, yet more preferably 17 to 30 nucleotides. When the primer or the probe is longer than an appropriate length, pseudo hybridization increases, and specificity decreases. Furthermore, when the length is shorter than an appropriate length, a mismatch occurs, and specificity decreases.

A preferred example of a specified region of the DNA represented by the nucleotide sequence of SEQ ID NO: 1 or a splicing variant of the DNA is a fragment of the protein encoded by the DNA, which is a region encoding a fragment including a ligand-acting site in the protein. The DNA represented by a partial nucleotide sequence fragment existing in such a region is a fragment of a protein encoded by this DNA and can be used for production of a fragment including a ligand-acting site in the protein. A fragment of a protein encoded by this DNA which includes a ligand-acting site in the protein is useful for detection of an action of a ligand on the protein, for example, detection of the binding of the protein and a ligand and detection of a compound that promotes or inhibits the action. Furthermore, the fragment is useful for identification of a compound having an action on the protein similar to that of a ligand, that is an agonist. Such a DNA fragment preferably comprises five or more continuous nucleotides, more preferably 10 or more, more preferably 20 or more nucleotides in the region as the minimum unit.

A fragment of a DNA represented by a partial nuceltoide sequence existing in a specified region in the DNA represented by the nucleotide sequence of SEQ ID NO: 1 or a splicing variant of the DNA is also useful as an antisense oligonucleotide that inhibits the expression of the DNA represented by the nucleotide sequence of SEQ ID NO: 1 or a splicing variant of the DNA when it is a DNA fragment complementary to a sense strand encoding a protein. In general, it is known that a DNA fragment comprising about 20 nucleotides can inhibit the expression of a gene. Therefore, the antisense oligonucleotide comprises preferably 15 or more, more preferably 20 or more nucleotides.

These DNA fragments can be designed as fragments having a target sequence according to the nucleotide sequence information of the DNA represented by the nucleotide sequence of SEQ ID NO: 1 or a splicing variant of the DNA and produced by a known chemical synthesis method. Simply, they can be produced using an automated DNA/RNA synthesis apparatus.

The proteins used in this method for identification can be the above-mentioned proteins, for example, a protein represented by an amino acid sequence comprising any one amino acid sequence of the amino acid sequence of SEQ ID NO: 2, 16, 18, 20, and 22.

Furthermore, the protein used in this method for identification can be a fragment represented by a partial amino acid sequence of a specified region in the protein. A fragment of this protein is useful because it can be used as an antigen for production of an antibody against this protein.

A preferred example of a specified region of this protein is a site at which a ligand act in this protein. A fragment including a ligand-acting site is useful for detection of an action of a ligand on this protein, for example, binding of this protein and a ligand and identification of a compound that promotes or inhibits the action. Furthermore, it is useful for identification of a compound that has an action similar to that of a ligand on this protein, that is an agonist. Furthermore, among the fragments including a ligand-acting site, a fragment that inhibits an interaction between a ligand and this protein is useful as a compound that inhibits induction of a function of this protein by an action of a ligand.

A fragment represented by a partial amino acid sequence existing in a specified region of this protein comprises preferably five or more, more preferably eight or more, yet more preferably 12 or more, particularly preferably 15 or more continuous amino acids as the minimum unit thereof. These fragments can be designed as fragments having a target sequence according to the amino acid sequence information of this protein and produced by, for example, a known chemical synthesis method.

This protein may be prepared from cells or biological samples expressing the gene encoding this protein with a genetic engineering technique or as a cell-free synthesis product or a chemical synthesis product, or may be further purified from these products. Furthermore, this protein can be a protein expressed in a cell containing the gene encoding this protein. The cell can be a transformant obtained by transfecting a vector carrying the gene encoding this protein.

This protein can be further modified by, for example, amidation of a component amino group, a carboxyl group or the like thereof so long as the modification causes no marked change in functions. Furthermore, this protein may be labeled by adding another protein or the like on the N-terminus or C terminus side directly or indirectly via a linker peptide or the like using a genetic engineering technique or the like. Preferably, labeling that does not inhibit basic properties of this protein is desired. Examples of proteins or the like to be added include enzymes such as GST, β-Ga1, HRP, and ALP, tag peptides such as His-tag, Myc-tag, HA-tag, FLAG-tag, and Xpress-tag, fluorescent dyes such as fluorescein isothiocyanate and phycoerythrin, maltose-binding proteins, Fc fragments of immunoglobulins, biotin, and so forth, but are not limited to these examples. Furthermore, radioactive isotopes can also be used for labeling. One or two or more of substances used for labeling in combination can be added. This protein can be easily detected or purified by measuring these substances used for labeling themselves or functions thereof, and for example, an interaction of this protein and other proteins can be detected.

This DNA can be easily obtained based on the sequence information of this DNA with a known genetic engineering technique (refer to Sambrook et al. ed., "Molecular Cloning, A Laboratory Manual 2nd ed.," 1989, Cold Spring Harbor Laboratory; Muramatsu, M. ed., "Genetic Engineering Lab Manual," 1988, Maruzen Co., Ltd., etc.). For example, the DNAs represented by the nuceltoide sequences of SEQ ID NOS: 1, 15, 17, 19, and 21 in the sequence listing can be obtained based on the sequence information thereof with a known genetic engineering technique.

Specifically, this DNA can be obtained by constructing a cDNA library according to a usual method from an appropriate source in which the expression of this DNA has been confirmed and selecting a desired clone using an appropriate probe or primer characteristic to the DNA from the library. Examples of the source of cDNA include various cells and tissues in which the expression of this DNA has been confirmed, cultured cells derived therefrom, and so forth. Examples of the source of the DNA represented by the nucleotide sequence of SEQ ID NO: 1 and splicing variants of the DNA include human brain tissues and brain cells.

Isolation of total RNA from the source, isolation and purification of mRNA, obtaining of cDNA, and cloning thereof are all implemented by usual methods. Furthermore, a cDNA library can be constructed from commercially available polyA+ RNA derived from the human brain, the fetal brain, and the brain hippocampus and used. The method for selecting a desired clone from a cDNA library is not particularly limited either, and commonly used methods can be used. Examples thereof include plaque hybridization methods using a probe selectively binding to a target DNA sequence, colony hybridization methods, and the like, methods using these techniques in combination, and so forth. As a probe used here, DNA chemically synthesized based on the information of the nucleotide sequence of the DNA represented by the nucleotide sequence of SEQ ID NO: 1 or a splicing variant of the DNA and the like can be generally used. Furthermore, sense primers and antisense primers designed based on the nucleotide sequence information of this DNA can be used as such probes.

A target clone can be selected from a cDNA library by, for example, utilizing a known protein expression system to confirm the expressed protein of each clone and using a biological function as an indicator.

In addition, to obtain DNA, DNA/RNA amplification methods by PCR (Ulmer, K. M., "Science," 1983, Vol. 219, p. 666-671; Ehrlich, H. A., ed., "PCR Technology: Principle and Application of DNA Amplification," 1989, Stockton Press; Saiki, R. K. et al., "Science," 1985, Vol. 230, p. 1350-1354) can be preferably used. When it is difficult to obtain a full-length cDNA from a cDNA library, RACE methods ("Experimental Medicine," 1994, Vol. 12, No. 6, p. 615-618), in particular, 5'-RACE methods (Frohman, M. A. et al., "Proceedings of The National Academy of Sciences of The United States of America," 1988, Vol. 85, No. 23, p. 8998-9002) and the like can be preferably used. Primers used in PCR can be suitably designed based on the nucleotide sequence information of DNA and can be obtained by synthesis according to a usual method. Amplified DNA/RNA fragments can be isolated and purified by usual methods. For example, DNA/RNA fragments can be isolated and purified by gel electrophoresis and the like.

The nucleotide sequence of DNA can be determined by usual methods such as, for example, dideoxy methods ("Proceedings of The National Academy of Sciences of The United States of America," 1977, Vol. 74, p. 5463-5467) and the Maxam-Gilbert method ("Methods in Enzymology," 1980, Vol. 65, p. 499-560), or simply using a commercially available sequence kit and the like.

This protein can be obtained, for example, with a common genetic engineering technique (refer to Sambrook et al. ed., "Molecular Cloning, A Laboratory Manual 2nd ed.," 1989, Cold Spring Harbor Laboratory; Muramatsu, M. ed., "Genetic Engineering Lab Manual," 1988, Maruzen Co., Ltd.; Ulmer, K. M., "Science," 1983, Vol. 219, p. 666-671; Ehrlich, H. A. ed., "PCR Technology, Principle and Application of DNA Amplification," 1989, Stockton Press, and the like) based on the nucleotide sequence information of the gene encoding this protein. For example, this protein can be obtained from various cells and tissues in which the expression of the gene encoding this protein has been confirmed, or cultured cells derived therefrom by, for example, constructing a cDNA library from human brain tissues according to a usual method, amplifying the gene using appropriate primers characteristic to the gene, and inducing expression of the obtained gene with a known genetic engineering technique.

Specifically, this protein can be produced by, for example, culturing a transformant transfected with a vector DNA containing the above-mentioned DNA and then recovering a target protein from the obtained culture. The transformant can be cultured by known culture conditions and culture methods optimal for each host. Culture may be performed using this protein expressed by a transformant itself or a function thereof as an indicator. Alternatively, culture may be performed using this protein itself produced in the host or out of the host or the amount of the protein as an indicator, or subculture or batch culture may be performed using the amount of the transformant in the medium as an indicator.

When a target protein is expressed in the cell or on the cell membrane of a transformant, the transformant is crushed to extract the target protein. Further, when a target protein is secreted out of a transformant, a culture broth is used as it is, or a culture broth from which a transformant is removed by centrifugation or the like is used.

This protein can also be produced by common chemical synthesis methods. For example, as a chemical synthesis method of a protein, solid-phase synthesis methods, liquid-phase synthesis methods, and the like are known, and any of these methods can be used. More specifically, such protein synthesis methods include a so-called stepwise elongation method, in which each amino acid is bound one by one to extend the strand based on the amino acid sequence information, and a fragment condensation method, in which fragments comprising several amino acids are synthesized beforehand, and then each fragment is coupled, and this protein can be synthesized by either of the methods. The above-mentioned condensation method used in protein synthesis can also be implemented according to a usual method, and examples thereof include azide methods, mixed anhydride methods, DCC methods, active ester methods, oxidation reduction methods, diphenylphosphoryl azide (DPPA) methods, DCC + additive (1-hydroxybenzotriazole, N-hydroxysuccinamide, N-hydroxy-5-norbornene-2,3-dicarboxyimide, etc.) methods, the Woodward method, and so forth. Proteins obtained by chemical synthesis can be further purified as required according to such various purification methods commonly used as mentioned above.

This protein can be fragmented by cleaving using an appropriate peptidase, and, as a result, fragments of this protein can be obtained.

Proteins can be purified and/or isolated by various isolation methods utilizing physical properties, chemical properties, and the like as desired. Isolation and/or purification can be performed using a function of this protein as an indicator. As isolation methods, for example, ammonium sulfate precipitation, ultrafiltration, gel chromatography, ion exchange chromatography, affinity chromatography, high performance liquid chromatography, dialysis, and the like can be used solely or suitably in combination. Preferably, use of methods in which a specific antibody against this protein is prepared based on the amino acid sequence information of the protein, and specifically is adsorbed using the antibody, for example, affinity chromatography utilizing a column bound with the antibody, is recommended.

An antibody against the protein can be prepared by using this protein or a fragment thereof as an antigen. The protein or a fragment used thereof as an antigen comprises at least eight, preferably at least 10, more preferably at least 12, yet more preferably 15 or more amino acids. To prepare an antibody specific to this protein and/or a fragment thereof, an amino acid sequence region unique to the protein or a fragment thereof is preferably used. The amino acid sequence of this region does not necessarily need to be homologous or identical to that of this protein or a fragment thereof, and a site exposed to the outside in the tertiary structure thereof is preferred. Even if the amino acid sequence of the exposed site is discontinuous on the primary structure, it is sufficient to be a continuous amino acid sequence at the exposed sites. The antibody is not particularly limited so long as it specifically binds to or recognizes this protein and/or a fragment thereof immunologically. The presence or absence of this binding or recognition can be determined by a known antigen-antibody binding reaction.

To produce an antibody, known methods for preparing antibodies can be used. For example, an antibody can be obtained by administering an antigen to an animal in the presence of or in the absence of an adjuvant, solely or by binding it to a carrier, and inducing immunoinduction such as a humoral response and/or a cell-mediated response. The carrier is not particularly limited so long as it does not have a harmful action on the host and enhance the antigenicity, and examples thereof include cellulose, polymerized amino acids, albumin, keyhole limpet hemocyanin, and so forth. Examples of the adjuvant include Freund's complete adjuvant (FCA), Freund's incomplete adjuvant (FIA), Ribi (MPL), Ribi (TDM), Ribi (MPL + TDM), whooping coughing vaccines (Bordetella pertussis vaccine), muramyl dipeptide (MDP), aluminium adjuvant (ALUM), and combinations thereof. As immunized animals, mouse, rat, rabbit, goat, horse, and the like are preferably used.

A polyclonal antibody can be obtained from serum of an animal receiving an antigen by a known method for recovering antibodies. A preferred example of the method for recovering antibodies is immunoaffinity chromatography.

A monoclonal antibody can be produced by recovering an antibody-producing cell (for example, lymphocytes derived from the spleen or lymph nodes) from an animal receiving an antigen and introducing known transformation means using a permanently proliferating cell (for example, myeloma strains such as the P3-X63-Ag8 strain). For example, an antibody-producing cell and a permanently proliferating cell are fused by a known method for preparing a hybridoma, this hybridoma is cloned, a hybridoma that produces an antibody specifically recognizing this protein is selected, and an antibody is recovered from a culture broth of the hybridoma.

The polyclonal antibody or the monoclonal antibody that can recognize and bind to this protein can be utilized as an antibody for purifying this protein, a reagent, a labeling marker, or the like. In particular, an antibody that inhibits a function of this protein or an antibody that binds to this protein and exhibits an action like a ligand of this protein can be used to regulate functions of this protein. These antibodies are useful for elucidation, prevention, improvement, and/or treatment of various diseases caused by abnormality in this protein and a function thereof.

A recombinant vector carrying this DNA can be obtained by inserting this DNA into an appropriate vector DNA. The recombinant vector may be any recombinant vector so long as the DNA is incorporated in the recombinant vector.

The vector DNA is not particularly limited so long as it can be replicated in the host, and is suitably selected depending on the type of the host and the purpose of use. The vector DNA may be a DNA obtained by extracting a naturally occurring DNA or a DNA lacking a portion of the DNA other than the portion required for replication. Representative examples thereof include vector DNAs derived from plasmid, bacteriophage and virus. Examples of the plasmid DNA include plasmids derived from Escherichia coli, plasmids derived from Bacillus subtilis, plasmids derived from yeasts, and so forth. Examples of the bacteriophage DNA include λ phage and the like. Examples of the virus-derived vector DNA include vectors derived from animal viruses such as retrovirus, vaccinia virus, adenovirus, papovavirus, SV40, fowlpox virus, and pseudorabies virus and vectors derived from insect viruses such as baculovirus. In addition, examples thereof include vector DNAs derived from transposons, insertion elements, yeast chromosome elements, and so forth. Alternatively, vector DNAs prepared by combining these DNAs, for example, vector DNAs prepared by combining genetic elements of plasmids and bacteriophages (cosmids, phagemids, etc.). Furthermore, any of expression vectors, cloning vectors, and the like can be used depending on the purpose.

A gene needs to be incorporated into a vector so that a function of a target gene is exhibited, and at least a target gene sequence and a promoter are contained as components thereof. In addition to these components, as desired, gene sequences carrying information about replication and control such as, for example, one or two or more gene sequences selected from cis-elements such as ribosome binding sequences, terminators, signal sequences, and enhancers, splicing signals, and selection markers in combination can be further incorporated into vector DNA by known methods. Examples of the selection markers include the dihydrofolate reductase gene, the ampicillin resistance gene, the neomycin resistance gene, and so forth.

As methods for incorporating a target gene sequence into a vector DNA, known methods can be used. For example, a method is used in which a target gene sequence is treated with suitable restriction enzymes to cleave it at specific sites, and then is mixed with a similarly treated vector DNA to ligate to the sequences with a ligase. Alternatively, a desired recombinant vector can also be obtained by ligating a suitable linker into a target gene sequence and inserting this into a multicloning site of the vector.

A transformant can be obtained by introducing a vector DNA incorporated with this DNA into a host. When an expression vector is used as a vector DNA, this DNA can be expressed, and the protein encoded by this DNA can be further produced. One or two or more vector DNAs incorporated with a desired gene other than this DNA can be further introduced into the transformant.

A transformant obtained by transfecting an expression vector incorporated with this DNA can be used in the method for identification of the present invention.

As a host, either a prokaryote or a eukaryote can be used. Examples of the prokaryote include bacteria belonging to the genus Escherichia such as Escherichia coli, bacteria belonging to the genus Bacillus such as Bacillus subtilis, bacteria belonging to the genus Pseudomonas such as Pseudomonas putida, and bacteria belonging to the genus Rhizobium such as Rhizobium meliloti. Examples of the eukaryote include yeasts such as Saccharomyces cerevisiae and Schizosaccharomyces pombe, insect cells such as Sf9 and Sf21, and animal cells such as monkey kidney-derived cells (COS cell, Vero cell), Chinese hamster ovary cell (CHO cell), mouse L cell, rat GH3 cell, human FL cell, 293 EBNA cell, African Xenopus laevis oocyte. Preferably, animal cells are used.

A vector DNA can be introduced into a host cell by applying a known method, for example, a standard method described in a book (Sambrook et al. ed., "Molecular Cloning, A Laboratory Manual 2nd ed.," 1989, Cold Spring Harbor Laboratory). A more preferred example of the method is integration into a chromosome taking into account stability of the gene. However, an autonomic replication system utilizing an extranuclear gene can be simply used. Specific examples of the method include calcium phosphate transfection, DEAE-dextran-mediated transfection, microinjection, cation lipid-mediated transfection, electroporation, transduction, scrape loading, ballistic introduction, infection, and so forth.

When an animal cell is used as a host, it is preferable that a recombinant vector is autonomously replicable in the cell and comprises a promoter, RNA splice sites, a target gene, a polyadenylation site, and a transcription termination sequence. Furthermore, a replication origin may be included as desired. As a promoter, the SRα promoter, the SV40 promoter, the LTR promoter, the CMV promoter, and the like can be used, and an early gene promoter of cytomegalovirus or the like can be further used. Preferred examples of methods for introducing a recombinant vector into an animal cell include electroporation methods, calcium phosphate methods, lipofection methods, and so forth.

When a prokaryote is used as a host, it is preferable that a recombinant vector is autonomously replicable in the bacterial cell and comprises a promoter, a ribosome binding sequence, a target gene, and a transcription termination sequence. Furthermore, a gene controlling a promoter may be included.

When a bacterium is used as a host, promoters are not particularly limited so long as they can be expressed in a host such as Escherichia coli, and any promoter can be used. Examples thereof include promoters derived from Escherichia coli or a phage, such as the trp promoter, the lac promoter, the PL promoter, and the PR promoter. Artificially designed and modified promoters such as the tac promoter may be used. Methods for introducing a recombinant vector into a bacterial cell are not particularly limited so long as they are methods for introducing a DNA into a bacterial cell. Preferred examples thereof include methods using calcium ion, electroporation, and so forth.

When a yeast is used as a host, promoters are not particularly limited so long as they can be expressed in a yeast, and any promoter may be used. Examples thereof include the gall promoter, the gal10 promoter, the heat shock protein promoter, the MFα1 promoter, the PHO5 promoter, the PGK promoter, the GAP promoter, the ADH promoter, the AOX1 promoter, and so forth. Methods for introducing a recombinant vector into a yeast are not particularly limited so long as they are methods for introducing a DNA into a yeast. Preferred examples thereof include electroporation methods, spheroplast method, lithium acetate methods, and so forth.

When an insect cell is used as a host, preferred examples of methods for introducing a recombinant vector include calcium phosphate methods, lipofection methods, electroporation methods, and so forth.

A specific example of a transformant obtained by transfecting a vector DNA including the DNA represented by the nucleotide sequence of SEQ ID NO: 1 in the sequence listing is the HA-ph01207#10-6 cell strain. The HA-ph01207#10-6 cell strain is a cell strain established by transfecting the CHO-K1 cell strain with a vector expressing a DNA having a nucleotide sequence excluding a region predicted to encode a signal sequence (20 amino acid residues from the N-terminus of the amino acid sequence of SEQ ID NO: 2) among ORFs of the DNA represented by the nucleotide sequence of SEQ ID NO: 1 as a fusion protein carrying an HA-tag at the N-terminus. The HA-ph01207#10-6 cell strain stably expresses the N-terminus HA-tag fusion protein. A specific method for preparing this cell strain will be described in detail in Example 2.

The HA-ph01207#10-6 cell strain was deposited at International Patent Organism Depositary, National Institute of Advanced Industrial Science and Technology (Tsukuba Central 6, 1-1-1 Higashi, Tsukuba, Ibaraki, Japan) on August 19, 2004 and received an accession number FERMBP-10101. Existence of this cell strain was confirmed in a study conducted by the above-mentioned depositary on September 22, 2004.

As described above, this protein is a protein that functions as a membrane protein receptor, and could induce cellular responses by a ligand when expressed in an animal cell. Hereinafter, a membrane protein receptor comprising this protein may be referred to as the membrane protein receptor of the present invention.

Cellular responses by a ligand in an animal cell in which this protein was expressed were specifically observed in the above-mentioned HA-ph01207#10-6 cell strain, into which the DNA represented by the nucleotide sequence of SEQ ID NO: 1 in the sequence listing was stably introduced. More specifically, when CCK-8S (SEQ ID NO: 14) was allowed to act as a ligand in the HA-ph01207#10-6 cell strain, a ligand-specific cellular response, specifically, an increase in the intracellular calcium concentration was observed (see Example 5). This result revealed that this protein functioned as a membrane protein receptor. Induction of cellular responses by a ligand was observed in a cell into which any of the DNA represented by the nucleotide sequence of SEQ ID NO: 15, the DNA represented by the nucleotide sequence of SEQ ID NO: 17, or the DNA having nucleotide sequence of SEQ ID NO: 19 was introduced (see Example 8).

Proteins encoded by a DNA represented by any one nucleotide sequence selected from the nucleotide sequences of SEQ ID NOS: 1, 15, 17, and 19 have a different number of repeats of the TSP-I domain in the N-terminal extracellular domain of the amino acid sequence thereof, but the amino acid sequences are virtually identical except this domain (Figure 1-B). Cellular responses by a ligand were induced in any cell into which a DNA represented by any one nucleotide sequence selected from the nucleotide sequence of SEQ ID NOS: 1, 15, 17, and 19 was introduced (see Examples 5, 8, and 9). Therefore, the inventors of the present invention consider that the TSP-I domain is not greatly involved in the binding between a protein encoded by a DNA represented by any one nucleotide sequence selected from the nucleotide sequences of SEQ ID NOS: 1, 15, 17, and 19 and a ligand and intracellular signal transduction induced by the binding.

The inventors of the present invention found that markedly strong expression of the DNA encoding this protein in tissues was specifically observed in the cerebral cortex, the hippocampus, and the amygdala (see Example 10 and Table 3).

It was revealed from results of an experiment using a knockout mouse deficient in a homologue gene of the DNA encoding the receptor protein that this membrane protein receptor is associated with an anxiety condition or an anxiety disorder.

Specifically, in a fear conditioning test (also referred to as a learned helplessness test) using knockout mice deficient in the mouse BAI2 gene, a homologue gene of the gene encoding this protein and splicing variants thereof, the mice showed an anti-anxiety phenotype (see Example 11). The knockout mice used in this study were knockout mice prepared by gene disruption by homologous recombination targeting a region common in the mouse BAI2 gene and splicing variants thereof, and the BAI2 gene and splicing variants thereof were not expressed.

The fear conditioning test is a technique commonly used as a test method for examining anxiety phenotypes, and is used as a test system for examining the association with an anxiety disorder in evaluation of an anxiolytic agent or the like (Yoshimizu, T. et al., "Psychopharmacology," 2006, Vol. 186, p. 587-593; Nakamura, K. et al., "European Journal of Pharmacology," 2001, Vol. 420, p. 33-43).

The fear conditioning test was specifically performed by giving an aversive stimulus to these knockout mice and wild-type mice and measuring an immobility state (also referred to as a helplessness state) as a fear response to the aversive stimulus in a state without an aversive stimulus on the following day. When time during which an immobility state is observed is short in this test, an anti-anxiety condition is determined. As a result of this test, these knockout mice rarely showed an immobility state as compared with the wild-type mice (see Example 11 and Figure 10). This result demonstrated that these knockout mice showed a strong anti-anxiety condition as compared with wild-type mice. On the other hand, no significant difference was observed between the knockout mice and the wild-type mice in many examination items such as physiological examinations, pathological examinations, and anatomical examinations.

Thus, anti-anxiety was induced in a mouse deficient in the gene product of the mouse BAI2 gene, a homologue gene of the gene encoding this protein and splicing variants thereof. That is, it can be considered that the expression of the mouse BAI2 gene and splicing variants thereof is associated with an anxiety condition.

The inventors of the present invention consider based on this result that the gene product of the DNA represented by the nucleotide sequence of SEQ ID NO: 1 and splicing variants of the DNA is also associated with an anxiety condition and an anxiety disorder in humans. The inventors of the present invention consider that, for example, an abnormality such as an increase in the expression of the DNA represented by the nucleotide sequence of SEQ ID NO: 1 or splicing variants of the DNA induces an anxiety condition and an anxiety disorder.

As described above, this membrane protein receptor activated intracellular signal transduction by the action of CCK-8S (SEQ ID NO: 14). CCK-8S (SEQ ID NO: 14) is a peptide resulting from splicing after translation of CCK. High expression of CCK is observed in brain tissues, in particular, the cerebral cortex, the hippocampus, the amygdala, and the hypothalamus (see Table 3). Furthermore, CCK is known to be associated with physiological functions such as anxiety, analgesia, sedation, suppression of food intake, memory, and learning.

As CCK receptors, two types of GPCRs, the CCK-A receptor (also referred to as CCK1 receptor) and the CCK-B receptor (also referred to as CCK2 receptor), have been reported (Herranz, R., "Medicinal Research Reviews," 2003, Vol. 23, No. 5, p. 559-605, Review). Both of these receptors are GPCRs belonging to class A (rhodopsin-like).

The CCK-A receptor is strongly expressed primarily in the digestive organs, and some are expressed in brain tissues. The affinity of the CCK-A receptors for ligands is stronger in the order of CCK-8S >> CCK-8NS, gastrin > CCK-4. The CCK-A receptor strongly responds to CCK-8S (SEQ ID NO: 14), but specificity of the CCK-A receptor to the ligand is not observed.

Meanwhile, the CCK-B receptor is also extensively expressed in brain tissues in addition to the digestive organs. The affinity of the CCK-B receptor for ligands is stronger in the order of CCK-8S ≥ CCK-8NS, gastrin > CCK-4, and selectivity thereof is lower than that of the CCK-A receptor.

Phenotypes of a CCK-A receptor gene-knockout mouse have been reported, but there has been no report showing the association between this knockout mouse and an anxiety disorder. As phenotypes of the CCK-A receptor-knockout mouse, abnormal functions in the digestive system such as gallstone, abnormal pancreatic enzyme secretion and gallbladder contraction, as well as abnormal homeostasis such as body temperature regulation (Nomoto, S. et al., "American Journal of Physiology. Regulatory, integrative and comparative physiology," 2004, Vol. 287, No. 3, R556-61), behavior activity (Miyasaka, K. et al., "Neuroscience Letters," 2002, Vol. 335, No. 2, p. 115-118), and abnormal appetite regulation (Bi, S. et al., "Neuropeptides," 2002, Vol. 36, No. 2-3, p. 171-181) have been reported.

Many reports have shown phenotypes of a CCK-B receptor-knockout mouse, including reports on abnormalities in the digestive system such as abnormal gastric acid secretion and dysplasia of the stomach mucous membrane, as well as phenotypes in the central nervous system such as anxiety, pain, and memory (Noble, F. et al., "Neuropeptides," 2002, Vol. 36, No. 2-3, p. 157-170). More specifically, suppressed anxiety behaviors (Raud, S. et al., "Psychopharmacology," 2005, Vol. 181, p. 347-357; Horinouchi, Y. et al., "European Neuropsychopharmacology," 2004, Vol. 14, No. 2, p. 157-161), increased anxiety behaviors (Miyasaka, K. et al., "Neuroscience Letters," 2002, Vol. 335, No. 2, p. 115-118), increased behavior activity and memory disturbance (Dauge, V. et al., "Neuropsychopharmacology," 2001, Vol. 25, No. 5, p. 690-698), abnormal sensation of pain and correlation with the opioid system (Kurrikoff, K. et al., "The European Journal of Neuroscience," 2004, Vol. 20, No. 6, p. 1577-1586), and the like have been reported.

Furthermore, phenotypes of a double-knockout mouse deficient in the CCK-A receptor and the CCK-B receptor have also been reported, but phenotypes characteristic to the double-knockout mouse have not been reported (Miyasaka, K. et al., "Neuroscience Letters," 2002, Vol. 335, No. 2, p. 115-118).

Although this membrane protein receptor has ligand affinity similar to that of the CCK-A receptor, the expression distributions thereof differ. Therefore, the inventors of the present invention consider that this membrane protein receptor is responsible for a physiological effect different from that of the CCK-A receptor. Specifically, since expression of the DNA encoding this protein is high specifically in brain tissues, the inventors of the present invention consider that this membrane protein receptor comprising the protein is involved in actions in the central nervous system. In contrast, since expression of the CCK-A receptor is low in brain tissues, it appears not to be associated with bioactivity of CCK in brain tissues.

Although this membrane protein receptor and the CCK-B receptor are both expressed in brain tissues and have similar expression distributions, the ligand affinity thereof differs. Furthermore, the sequence of this membrane protein receptor has very low homology with the sequence of the CCK-B receptor. Specifically, sequence homology of this membrane protein receptor and the CCK-B receptor was analyzed with a computer using several different homology search programs, the identity and the similarity of the nucleotide sequences were 21.0% and 21.0%, respectively, and the identity and the similarity of the amino acid sequences were 5.6% and 9.7%, respectively. Thus, since this membrane protein receptor and the CCK-B receptor have low sequence homology with each other, and the ligand affinity thereof differ, the inventors of the present invention consider that this membrane protein receptor and the CCK-B receptor are separate receptors and play separate physiological roles in the brain.

Since this membrane protein receptor, that is, a gene product of the DNA represented by the nucleotide sequence of SEQ ID NO: 1 and splicing variants of the DNA appears to be associated with an anxiety condition and an anxiety disorder, an anxiety condition can be improved, and an anxiety disorder can be relieved by inhibiting functions and/or the expression of the protein encoded by this DNA or splicing variants of the protein.

In other words, an anxiety condition can be improved, and an anxiety disorder can be treated by an inhibitor of functions of the protein encoded by the DNA represented by the nucleotide sequence of SEQ ID NO: 1 or splicing variants of the protein.

The method for identifying a compound having an anti-anxiety effect of the present invention is characterized by screening for an inhibitor of a function of the protein encoded by the DNA represented by the nucleotide sequence of SEQ ID NO: 1 or a homologue protein thereof.

More specifically, a method for identifying this inhibitor of a function of the protein can be implemented by, for example, using an experimental system that can measure functions of this protein. This method for identification can be implemented by allowing the protein and a compound to be examined (hereinafter, referred to as a test compound) to coexist and measuring a function thereof under a condition under which the protein and the test compound can be interacted in this experimental system, and then comparing with results of measurement in the absence of the test compound to detect changes (decrease, increase, elimination, or occurrence) in the protein function. Effects of the test compound on a function of this protein can be determined by comparing the function of this protein with the coexistence of the test compound with the function of this protein in the absence of the test compound. For example, when the function of this protein is decreased when the test compound coexists as compared with the function of this protein when the test compound does not coexist, it can be determined that the test compound inhibits the function of this protein. Furthermore, it can be determined that such a test compound has an anti-anxiety effect because it inhibits functions of this protein.

Since this protein functions as a membrane protein receptor, examples of functions thereof include binding with a ligand, activation of an intracellular signal transduction mechanism, and induction of cellular responses. More specific examples of the functions of this protein include binding with CCK-8S (SEQ ID NO: 14), interactions with MAGUK family-related proteins, increasing the intracellular calcium concentration, changing the level of intracellular cAMP, changing the membrane potential, and so forth. Specifically, the method for identifying an inhibitor of functions of this protein can be achieved by identifying a compound that inhibits binding of this protein and a ligand of the protein, a compound that inhibits an interaction between the protein and another protein, for example, a MAGUK family-related protein, a compound that inhibits activation of an intracellular signal transduction mechanism by the protein, a compound that inhibits induction of cellular responses by the protein, or the like.

A compound that inhibits the binding of this protein and a ligand of the protein can be identified by allowing this protein and a ligand of the protein to react in the presence of and in the absence of a test compound and measuring the binding of this protein and the ligand. The protein used in this method for identification can be a protein expressed in the cell membrane of the cell. As a cell expressing this protein in the cell membrane, such a cell that naturally occurs can be used, or a transformant obtained by transfection with a vector carrying the DNA encoding this protein can be used. The binding of this protein and a ligand of the protein can be measured by utilizing various binding analysis methods commonly used in drug screening systems. For example, measurement can be performed by allowing a binding reaction of a ligand and this protein, separating a complex formed by the binding of this protein and the ligand from a free ligand and this protein that are not bound, and detecting the complex by a known method such as immunoblotting. Furthermore, the binding can be measured by, for example, allowing a binding reaction of a ligand and this protein, then measuring the ligand binding to this protein using an anti-ligand antibody. The anti-ligand antibody binding to the ligand can be detected by using a secondary antibody labeled with HRP, biotin, or the like. A ligand binding to this protein can also be detected using an anti-ligand antibody labeled beforehand with HRP, biotin, or the like. Alternatively, a ligand binding to this protein can be measured by implementing the above-mentioned method for identification using a ligand labeled beforehand with a desired labeling substance as a ligand used in a binding reaction with this protein and detecting the labeling substance. Examples of the labeling substance include any substances used in common binding analysis methods including tag peptides such as GST, His-tag, Myc-tag, HA-tag, FLAG-tag, and Xpress-tag, fluorescent dyes, and the like. Simply, radioactive isotope elements can be utilized.

A compound that inhibits the activation of an intracellular signal transduction mechanism by this protein and a compound that inhibits cellular responses by the protein can be identified in an experimental system using a cell expressing this protein, bringing the cell into contact with a test compound in advance, or with the coexistence of a test compound, then allowing a ligand of the protein to act, and measuring changes in intracellular signal transduction or changes in a cellular response. The cell can be a transformant obtained by transfection with a vector carrying the gene encoding this protein. A compound that inhibits a function of this protein can be selected by detecting changes (decrease, increase, elimination, or occurrence) in intracellular signal transduction and cellular responses in comparison with results of measurement in the absence of the test compound. Examples of changes in the intracellular signal transduction and changes in cellular responses include changes in the intracellular calcium concentration, changes in the level of intracellular cAMP, changes in the cell membrane potential, changes in the interaction with a MAGUK family-related protein, changes in binding to G-protein, and so forth. When changes are induced by the test compound such as, for example, a decrease in the intracellular signal transduction in the cell or a decrease in cellular responses, it can be determined that the test compound inhibits a function of this protein. Changes in the intracellular calcium concentration, changes in cAMP, changes in the cell membrane potential, changes in the interaction with a MAGUK family-related protein, and changes in the binding to G-protein can be measured by using known methods.

As a ligand, a peptide selected from the group consisting of (i) CCK-8S (SEQ ID NO: 14), (ii) a peptide having a mutation such as deletion, substitution, or addition of one or several, preferably one or two, more preferably one amino acid in the amino acid sequence of CCK-8S, and having functions identical to those of CCK-8S, and (iii) a peptide including the amino acid sequence of the above-mentioned peptide (i) or (ii), and having functions identical to those CCK-8S can be used. The "functions identical to those of CCK-8S" means functions of inducing biological functions of this membrane protein receptor, more specifically, functions of inducing a cellular response such as an increase in the intracellular calcium concentration or a change in the membrane potential in a cell expressing this membrane protein receptor. Since sulfation of the seventh tyrosine residue from the C terminus in CCK-8S (SEQ ID NO: 14) appears to be important for a ligand action of CCK-8S (SEQ ID NO: 14), it is preferable that the sulfated tyrosine residue is conserved in a peptide having functions identical to those of a ligand of this membrane protein receptor, for example, CCK-8S. A protein having a mutation may be naturally occurring, for example, by mutation, posttranslational modification, or the like, or obtained by introducing a mutation based on the naturally occurring gene. Methods for introducing a mutation are known, and the above-mentioned methods can be used. In view of not altering basis properties of this protein (physical properties, functions, bioactivity, immunological activity, etc.), for example, substitutions between amino acids in the same class (polar amino acids, nonpolar amino acids, hydrophobic amino acids, hydrophilic amino acids, positively charged amino acids, negatively charged amino acids, aromatic amino acids, etc.) can be easily assumed.

As a ligand, various samples prepared from cells or tissues themselves can be used. A sample can be prepared by, for example, a method comprising culturing a cell or a tissue by a known method and obtaining a conditioned medium by centrifugation or the like or a method comprising crushing or dissolving a cell or a tissue by a known method. A ligand can be used after purifying the above-mentioned sample by a known protein purification method such as, for example, gel filtration chromatography. Specifically, a conditioned medium of the HeLa cell can be used as a ligand. Alternatively, a compound identified by the above-mentioned methods for identifying a ligand can be used.

In addition, this method for identifying an inhibitor of a function of the protein can be implemented using a cell that overexpresses this protein. It has been reported that, when a GPCR is overexpressed in a cell, activation of a downstream signal may be induced without allowing a ligand to act (Chen, G. et al., "Molecular Pharmacology," 2000, Vol. 57, p. 125-134; Samama, P. et al., "The Journal of Biological Chemistry," 1993, Vol. 286, p. 4625-4636). It is considered that, by using such a cell, a method for identifying an antagonist (inverse agonist) can be implemented without using a ligand. Specifically, it is considered that an antagonist (inverse agonist) of this protein can be identified by overexpressing this protein in a cell to activate a downstream signal and identifying a compound that inhibits the activated signal. Specifically, an antagonist (inverse agonist) of this protein can be identified by bringing a cell overexpressing this protein and a test compound into contact with each other and measuring changes in intracellular signal transduction and changes in cellular responses. A cell overexpressing this protein can be obtained by transfection with a vector carrying the gene encoding this protein by a known genetic manipulation method. The cell overexpressing this protein means a cell in which downstream signals detected in a cell into which the gene was introduced are more activated by introduction of the gene encoding this protein than those in a cell into which the gene is not introduced. An antagonist (inverse agonist) of this protein can be selected by detecting changes (decrease, increase, elimination, or occurrence) in intracellular signal transduction or cellular responses in comparison with results of measurement in the absence of a test compound. Examples of changes in intracellular signal transduction and changes in cellular responses include changes in the intracellular calcium concentration, changes in cAMP, changes in the cell membrane potential, changes in the interaction with a MAGUK family-related protein, changes in the binding to G-protein, and so forth. When changes such as a decrease in changes in intracellular signal transduction in the cell or a decrease in changes in cellular responses are induced by a test compound in a cell expressing this protein, it can be determined that the test compound is an antagonist (inverse agonist) of this protein. Changes in the intracellular calcium concentration, changes in cAMP, changes in the cell membrane potential, changes in the interaction with a MAGUK family-related protein, and changes in the binding to G-protein can be measured by using known methods.

In fact, a compound that exhibited inhibitory effects on cellular responses was identified using an experimental system for measuring changes in intracellular signal transduction using a cell transfected with a vector carrying the DNA represented by the nucleotide sequence of SEQ ID NO: 1 in the sequence listing (see Example 12). In this experimental system, CCK-8S was allowed to act on the above-mentioned cell to activate an intracellular signal transduction mechanism, and changes in intracellular signal transduction were measured by measuring changes in the intracellular calcium concentration. As a test compound, Soft Focus GPCR Target-Directed Library (BioFocus), a compound library, was used. As a result, three types of compounds were identified that inhibit changes in the intracellular calcium concentration resulting from responses of the above-mentioned cell to CCK-8S (formulas [I], [II], and [III]). The inventors of the present invention consider that these compounds act as function inhibitors of the protein encoded by the DNA represented by the nucleotide sequence of SEQ ID NO: 1.

### Formula (I):

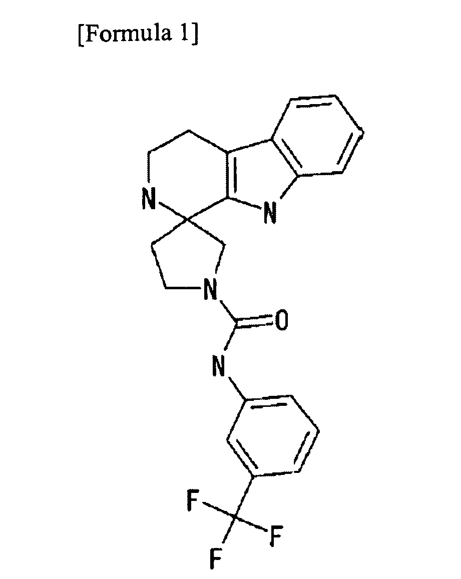

### Formula (II):

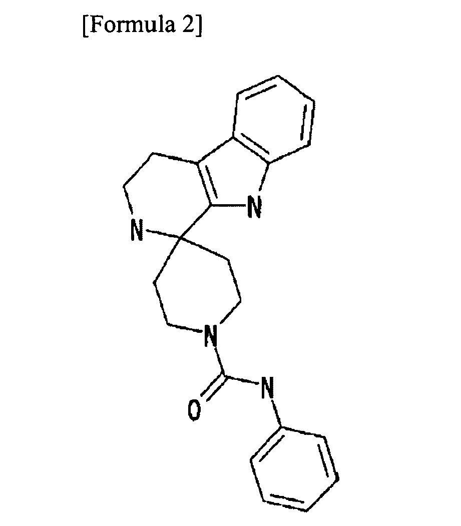

### Formula (III):

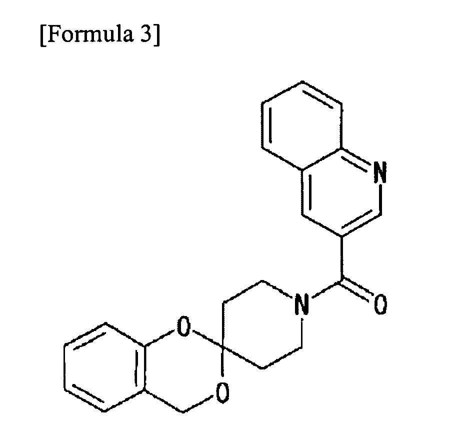

The inventors of the present invention consider based on these results that an inhibitor of a function of this protein can be identified by an experimental system using a cell expressing this protein, for example, a system for assaying changes in the intracellular calcium concentration using the cell.

A compound that inhibits an interaction between this protein and another protein, for example, a MAGUK family-related protein can be identified by, for example, detecting the binding of this protein and another protein, for example, a MAGUK family-related protein using this protein that has been isolated and another protein, for example, a MAGUK family-related protein, that has been isolated by a known protein binding analysis method. Specifically, for example, a MAGUK family-related protein is expressed as a GST-tag fusion protein with a genetic engineering technique and then bound to glutathione sepharose, and the amount of this protein binding thereto can be quantified using an antibody against this protein, for example, an antibody labeled with an enzyme such as HRP or ALP, a radioactive isotope, a fluorescent dye, biotin, or the like. Alternatively, when this protein that is fused with a tag peptide is used, the amount of the protein can be quantified using an anti-tag antibody. It is needless to say that this protein may be labeled directly with the above-mentioned enzymes, radioactive isotope, fluorescent dye, biotin, or the like. Alternatively, a secondary antibody labeled with the above-mentioned enzyme, radioactive isotope, fluorescent dye, biotin, or the like may be used. Alternatively, by coexpressing the DNA encoding this protein and DNA encoding a MAGUK family-related protein using a suitable cell and detecting the binding of these proteins with a pull-down technique, interactions therebetween can be measured.

A method for identifying a compound that affects an interaction between this protein and other proteins including a MAGUK family-related protein can be implemented by, for example, introducing a plasmid that expresses this protein and a DNA-binding protein as a fusion protein, a plasmid that expresses a MAGUK family-related protein and a transcription activating protein as a fusion protein, or a plasmid carrying a reporter gene such as lacZ linked to an appropriate promoter gene into a yeast, a eukaryotic cell, or the like using a two-hybrid method, and comparing the expression level of the reporter gene with the coexistence of a test compound with the expression level of the reporter gene in the absence of the test compound. When the expression level of the reporter gene with the coexistence of a test compound is decreased as compared with the expression level of the reporter gene in the absence of the test compound, it can be determined that the test compound has an inhibitory action on the binding of this protein and a MAGUK family-related protein. On the other hand, when the expression level of the reporter gene with the coexistence of a test compound is increased as compared with the expression level of the reporter gene in the absence of the test compound, it can be determined that the test compound has an action of stabilizing the binding of this protein and a MAGUK family-related protein.

A compound that affects an interaction between this protein and another protein, for example, a MAGUK family-related protein, can also be identified using a surface plasmon resonance sensor such as the BIACORE system.

Furthermore, a compound that affects an interaction between this protein and another protein, for example, a MAGUK family-related protein, can be identified by a method using a Scintillation proximity assay (SPA) or a fluorescence resonance energy transfer (FRET).

Specific examples of the MAGUK family-related protein used in this method for identification include DLG2, DLG3, DLG4, AIP1, MAGI3, and so forth. A portion of the MAGUK family-related protein may be lost so long as an interaction with this protein is not affected, or a labeling substance such as another protein may be added.

More specifically, a method for identifying a compound that can inhibit the expression of this protein can be implemented by, for example, using an experimental system that can measure the expression of this protein. This experimental system can be constructed by utilizing a known drug screening system using at least one of this DNA, a recombinant vector, and a transformant.

A method for identifying a compound that can inhibit the expression of this protein can be implemented by allowing the DNA and a test compound to coexist in an experimental system that can measure the expression of the DNA encoding this protein, measuring the expression, and then detecting a change (decrease or elimination) in the expression in comparison with results of measurement in the absence of the test compound. When the expression with the coexistence of the test compound is decreased as compared with the expression in the absence of the test compound, it can be determined that the test compound inhibits the expression of this protein. The expression can be measured by directly detecting the protein encoded by the DNA or, for example, introducing a signal as an indicator of expression into an experimental system and detecting the signal. As a signal, tag peptides such as GST, His-tag, Myc-tag, HA-tag, FLAG-tag, and Xpress-tag, a fluorescent dye, or the like can be used.

In a method for identifying a compound that can inhibit the expression of this protein, an experimental system using a cell containing the DNA can be used as an experimental system that can measure the expression of the DNA encoding this protein. As a cell, a transformant transfected with an expression vector carrying the DNA encoding this protein is preferably used.

By using a nucleic acid molecule such as DNA, RNA, or DNA/RNA as a test compound in a method for identifying a compound that can inhibit the expression of this protein, small interfering RNA (siRNA) and short hairpin RNA (shRNA), which decrease or eliminate the gene expression by an RNA interference technique (Elbashir, S. M. et al., "Nature," 2001, Vol. 411, p. 494-498; and Paddison, P. J. et al., "Genes and Development," 2002, Vol. 16, p. 948-958), can be identified with an antisense oligonucleotide of the gene encoding this protein. A nucleic acid molecule can be obtained by designing based on the gene encoding this protein and synthesizing by a known chemical synthesis. Nucleic acid molecules can be designed to obtain antisense oligonucleotides, siRNA, and shRNA by using many publications reporting nucleic acid molecules as reference.

When a nucleic acid molecule is used as a test compound in an experimental system using a cell, coexistence of this DNA and the nucleic acid molecule can be achieved by introducing the nucleic acid molecule into a cell. A nucleic acid molecule can be introduced into a cell by a known genetic manipulation method such as, for example, lipofection.

Specifically, a method for identifying a compound that can inhibit the expression of this protein can be implemented by bringing the cell and a test compound into contact with each other in an experimental system using a cell containing the DNA encoding the protein or introducing a test compound into the cell, and then measuring the protein. A compound that inhibits the expression of this protein can be selected by detecting a change (decrease or elimination) in the expression in comparison with results of measurement in the absence of the test compound. Presence or absence of, or a change in the expression of a protein can be detected by a known protein detection method such as, for example, western blotting. Furthermore, presence or absence of, or a change in the expression of a protein can be detected by using a biological function of the expressed protein or a cellular response mediated by the protein such as, for example, an interaction with a MAGUK family-related protein induced when a ligand is allowed to act, a change in the cell membrane potential, or a change in the intracellular calcium concentration as an indicator.

A method for identifying a compound that inhibits the expression of this protein can also be implemented by, for example, preparing a vector to which a reporter gene, instead of the DNA, is ligated to downstream of a promoter region in a gene corresponded to the DNA, bringing a cell into which the vector is introduced, for example, a eukaryotic cell or the like and a test compound into contact with each other, and measuring the presence or absence of or a change in the expression of the reporter gene. As a reporter gene, genes commonly used in a reporter assay can be used, and examples thereof include genes having an enzyme activity of luciferase, β-galactosidase, chloramphenicol acetyl transferase, or the like. Expression of a reporter gene can be detected by detecting an activity of the gene product thereof, for example, detecting an enzyme activity in the case of the above-mentioned examples of a reporter gene.

As a test compound, for example, a compound library, a peptide library, a compound derived from a natural product, or a compound designed based on the primary structure or the tertiary structure of this protein, or the like can be used. Alternatively, a nucleic acid molecule such as DNA or RNA can be used as a test compound.

A compound judged to have an inhibitor effect on a function of this protein is preferably further evaluated for an anti-anxiety effect using a nonhuman animal. Whether the compound has an anti-anxiety effect or not can be evaluated by a test method of an anti-anxiety effect commonly used in nonhuman animals. Many test methods of an anxiety condition using nonhuman animals have been developed and used, and examples thereof include the follow methods: fear conditioning tests (Yoshimizu, T. et al., "Psychopharmacology," 2006, Vol. 186, p. 587-593; Nakamura, K. et al., "European Journal of Pharmacology," 2001, Vol. 420, p. 33-43), the Vogel anti-conflict test (Ballard, T. M. et al., "Psychopharmacology," 2005, Vol. 179, p. 218-229), the Geller-Seifter anti-conflict test (Ballard, T. M. et al., "Psychopharmacology," 2005, Vol. 79, p. 218-229), elevated maze methods (Nakamura, K. et al., "European Journal of Pharmacology," 2001, Vol. 420, p. 33-43; Treit, D. et al., "Pharmacology, Biochemistry, and Behavior," 1993, Vol. 44, p. 463-469; Shepherd, J. K. et al., "Psychopharmacology," 1994, Vol. 116, p. 56-64; Zangrossi, H. Jr. et al., "Brain Research Bulletin," 1997, Vol. 44, p. 1-5; Graeff, F. G. et al., "Neuroscience and Biobehavioral Reviews," 1998, Vol. 23, p. 237-246), hole-board tests (Takeda, H. et al., "European Journal of Pharmacology," 1998, Vol. 350, p. 21-29), light-dark tests (Young, R. et al., "Pharmacology, Biochemistry, and Behavior," 1991, Vol. 40, p. 733-737), social interaction tests (File, S. H. et al., "Journal of Neuroscience Methods," 1980, Vol. 2, p. 219-238), and marble-burying tests (Londei, T. et al., "Behavioural Brain Research," 1998, Vol. 94, p. 249-254). Actions of various anxiolytic agents are tested by these test methods. These test methods can be implemented by using publications or books mentioning these test methods and the like as reference.

As nonhuman animals, specifically, mouse, rat, rabbit, monkey, and the like can be preferably used. More preferably, mouse and rat, yet more preferably, mouse is used.

A fear conditioning test is a technique commonly used as a test method to examine anxiety phenotypes, and is used as a test method for evaluating the associations with an anxiety disorder such as evaluation of anxiolytic agents. The fear conditioning test is specifically performed by, for example, giving an aversive stimulus to a nonhuman animal and measuring an immobility state (also referred to as a helplessness state) as a fear response to the aversive stimulus under a condition with no aversive stimulus on the following day. In this test, when a test compound is given to a nonhuman animal, and time during which an immobility state is observed is shortened compared with a mouse not given the test compound, it can be determined that the test compound has an anti-anxiety effect. The route of administration and the dosage of a test compound given to a nonhuman animal can be determined according to a method commonly used in a drug testing method using a human animal. Such a compound is useful as an anxiolytic agent.

A compound obtained by the method for identification of the present invention can be used as an inhibitor, an antagonist, or the like of a function of this protein such as, for example, binding with a ligand, activation of intracellular signal transduction, induction of a cellular response, or the like. Furthermore, a compound that inhibits the expression of this protein is also identified by the method for identification of the present invention, and such a compound can be used as an expression inhibitor against this protein at a gene level.

Since the gene encoding this protein is associated with an anxiety condition or an anxiety disorder, the inventors of the present invention consider that a compound that inhibits a function of this protein has an anti-anxiety effect. Such a compound can be used as an agent for improving an anxiety condition. That is, agents for improving an anxiety condition including inhibitors of a function of the protein encoded by the DNA represented by the nucleotide sequence of SEQ ID NO: 1 in the sequence listing or splicing variants of the protein can be provided in the present invention.

An inhibitor of a function of this protein can preferably be an inhibitor of a function of a membrane protein receptor comprising this protein induced by CCK-8S. Furthermore, an inhibitor of a function of this protein can be an inhibitor of a function of a membrane protein receptor comprising this protein induced by a peptide having a function identical to that of CCK-8S.

Examples of the function inhibitor of this protein include three types of compounds (represented by the above-mentioned formulas [I], [II], and [III]) identified by the above-mentioned method for identifications (see Example 12).

When a ligand of a membrane protein receptor comprising this protein is a protein, a peptide having a partial peptide of the ligand that binds to the membrane protein receptor but cannot exhibit an effect shown by the ligand can be used as a function inhibitor of this protein. The partial peptide of a ligand of the membrane protein receptor can be obtained by identifying the ligand, designing various partial peptides from the amino acid sequence thereof and synthesizing them, and selecting the one having an activity as an antagonist from the synthesized partial peptides by the compound method for identification of the present invention.

A function inhibitor of this protein can be prepared as a drug by further screening taking into account a balance between biological usefulness and toxicity. These compounds can be expected to have prophylactic and/or therapeutic effect on various pathological symptoms caused by abnormalities in functions of this protein and/or the expression of the DNA encoding the protein.

These proteins, DNAs, recombinant vectors, transformants, antibodies, ligands, and compounds are useful as an active ingredient of a drug or a pharmaceutical composition based on inhibition, antagonism, or promotion of a function and/or the expression of this protein.

The drug or the pharmaceutical composition of the present invention can be used as a prophylactic and/or therapeutic agent for a disease caused by an abnormality in a function of this protein and/or the expression of the DNA encoding the protein. Furthermore, it can be used in a method for prophylactic and/or therapeutic treatment of the disease.

When a function of this protein and/or the expression of the DNA encoding the protein is excessive, as one method, an effective amount of an inhibitor that inhibits a function of the protein and/or the expression of the DNA is administered together with a pharmaceutically acceptable carrier to a target patient to inhibit a function of the protein, and thereby an abnormal symptom can be improved. Furthermore, expression of the DNA encoding an endogenous protein may be inhibited using an expression blocking method. For example, the expression of the DNA encoding this protein can be inhibited by using a fragment of this DNA as an antisense oligonucleotide in gene treatment. The DNA fragment used as an antisense oligonucleotide is useful when it is not only a translated region of this DNA, but a fragment corresponding to an untranslated region. To specifically inhibit the expression of the DNA, it is preferable to use the nucleotide sequence of a region unique to the DNA.

Preferred examples of diseases caused by abnormality in a function of this protein and/or the expression of the DNA encoding the protein include neurogenic diseases such as, for example, anxiety disorders.

The inventors of the present invention consider that a function inhibitor of this protein and a pharmaceutical composition containing an effective amount of this inhibitor are effective for relief, improvement, and prophylactic and/or therapeutic treatment of an anxiety disorder. The drug or the pharmaceutical composition of the present invention can be used in a method for prophylactic and/or therapeutic treatment of an anxiety disorder. Specifically, this drug or pharmaceutical composition can be a prophylactic and/or therapeutic agent for an anxiety disorder containing an effective amount of a function inhibitor of this protein as an active ingredient. In other words, this drug or pharmaceutical composition can be a prophylactic and/or therapeutic agent for an anxiety disorder comprising an effective amount of the anxiolytic agent of the present invention as an active ingredient. A prophylactic and/or therapeutic method of an anxiety disorder can be implemented by applying this anxiolytic agent.

The drug of the present invention may be a drug comprising an effective amount of at least one of the above-mentioned proteins, DNAs, recombinant vectors, transformants, antibodies, ligands, and compounds as an active ingredient, but usually it is preferable to produce a pharmaceutical composition using one or two or more types of carriers for drugs.

The amount of the active ingredient contained in the pharmaceutical preparation of the present invention is suitably selected from a wide range, but usually it is appropriate to select within the range of approx. 0.00001 to 70% by weight, preferably approx. 0.0001 to 5% by weight.

Examples of the carriers for drugs include diluents and excipients such as fillers, extenders, binders, moisturizers, disintegrating agents, surfactants, and lubricants and so forth usually used depending on the use form of a formulation. These are suitably selected and used depending on the administration form of the obtained formulation.

Examples thereof include water, pharmaceutically acceptable organic solvents, collagen, polyvinyl alcohol, polyvinylpyrrolidone, carboxyvinyl polymers, sodium alginate, water-soluble dextran, carboxymethyl starch sodium, pectin, xanthan gum, gum arabic, casein, gelatin, agar, glycerine, propylene glycol, polyethylene glycol, Vaseline, paraffin, stearyl alcohol, stearic acid, human serum albumin, mannitol, sorbitol, lactose, and so forth. These are used suitably combining one or two or more types thereof depending on the dosage form.

As desired, they can also be prepared by suitably using various components usually used in protein formulations such as, for example, stabilizers, disinfectants, buffers, isotonizing agents, chelating agents, pH modifiers, and surfactants.

Example of stabilizers include human serum albumin, usual L-amino acids, saccharides, cellulose derivatives, and so forth, and these stabilizers can be used solely or in combination with a surfactant or the like. Particularly with this combination, stability of the active ingredient may be improved. The above-mentioned L-amino acids are not particularly limited, and may be any of, for example, glycine, cysteine, glutamic acid, and the like. The saccharides are not particularly limited, and may be any of, for example, monosaccharides such as glucose, mannose, galactose, and fructose, sugar alcohols such as mannitol, inositol, and xylitol, disaccharides such as sucrose, maltose, and lactose, polysaccharides such as dextran, hydroxypropyl starch, chondroitin sulfuric acid, and hyaluronic acid, and so forth and derivatives thereof. The cellulose derivatives are not particularly limited either, and may be any of methylcellulose, ethylcellulose, hydroxyethylcellulose, hydroxypropylcellulose, hydroxypropylmethylcellulose, carboxymethylcellulose sodium, and the like. The surfactants are not particularly limited either, and any of ionic and nonionic surfactants can be used. Examples thereof include polyoxyethylene glycol sorbitan alkyl ester, polyoxyethylene alkyl ether, sorbitan monoacyl ester, fatty acid glyceride, and so forth.

Examples of the buffers include boric acid, phosphoric acid, acetic acid, citric acid, ε-aminocapronic acid, glutamic acid, and/or corresponding salts thereof (for example, alkali metal salts thereof such as sodium salts, potassium salts, calcium salts, magnesium salts and alkaline earth metal salts thereof), and so forth.

Examples of the isotonizing agents include sodium chloride, potassium chloride, saccharide, glycerine, and so forth.

Examples of the chelating agents include sodium edetate, citric acid, and so forth.

The drug or pharmaceutical composition of the present invention can be used not only as a solution, but can also be used after lyophilized to convert to a state that can be stored and then dissolved in a buffer or the like containing water, physiological saline, or the like to prepare at an appropriate concentration before use.

The dose range of the pharmaceutical composition is not particularly limited, and is suitably selected depending on the efficacy of contained ingredients, the administration form, the route of administration, the type of the disease, the characteristics of target patients (body weight, age, pathological condition, use of another drug, etc.), the judgment by a physician in charge, and the like. In general, the dose is preferably in the range of approx. 0.01 µg to 100 mg, preferably approx. 0.1 µg to 1 mg per kg body weight of a target patient. However, these doses can be changed using a common experiment for optimization well known in this field. The above-mentioned dose can be given once daily or divided into several times, or intermittently given once in several days or several weeks.

When the pharmaceutical composition of the present invention is administered, the pharmaceutical composition may be used solely or in combination with another compound or drug required for treatment.

As the route of administration, either systemic administration or local administration can be selected. In this case, an appropriate route of administration is selected depending on the disease, the symptom, and the like. Examples of parenteral routes include usual intravenous administration and intraarterial administration as well as subcutaneous, intracutaneous, intramuscular administration, and so forth. Alternatively, the pharmaceutical composition of the present invention can be administered in an oral route. Furthermore, transmucosal administration or percutaneous administration can be performed.

Various dosage forms can be selected depending on the purpose. Representative examples thereof include solid dosage forms such as tablet, pill, powder, powder, subtilized granule, granule, and capsule, inclusion forms such as aqueous solution, ethanol solution, suspension, fat emulsion, ribosome formulation, and cyclodextrin, and solution dosage forms such as syrup and elixir. These are further classified into oral agents, parenteral agents (drip infusion agents, injections), nasal agents, inhalants, transvaginal agents, suppositories, sublingual tablets, eye drops, ear drops, ointments, creams, percutaneous absorption agents, transmucosal absorption agents, and so forth depending on the route of administration, and can be blended, shape-formed, and prepared according to a usual method.

When the pharmaceutical composition of the present invention is used as an agent for gene therapy, it is generally preferable to be prepared as an injection, a drip infusion agent, or a ribosome formulation. When the agent for gene therapy is prepared in a form containing a cell into which a gene is introduced, it can be prepared in a form in which the cell is mixed in a phosphate-buffered physiological saline (pH 7.4), a Ringer's solution, or an intracellular composition injection, or the like. Furthermore, the pharmaceutical composition of the present invention can be prepared in a form to be administered together with a substance that improves the gene introduction efficiency such as protamine. When used as an agent for gene therapy, this pharmaceutical composition can be administered once daily or divided into several times or intermittently at intervals of one day to several weeks. The administration method can be implemented according to a common method used in gene treatment methods.

The present invention also relates to a method for improving an anxiety condition by means of inhibiting a function and/or the expression of the protein encoded by the DNA represented by the nucleotide sequence of SEQ ID NO: 1 in the sequence listing or splicing variants of the protein.

Furthermore, the present invention relates to a method for prophylactic and/or therapeutic treatment of an anxiety disorder, characterized by using a method for improving an anxiety condition comprising the step of inhibiting a function and/or the expression of the protein encoded by the DNA represented by the nucleotide sequence of SEQ ID NO: 1 in the sequence listing or a splicing variant of the protein.

A function inhibitor of this protein has an effect of inhibiting at least one of functions of a membrane protein receptor comprising this protein such as, for example, binding with a ligand, intracellular signal transduction by the membrane protein receptor, and cellular responses by the membrane protein receptor, and expression of the receptor protein. Since it is considered that an anxiety condition or an anxiety disorder is induced by an abnormality such as an increase in the expression of the DNA encoding this protein, the inventors of the present invention consider that an anxiety condition or an anxiety disorder can be improved by inhibiting a function of a membrane protein receptor comprising this protein by a function inhibitor of this protein.

Furthermore, the present invention relates to a measurement method used for diagnosis of an anxiety disorder, comprising quantitatively or qualitatively analyzing any one DNA selected from the DNA represented by the nucleotide sequence of SEQ ID NO: 1 in the sequence listing and splicing variants of the DNA and/or the protein encoded by the DNA as a marker.

This measurement method includes a qualitative or quantitative measurement method of a target gene in a test sample, a qualitative or quantitative measurement method of the protein encoded by the gene, and a qualitative or quantitative measurement method of a mutation of a specific region in the gene or the protein.

Furthermore, the present invention relates to a method for diagnosing an anxiety disorder, comprising quantitatively or qualitatively analyzing any one DNA selected from the DNA represented by the nucleotide sequence of SEQ ID NO: 1 in the sequence listing and splicing variants of the DNA and/or the protein encoded by DNA as a marker.

The presence or absence of an abnormality in a gene including this DNA, an individual, or various tissues or the presence or absence of the expression thereof can be specifically detected by, for example, utilizing a part of or an entire nucleotide sequence of this DNA. By detecting this DNA, susceptibility, onset, and/or prognosis of a disease caused by the gene can be diagnosed. The disease caused by the gene means a disease caused by a quantitative abnormality and/or a functional abnormality or the like of the gene. Examples of the disease caused by this gene include neurogenic diseases such as anxiety disorders.

The presence or absence of an abnormality in the gene or the presence or absence of the expression thereof can be detected by, for example, detecting the existence of a nucleic acid corresponding to the gene in a test sample, determining the abundance thereof, and/or identifying a mutation. In comparison with a normal control sample, changes in the existence of a nucleic acid corresponding to a target gene and quantitative changes thereof can be detected. Furthermore, in comparison with a normal genotype, deletion or insertion can be detected by, for example, measuring changes in the size of the amplification product of a nucleic acid corresponding to a target gene amplified with a known technique. Furthermore, a point mutation can be identified by, for example, hybridizing an amplified DNA with this labeled DNA. The above-mentioned measurement methods and diagnostic methods can be implemented by such detection of a quantitative change and a mutation in a gene.

The test sample is not particularly limited so long as it is a nucleic acid molecule of a target gene and/or a mutant gene thereof, and examples thereof include cells, blood, urine, saliva, cerebrospinal fluid, and biological samples of tissue biopsy, necropsy materials, or the like derived from an organism. Alternatively, as desired, a nucleic acid can be extracted from a biological sample, and a nucleic acid sample can be prepared and used. The nucleic acid may be directly used for detection of a genomic DNA or enzymatically amplified before subjected to an analysis by using PCR or other amplification methods. RNA or cDNA may be similarly used. Furthermore, a nucleic acid sample may be prepared by methods that facilitate the detection of a target sequence such as, for example, denaturation, digestion with a restriction enzyme, electrophoresis, and dot blot.

As a detection method, known gene detection methods such as, for example, plaque hybridization, colony hybridization, southern blot, northern blot, nucleic acid sequence-based amplification (NASBA), and reverse transcriptase polymerase chain reaction (RT-PCR) can be used. Furthermore, measurement at a cell level utilizing in-situ RT-PCR, in-situ hybridization, or the like can also be used. Methods that can be used for detection of a target gene are not limited to the above-mentioned methods, and any of known gene detection methods can be used.

In such gene detection methods, a fragment of this DNA that has a property as a probe or a property as a primer is useful for identification and/or amplification of a target gene or a mutant gene thereof. The DNA fragment having a property as a probe means a fragment comprising a sequence characteristic to the DNA specifically hybridizable with only the target DNA. The DNA fragment having a property as a primer means a fragment comprising a sequence characteristic to the DNA that can specifically amplify only this DNA. Furthermore, when an amplifiable mutant gene is detected, a primer or a probe having a sequence in a predetermined length including a site of a mutation in the gene is prepared and used. In general, the probe or the primer preferably has a nucleotide sequence length of approx. five to 50 nucleotides, more preferably approx. 10 to 35 nucleotides, yet more preferably approx. 15 to 30 nucleotides. As a probe, a labeled probe is usually used, but a probe may be unlabeled or detected by a specific binding to a directly or indirectly labeled ligand. Various methods for labeling a probe and a ligand are known, and examples thereof include nick translation, random priming, methods utilizing treatment with a kinase, and so forth. Examples of appropriate labeling substances include radioactive isotopes, biotin, fluorescent dyes, chemiluminescence substances, enzymes, antibodies, and so forth.

As a gene detection method, PCR is preferred in view of sensitivity. The PCR method is not particularly limited so long as it is a method using DNA fragments as primers that can specifically amplify a target gene. Examples thereof include conventional known methods such as, for example, RT-PCR, but various modified methods used in this field can be applied.

In addition to detection of a gene, a DNA of a target gene and/or a mutant gene thereof can be quantified by PCR. Examples of such analytical methods include competitive assays such as multi-channel simplex stimulated annealing (MSSA) and PCR-single strand conformation polymorphism (PCR-SSCP), which is known as a mutation detection method utilizing a change in mobility along with a change in a higher-order structure of a single-stranded DNA.

The presence or absence of an abnormality in a protein encoded by this DNA, an individual, or various tissues or the presence or absence of the expression thereof can be specifically detected according to known protein detection methods. By detection of an abnormality in this protein and a function thereof, susceptibility, onset, and/or prognosis of a disease caused by a gene including the DNA can be diagnosed.

An abnormality in the presence or absence of the protein or the presence or absence of the expression thereof can be detected by, for example, detecting the existence of a test sample and the protein, determining the abundance thereof, and/or detecting a mutation thereof. Specifically, this protein and/or a mutant thereof are quantitatively or qualitatively measured. Changes in the existence of a target protein and quantitative changes thereof can be detected in comparison with a normal control sample. A mutation thereof can be detected by, for example, determining an amino acid sequence in comparison with a normal protein. The above-mentioned measurement methods and diagnostic methods can be implemented by such detection of a quantitative change and a mutation in a protein.

The test sample is not particularly limited so long as it contains a target protein and/or a mutant thereof, and examples thereof include biological samples of biologic origin such as blood, serum, urine, and biopsy tissues.

This protein and the protein having a mutation in this protein can be measured by, for example, using the protein represented by the amino acid sequence of SEQ ID NO: 2 in the sequence listing, an amino acid sequence including deletion, substitution, insertion, or addition of one or two or more amino acids in the amino acid sequence of the protein, or a fragment thereof as an indicator.

A protein can be quantitatively or qualitatively measured by using protein detection methods or assays according to the conventional art in this field. For example, a mutant protein can be detected by an amino acid sequence analysis of a target protein. More preferably, a difference in the sequence of a target protein or the presence or absence of a target protein can be detected using an antibody (polyclonal or monoclonal antibody).

Specifically, a test sample is subjected to immunosedimentation using an antibody specific to a target protein, and the above-mentioned detection method can be implemented by analyzing the target protein by western blot or immunoblot. Furthermore, a target protein in a paraffin or frozen tissue section can be detected with immunohistochemical techniques using an antibody against a target protein.

Preferred examples of the method for detecting a target protein or a mutant thereof include enzyme-linked immunosorbent assay (ELISA), radioimmunoassay (RIA), immunoradiometric assay (IRMA), endomysial autoantibody (IEMA) assay, and so forth, including sandwich methods using a monoclonal antibody and/or a polyclonal antibody. In addition, competitive binding assays and the like can be utilized.

Any of these protein, DNAs, recombinant vectors, transformants, and antibodies can be used solely as it is as a reagent or the like, for example, as a reagent used in the compound method for identification of the present invention, or the protein and/or DNA measurement method or the diagnostic method of the present invention. The reagent is also useful for elucidation of a cell signal transduction route involving these proteins or DNAs, basic research of diseases caused by abnormalities in these proteins and/or DNAs, and the like.

Any of these protein, DNAs, recombinant vectors, transformants, and antibodies can be used as a reagent included in a reagent kit comprising at least one of these. A reagent kit comprising these can include substances required for measurement, such as a labeling substance for detecting this protein or DNA, a label detecting agent, a reaction diluting solution, a standard antibody, a buffer, a detergent, and a reaction terminating solution. Examples of the labeling substance include the above-mentioned labeling proteins, chemically-labeling substances, and so forth. The labeling substance may be added to this protein or DNA beforehand.

A specific example of the reagent kit of the present invention is a reagent kit comprising at least one of a DNA represented by any one nucleotide sequence of the nucleotide sequences of SEQ ID NOS: 1, 15, and 17 in the sequence listing, a recombinant vector carrying the DNA, a transformant obtained by introducing the recombinant vector, the protein encoded by the DNA, and an antibody that recognizes the protein. A more specific example is a reagent kit comprising at least one DNA represented by any one nucleotide sequence of the nucleotide sequences of SEQ ID NOS: 1, 15, and 17 in the sequence listing, a recombinant vector carrying the DNA, a transformant obtained by introducing the recombinant vector, a protein represented by any one amino acid sequence of the amino acid sequences of SEQ ID NOS: No. 2, 16, and 18, and an antibody that recognizes the protein.

When these are reagents, they may contain substances such as a buffer, a salt, a stabilizer, and/or a preservative. To prepare them as a formulation, known formulation means depending on each property can be introduced.

The reagent kit of the present invention can be used in the above-mentioned identification and measurement methods. Furthermore, the present invention can be used in test methods using the above-mentioned measurement methods as a test agent or a test kit. Furthermore, the present invention can be used in diagnostic methods using the above-mentioned measurement methods as a diagnostic agent or a diagnostic kit.

Hereafter, the present invention will be specifically explained with reference to the following examples. However, the scope of the present invention is not limited to the following examples.

### Example 1

### (Construction of human brain-derived cDNA library and isolation of genes)

A cDNA library was constructed by a usual method using commercially available polyA+ RNA derived from the human brain, the fetal brain, and the brain hippocampus (Clontech: Catalog No. 6516-1, 6525-1, and 6578-1) as starting materials, cDNA fragments were isolated by the dbEST analysis, and the nucleotide sequences of cDNA clones were determined. Specifically, approx. 50,000 recombinants were randomly selected from the above-mentioned human brain-derived cDNA library prepared by the method by Ohara et al. (Ohara, O. et al., "DNA Research," 1997, Vol. 4, p. 53-59), and, of these, the nucleotide sequences at the 5' end and the 3' end of approx. 30,000 cDNA clones were determined. Furthermore, approx. 1100 clones were selected mainly by an in-vitro transcription translation experiment, and the nucleotide sequences of these cDNAs were determined according to the method by Ohara et al.

For cDNA clones of which entire nucleotide sequence was determined, and an ORF was predicted by a general-purpose analysis method using a computer program to obtain a cDNA clone having a seven-transmembrane domain in this region.

The identified cDNA clone ph01207 is a DNA represented by the nucleotide sequence (SEQ ID NO: 1) with a full length of 4557 bp including an ORF comprising 1518 amino acid residues having a region expected to be a signal sequence (20 amino acid residues from the N-terminus). From homology search, ph01207 appeared to be a splicing variant of the sequence of hBAI2 (GenBank accession number, AB005298) which was deficient in a region encoding 55 amino acid residues in the N-terminal region, and into which one amino acid residue (the 1406th lysine in the amino acid sequence of SEQ ID NO: 2) was further inserted in a region on the C terminus side (Figure 1-A).

As structural characteristics, existence of a GPS domain and seven-transmembrane domains in addition to three TSP-I domains was found in the protein encoded by ph01207.

Meanwhile, the protein encoded by hBAI2 appears to have a GPS domain and a seven-transmembrane domain in addition to four TSP-I domains. It was found that 55 amino acid residues corresponding to a region including one TSP-I domain on the N-terminal region side of hBAI2 was deleted in the protein encoded by ph01207.

The expression tissue analysis of the ph01207 gene showed specifically high expression of this gene in overall normal brain tissues (for example, the temporal pole of cerebrum, the motor cortex, the hippocampus, the amygdala, etc.). Furthermore, the expression increased in tissues with ovary cancer, liver cancer, and adrenal gland cancer as compared with normal tissues in each organ.

### Example 2

### (Preparation of ph01207 expressing cell strain and expression of DNA in the cell strain)

By using the clone ph01207 identified in Example 1, the protein encoded by ph01207 was expressed as an N-terminus epitope tag fusion protein.

First, an expression vector carrying the ph01207 gene was constructed. By using the clone ph01207 identified in Example 1, a DNA encoding an amino acid sequence excluding a region expected to be a signal sequence (20 amino acid residues from the N-terminus) was cloned into pDONR201 by PCR and restriction enzyme treatment to construct a ph01207 entry vector. PCR was performed using the oligonucleotides having the nucleotide sequences of SEQ ID NOS: 4 to 11 in the sequence listing as primers and Pfu turbo DNA polymerase (Stratagene) as a polymerase.

As N-terminus epitope-tag fusion-type expression vectors, two types, p3xFLAG-CMV9-attR and T8HA-attR/pCINeo, were prepared. p3xFLAG-CMV9-attR is a vector obtained by converting p3xFLAG-CMV9 (Sigma) to a GATEWAY system destination vector by the GATEWAY Vector Conversion System (Invitrogen). T8HA-attR/pCINeo was constructed according to the information from a publication (Koller, K. J. et al., "Analytical Biochemistry," 1997, Vol. 250, p. 51-60) using pCINeo (Promega), synthetic oligonucleotides (T8SP-HA [SEQ ID NO: 12] and T8SP-HAas [SEQ ID NO: 13]), and the GATEWAY Vector Conversion System. Both the vectors have a secretory signal sequence (FLAG:PPTLS, HA:T8) before the epitope-tag (on the N-terminus side). By using these vectors, an N-terminal FLAG-tag or HA-tag fusion-type expression vector was constructed by LR reaction of the Mammalian Expression system with GATEWAY technology (Invitrogen). It was confirmed by restriction enzyme treatment and a sequence analysis that each of the constructed expression vectors included no nucleotide substitution or loss in a translated region of the introduced sequence.

The two types of constructed expression vectors were transfected into CHO-K1 cell strains using Fugene 6 (Roche), and cells were cultured in a medium containing G418 (DMEM/F12 medium containing 400 µg/mL G418 and 10% fetal calf serum) to screen for expression cell strains. The grown expression cell strains were further screened by cell enzyme immunoassay (cell EIA) using peroxidase (POD)-labeled antibodies against the epitope-tag (anti-FLAG M2-POD antibody and anti-HA-POD antibody: 3F10). The selected cell strains were further screened by flow cytometry (FCM) analysis using primary antibodies (anti-FLAG M2 antibody and anti-HA antibody: clone HA-7) and a fluorescein isothiocyanate (FITC)-labeled secondary antibody (FITC-anti-mouse IgG antibody) to obtain expression cell strains.

As a result of the FCM analysis, eight clone strains expressing a protein recognized by the anti-FLAG antibody (FLAG-tag fusion protein) on the cell membrane and four clone strains expressing a protein recognized by the anti-HA antibody (HA-tag fusion protein) were obtained. Since a fluorescence signal due to binding of an antibody recognizing FLAG-tag or HA-tag was clearly stronger in all the clones than that of the host cell strain (CHO-K1), expression of the target gene was demonstrated. The representative results are shown in Figure 2.

The results of the FCM analysis demonstrated that the N-terminus epitope-tag fusion-type ph01207 gene product was expressed on the cell membrane.

Of the clones found to express the HA-tag fusion protein, a cell strain designated as HA-ph01207#10-6 was deposited in the International Patent Organism Depositary, National Institute of Advanced Industrial Science and Technology on August 19, 2004 as accession number FERMBP-10101. The existence of this cell strain was confirmed by a test conducted by the above-mentioned depositary on September 22, 2004. The HA-ph01207#10-6 cell strain is a cell strain established by transfecting the CHO-K1 cell strain with a vector expressing, as an N-terminus HA-tag fusion protein, a DNA having a nucleotide sequence excluding the region predicted to encode a signal sequence (20 amino acid residues from the N-terminus of the amino acid sequence of SEQ ID NO: 2) from open reading frames (ORFs) in the DNA represented by the nucleotide sequence of SEQ ID NO: 1, and stably expresses the N-terminus HA-tag fusion protein.

### Example 3

### (Functional analysis of ph01207 gene product)

The functional analysis of the ph01207 gene product was performed by measuring cellular responses when a ligand was added to an African Xenopus laevis oocyte expressing ph01207. The cellular responses were measured by using six each of control oocytes (into which the gene was not introduced) and oocytes expressing the gene (ph01207 cDNA clones) and measuring changes in the membrane potential in the oocytes after addition of a ligand. As a ligand, a conditioned medium of a HeLa cell strain in which the expression of ph01207 was recognized was used. The conditioned medium was prepared by culturing 1.2 × 10⁶ HeLa cell strains in DMEM containing 10% fetal calf serum for 2 days and then recovering and filtering the conditioned medium with a filter (0.45 µm). As ligands, two different lots of conditioned media similarly prepared were used (hereinafter, referred to as ligand samples 1 and 2).

The ligand sample 1 or 2 was added to the oocytes expressing the ph01207 gene and the control oocytes, and changes in the membrane potential were measured. Evaluation was made using changes in the amount of electrical current and waveforms representing changes in the amount of electrical current. When a change in the amount of electrical current was 0.2 µA or more, and a waveform having a GPCR-specific pattern was observed, it was determined that there was a response to a ligand stimulus. The GPCR-specific pattern means the pattern of waveform 1 shown in Figure 3. It was determined that the patterns of waveforms 2 to 4 shown in Figure 3 were waveform patterns that occurred due to artificial factors, such as an effect of any component such as a solvent or high-concentration ligand, and did not represent a response to a ligand stimulus. Furthermore, when such patterns as waveforms 5 and 6 were observed, it was determined that there was no response to a ligand stimulus.

As a results, a change in the amount of electrical current was observed only in the ph01207 expressing cells (Tables 1 and 2). ND in Table 2 denotes a change in the amount of electrical current being 0.2 µA or less, showing that no response was detected. As shown in Table 1, a response to a ligand stimulus was observed in all the six ph01207 expressing cell strain samples. On the other hand, the control cells did not show any response to a ligand (Table 2). These results suggest that a response to a ligand stimulus in the ph01207 expressing cells is a response specific to the expressed receptor. Similar results were obtained using either of the ligand samples 1 and 2.

**[Table 1]**

| | ph01207-expressing oocytes | | | | | | | | |
|---|---|---|---|---|---|---|---|---|---|
| Ligand sample | Response rate | Representative waveform | Mean change in amount of electrical current ± S.D. | Change in amount of electrical current (µA) | | | | | |
| | | | | 1 | 2 | 3 | 4 | 5 | 6 |
| 1 | 6/6 | GPCR | 1.21 ± 0.54 | 1.33 | 1.81 | 1.30 | 0.50 | 0.63 | 1.70 |
| 2 | 6/6 | GPCR | 0.48 ± 0.24 | 0.66 | 0.24 | 0.29 | 0.58 | 0.82 | 0.27 |

**[Table 2]**

| | Control oocytes | | | | | | | | |
|---|---|---|---|---|---|---|---|---|---|
| Ligand sample | Response rate | Representative waveform | Mean change in amount of electrical current ± S.D. | Change in amount of electrical current (µA) | | | | | |
| | | | | 1 | 2 | 3 | 4 | 5 | 6 |
| 1 | 0/6 | ND | | ND | ND | ND | ND | ND | ND |
| 2 | 0/6 | ND | | ND | ND | ND | ND | ND | ND |

Since a cellular response to a ligand stimulus was induced by the expression of the ph01207 gene, the ph01207 gene product appears to be a GPCR having a function of activating an intracellular signal transduction pathway in response to a ligand.

### Example 4

### (Protein interaction analysis of ph01207 gene product)

The protein interaction analysis of the ph01207 gene product was performed using a yeast two-hybrid system.

Based on the sequence information of ph01207 and the sequence information of hBAI2, a bait was established in an N-terminal region (four sites) and a C-terminal region (two sites) of the ph01207 gene product and an N-terminal region (a region in ph01207 where 55 amino acid residues were deleted) and a C-terminal region (a region in ph01207 where one amino acid residue was inserted) of the hBAI2 gene product, and a cDNA library (derived from the brain, the hippocampus, breast cancer and prostatic cancer cells, the heart, and the skeletal muscle) selected based on a report on expression distributions of hBAI2 (Non-Patent Document 1) was screened.

When a bait was used in the C-terminal region of each of the ph01207 gene product (with insertion of the 1461st amino acid residue in the amino acid sequence of SEQ ID NO: 20) and the hBAI2 gene product (without insertion of the above-mentioned amino acid residue), MAGUK family-related proteins such as DLG2, DLG3, and DLG4, AIP1, MAGI3, and the like were obtained as preys. In addition, HOMER2, Citron, SYNE-1, KIF5A, and KIFAP3 were obtained as preys specific to the hBAI2 gene product.

For the bait designed in the N-terminal region (with deletion of 55 amino acid residues) of the ph01207 gene product, only BAT1 (HLA-B associated transcript 3) was obtained as a prey, and potassium intermediate/small conductance calcium-activated channel, subfamily N, member 2 (KCNN2) and the like hit the bait in the N-terminal region (without deletion of the above-mentioned amino acid residue) of the hBAI2 gene product.

Thus, it was found that the ph01207 gene product and the hBAI2 gene product differ in their interacting proteins detected by a yeast two-hybrid system except MAGUK family-related proteins.

It was found that the ph01207 gene product and the hBAI2 gene product interacted with MAGUK family-related proteins in the respective C-terminal region. MAGUK family-related proteins are involved in signal transduction from membrane proteins by existing in the cytoplasm and binding to membrane proteins such as receptors and ion channels existing in the cell membrane. Therefore, it is considered that the ph01207 gene product and the hBAI2 gene product are functional membrane protein receptors involved in intracellular signal transduction via MAGUK family-related proteins.

### Example 5

### (Measurement of changes in intracellular calcium concentration induced by CCK8 in ph01207 expressing cell strain)

Changes in the intracellular calcium (Ca²⁺) concentration induced by CCK-8S (SEQ ID NO: 14), CCK-8NS, or CCK-4 were measured using the cell strains expressing ph01207 prepared in Example 2. The cell strains prepared in Example 2 were eight strains that expressed the protein encoded by ph01207 as FLAG-tag fusion proteins and four strains that expressed the protein encoded by ph01207 as HA-tag fusion proteins. Of these, the HA-ph01207#10-6 cell strain, a strain obtained by cloning one of the cell strains that stably expressed the protein encoded by ph01207 as HA-tag fusion proteins, was used in this example. CCK-8NS was a CCK octapeptide having the same amino acid sequence as that of CCK-8S (SEQ ID NO: 14), but a region from the C terminus to the seventh tyrosine residue was not sulfated. CCK-4 was a tetrapeptide comprising residues from the C terminus to the fourth amino acid of CCK-8S (SEQ ID NO: 14).

The specific method for measuring changes in the intracellular Ca²⁺ concentration in the HA-ph01207#10-6 cell strain induced by CCK-8S (SEQ ID NO: 14) and the results are described below. The HA-ph01207#10-6 cell strain was seeded on a 96-well plate (a plate with a black wall and a transparent bottom) at a cell count of 2 × 10⁴/100 µL medium/well and cultured at 37°C in the presence of 5% CO₂. As a medium, DMEM/F12 (Gibco) containing 10% fetal calf serum (Moregate) was used. On the following day, 50 µL of the medium was removed from each well, 50 µL of a loading buffer was added to each well, and the mixture was reacted at room temperature for 1.5 h, so that a fluorescent dye contained in the loading buffer should be taken up into the cell. The loading buffer was prepared by dissolving component A of the FLIPR Calcium 3 Assay Kit (Molecular Device) in a solution obtained by adding 0.1 mL of 500 mM probenecid (Sigma) to 9.9 mL of component B. After reaction, changes over time in the fluorescence intensity in each well after addition of CCK-8S (Peptide Institute, Inc.) were measured using the FLEX station (Molecular Device). Furthermore, similar measurement was performed for CCK-8NS (Peptide Institute, Inc.) and CCK-4 (Peptide Institute, Inc.). 0.52 mg of CCK-8S (SEQ ID NO: 14) was dissolved in 4.5 mL of 1% NaHCO₃ (Wako) to prepare a solution having 0.1 mM concentration. 0.53 mg of CCK-8NS was dissolved in 0.50 mL of dimethyl sulfoxide (DMSO, Sigma), and then 4.50 mL of redistilled water H₂O was added to prepare a solution having 0.1 mM concentration. 0.54 mg of CCK-4 was dissolved in 0.46 mL of DMSO, 4.09 mL of redistilled water was added to prepare a solution having 0.2 mM concentration. CCK-8S (SEQ ID NO: 14), CCK-8NS, and CCK-4 were diluted in phosphate-buffered physiological saline (PBS) to prepare 5 nM solutions, and 25 µL each was added (final concentration 1 nM) to perform measurement. As a positive control for including an increase in the intracellular Ca²⁺ concentration, A23187 (Calbiochem) was used at a final concentration of 10 µM. As a negative control, the CHO-K1 cell strain used to prepare the HA-ph01207#10-6 cell strain as a host was used. Since the CHO-K1 cell strain was not transfected with the ph01207-expressing vector, the ph01207 gene product was not expressed.

As a result, the intracellular Ca²⁺ concentration in the HA-ph01207#10-6 cell strain stimulated by CCK-8S (SEQ ID NO: 14) (Figure 4-A) increased as compared with that in the HA-ph01207#10-6 cell strain with no stimulus (Figure 4-E). However, the intracellular Ca²⁺ concentration in the HA-ph01207#10-6 cell strain did not increase by a stimulus of CCK-8NS or CCK-4 (Figures 4-B and 4-C). Furthermore, the intracellular Ca²⁺ concentration in the HA-ph01207#10-6 cell strain was increased by A23187 (Figure 4-D).

The degree of the increase in the intracellular Ca²⁺ concentration induced by 1 nM CCK-8S (SEQ ID NO: 14) in the HA-ph01207#10-6 cell strain (Figure 4-A) was similar to the degree of the increase in the intracellular Ca²⁺ concentration induced by A23187 used as a positive control (Figure 4-D).

On the other hand, the intracellular Ca²⁺ concentration in the CHO-K1 cell strain was not increased by a stimulus of CCK-8S (SEQ ID NO: 14), CCK-8NS, or CCK-4 (Figures 4-A, 4-B, and 4-C). However, the intracellular Ca²⁺ concentration in the CHO-K1 cell was increased by A23187 (Figure 4-D).

These results revealed that the HA-ph01207#10-6 cell strain responded specifically to CCK-8S (SEQ ID NO: 14). Furthermore, the increase in the intracellular Ca²⁺ concentration in the HA-ph01207#10-6 cell strain induced by CCK-8S (SEQ ID NO: 14) involved the protein encoded by ph01207 expressed in this cell strain. Based on this finding, it is concluded that CCK-8S (SEQ ID NO: 14) is a ligand of the ph01207 gene product.

Furthermore, the HA-ph01207#10-6 cell strain showed a sufficiently high cellular response to 1 nM CCK-8S (SEQ ID NO: 14). Since CCK-8S (SEQ ID NO: 14) at as low a concentration as 1 nM induced a cellular response in the HA-ph01207#10-6 cell strain, CCK-8 appears to actually induce a cellular response in the organism via the protein encoded by ph01207. That is, it is concluded that CCK-8S (SEQ ID NO: 14) is one of ligands in an organism of ph01207 predicted as a GPCR.

### Example 6

### (Identification of splicing variants of ph01207 gene)

Splicing variants of the ph01207 gene were obtained by cloning by RT-PCR.

Splicing variants of the ph01207 gene were obtained as follows. First, a cDNA library was constructed by a usual method using polyA(+) RNA (Clontech) derived from the human brain as a starting material, and cDNA fragments were isolated by the dbEST analysis to determine nucleotide sequences of cDNA clones. Specifically, RT-PCR was performed using the Superscript First-Strand Synthesis System for RT-PCR (Invitrogen) in 10 µL of a reaction system containing 0.1 µg of polyA(+) RNA to construct a cDNA library. PCR was performed using this cDNA as a template and an oligonucleotide nucleotide sequence comprising the nucleotide sequence of SEQ ID NO: 23 and an oligonucleotide comprising the nucleotide sequence of SEQ ID NO: 2 as primers. A DNA of a region encoding the amino acid sequence from the 44th aspartic acid (D) to the 475th cysteine (C) in hBAI2 (SEQ ID NO: 22) and a DNA corresponding to this region in splicing variants of hBAI2 (SEQ ID NO: 22) were amplified by PCR using these primers (see Figure 1-C). Recombination was allowed to occur between each of the obtained clones and the ph01207 cDNA clone with ScaI and XhoI to obtain three types of full-length cDNA clones. The nucleotide sequence of each clone was confirmed by sequence analysis.

As a result, three types of full-length cDNA clones that appeared to be splicing variants of the ph01207 gene based on the sequence homology and the structural similarity were obtained. These cDNA clones were designated as the 7tmHR gene, the hk01941 gene, and the variant 3 gene. All of these splicing variants are variants with a different repeat number of the TSP-I domain in the extracellular domain at the N-terminus (Figure 1-B). Of these splicing variants, the 7tmHR gene encodes the longest protein.

The 7tmHR gene comprises a 4719-bp nucleotide sequence (SEQ ID NO: 19) including an ORF encoding 1573 amino acid residues (SEQ ID NO: 20) having a region predicted to be a signal sequence (20 amino acid residues from the N-terminus). The 7tmHR gene is registered in GenBank as an accession number AB065648. The protein encoded by this gene has a seven-transmembrane domain, four TSP-I domains, and one GPS domain (see Figure 1-B). The protein has an amino acid sequence identical to the sequence of hBAI2 (GenBank accession number AB005298), except that one amino acid residue is inserted in a C-terminal region. The insertion of one amino acid residue is observed between the 1460th glutamic acid (E) and the 1461st valine (V) in the amino acid sequence of hBAI2, and the inserted amino acid residue is lysine. The inserted lysine corresponds to the 1461 st position in the amino acid sequence of the protein encoded by the 7tmHR gene (SEQ ID NO: 20). Proteins encoded by the 7tmHR gene and the gene appeared to be splicing variants of the hBAI2 gene based on the sequence homology and the structural similarity.

The hk01941 gene is a novel gene which comprises a 4389-bp nucleotide sequence (SEQ ID NO: 15) including an ORF encoding 1463 amino acid residues (SEQ ID NO: 16) having a region predicted to be a signal sequence (20 amino acid residues from the N-terminus). The protein encoded by this DNA has a seven-transmembrane domain, two TSP-I domain, and one GPS domain (see Figure 1-B). The amino acid sequence of the protein encoded by this DNA is identical to the protein encoded by the 7tmHR gene (SEQ ID NO: 20), except that 110 amino acid residues including two TSP-I domains on the N-terminus side are deleted. The 110 deleted amino acid residues correspond to the 296th glycine (G) to the 405th proline (P) in the amino acid sequence (SEQ ID NO: 20) of the protein encoded by the 7tmHR gene.

The variant 3 gene is a novel gene which comprises a 4554-bp nucleotide sequence (SEQ ID NO: 17) including an ORF encoding 1518 amino acid residues (SEQ ID NO: 18) having a region predicted to be a signal sequence (20 amino acid residues from the N-terminus). The protein encoded by this DNA has a seven-transmembrane domain, three TSP-I domains, and one GPS domain (see Figure 1-B). The amino acid sequence of the protein encoded by this DNA is identical to that of the protein encoded by the 7tmHR gene (SEQ ID NO: 20), except that 55 amino acid residues including one TSP-I domain, the 2nd from the N-terminus side, are deleted. The 55 deleted amino acid residues correspond to the 351st valine (V) to the 405th proline (P) in the amino acid sequence (SEQ ID NO: 20) of the protein encoded by the 7tmHR gene.

The amino acid sequence of the protein encoded by the ph01207 gene is identical to the amino acid sequence (SEQ ID NO: 20) of the protein encoded by the 7tmHR gene, except that 55 amino acid residues including one TSP-I domain on the N-terminus side are deleted. The 55 deleted amino acid residues correspond to the 296th glycine (G) to the 350th proline (P) in the amino acid sequence (SEQ ID NO: 20) of the protein encoded by the 7tmHR gene.

### Example 7

### (Construction of strains stably expressing splicing variants of ph01207 gene)

Strains stably expressing splicing variants of the ph01207 gene were constructed to examine whether cellular responses were induced by CCK-8S in these stably-expressing strains. As the splicing variants, three types of splicing variants obtained in Example 6, the 7tmHR gene, the hk01941 gene, and the variant 3 gene, were used.

First, a vector expressing each full-length cDNA clone was constructed by allowing a recombination reaction to occur with each full-length cDNA clone using pcDNA3.1(+) (Invitrogen), an expression vector for animal cells, with Asp718I and NotI. The obtained expression vectors were designated as 7tmHR/pcDNA3.1, hk01941/pcDNA3.1, and variant3/pcDNA3.1.

The 7tmHR stably-expressing strain and the hk01941 stably-expressing strain were prepared by transfecting the 7tmHR-expressing vector (7tmHR/pcDNA3.1) and the hk01941-expressing vector (hk01941/pcDNA3.1), respectively, into a CHO-K1 cell strain. Specifically, the expression vector was mixed with Lipofectamine 2000, (may be referred to as LF2000, Invitrogen) (4 µg DNA/250 µL of DMEM/F12 medium + 10 µL of LF2000/250 µL DMEM/F12 medium) and added the CHO-K1 strain (2 mL medium/well) cultured in a 6-well plate to be transfected thereinto. On the following day, cells were recovered and seeded in a 96-well plate at cell density of 1 cell/well, cultured in a selective medium containing G418 (400 µg/mL) (DMEM/F12 medium containing 10% FCS), and screened for expression cell strains. Further, the selected expression cell strains were subjected to the FCM analysis and the western blotting analysis using an anti-hBAI2 antibody to confirm the expression of the introduced gene.

Strains stably expressing splicing variants of the variant 3 gene were prepared by a method similar to the above-mentioned method using a variant 3 gene-expressing vector.

### Example 8

### (Functional analysis of 7tmHR stably-expressing strain and hk01941stably-expressing strain)

Changes in the intracellular calcium (Ca²⁺) concentration induced by CCK-8S were measured by a method similar to the method described in Example 5 using the 7tmHR stably-expressing strain and the hk01941 stably-expressing strain prepared in Example 7. As a control, a host cell not transfected with the 7tmHR-expressing vector or the hk01941-expressing vector was also used.

Cells were seeded in a 96-well plate (a plate with a black wall and a transparent bottom) at a cell count of 3 × 10⁴/100 µL of medium/well and cultured overnight at 37°C in a 5% CO₂ incubator. On the following day, 50 µL of the medium was removed from each well, 50 µL of a loading buffer was added to each well, and the mixture was reacted at room temperature for 1 h, so that a fluorescent dye contained in the loading buffer was taken up into the cell. The loading buffer was prepared by a method similar to the method described in Example 5. After reaction, changes over time in the fluorescence intensity in each well after addition of CCK-8S were measured using the FLEX station (Molecular Device). CCK-8S was used at a final concentration of 10 pM to 1 µM. Furthermore, as a positive control of the assay, A23187 was used at a final concentration of 20 µM.

An increase in the intracellular calcium (Ca²⁺) concentration was observed after addition of CCK-8S in both the 7tmHR stably-expressing strain and the hk01941 stably-expressing strain. On the other hand, no change in the intracellular calcium concentration was observed after addition of CCK-8S in the host cell not transfected with the 7tmHR-expressing vector or the hk01941-expressing vector. Similar trends were confirmed in a plurality of clones of both the stably-expressing strains. Changes in the intracellular calcium (Ca²⁺) concentration after addition of CCK-8S at a physiological concentration (1 nM) in representative clones of the 7tmHR stably-expressing strain and the hk01941 stably-expressing strain are shown in Figures 5-A and 6-A. Furthermore, similar responses to A23187 (20 µM) used as the positive control were observed in its expressing cell strain and the host cell (Figures 5-B and 6-B).

Since changes in the intracellular calcium (Ca²⁺) concentration which were not observed in the host cell after addition of CCK-8S were observed in the 7tmHR stably-expressing strain and the hk01941 stably-expressing strain, it is considered that both the gene products of the 7tmHR gene and the hk01941 gene mediate cellular responses induced by CCK-8S. That is, it is considered that both the gene products of the 7tmHR gene and the hk01941 gene were expressed on the cell membrane surface, and an intracellular signal transduction pathway was activated by the action of extracellular CCK-8S to induce a cellular response which is an increase in the intracellular calcium (Ca²⁺) concentration.

### Example 9

### (Functional analysis of variant 3 gene stably-expressing strain)

To analyze functions of the variant 3 gene, changes in the intracellular calcium (Ca²⁺) concentration induced by CCK-8S were examined using a strain stably expressing the variant 3 gene prepared in Example 7. Changes in the intracellular calcium (Ca²⁺) concentration were measured by a method similar to the method described in Example 5.

Cells were seeded in a 96-well plate (a plate with a black wall and a transparent bottom) at a cell count of 3 × 10⁴/100 µL of medium/well and cultured overnight at 37°C in a 5% CO₂ incubator. On the following day, 50 µL of the medium was removed from each well, 50 µL of a loading buffer was added to each well, the mixture was reacted at room temperature for 1 h, so that a fluorescent dye contained in the loading buffer was taken up into the cell. The loading buffer was prepared by a method similar to the method described in Example 5. After reaction, changes over time in the fluorescence intensity after addition of CCK-8S in each well were measured using the FLEX station (Molecular Device) for 40 sec. CCK-8S was used at a final concentration of 10 pM to 1 µM. Furthermore, as a positive control of the assay, A23187 was used at a final concentration of 20 µM.

In the variant 3 gene stably-expressing strain, an increase in the intracellular calcium (Ca²⁺) concentration was observed after addition of CCK-8S. On the other hand, no change in the intracellular calcium concentration was observed after addition of CCK-8S in the host cell not transfected with the variant 3-expressing vector. Changes in the intracellular calcium (Ca²⁺) concentration in representative clones of the variant 3 stably-expressing strain after addition of CCK-8S at a physiological concentration (1 nM) are shown in Figure 7-A. Furthermore, similar responses to A23187 (20 µM) used as the positive control were observed in its expressing cell strain and the host cell (Figure 7-B).

Since a change in the intracellular calcium (Ca²⁺) concentration which was not observed in the host cell was observed after addition of CCK-8S in the strain stably expressing the variant 3 gene, it is considered that the gene product of the variant 3 gene mediates cellular responses induced by CCK-8S. That is, it is considered that all the gene products of the variant 3 gene were all expressed on the cell membrane surface, and an intracellular signal transduction pathway was activated by the action of extracellular CCK-8S to induce a cellular response which is an increase in the intracellular calcium (Ca²⁺) concentration.

### Example 10

### (Tissue expression of ph01207 gene)

Tissue expression of the ph01207 gene was analyzed by search of the Life Span Drug Target Database^{™} (Life Span Biosciences) and the BioExpress Database (Gene Logic).

In the analysis by search of the Life Span Drug Target Database^{™} (Life Span Biosciences), the tissue immunostaining data obtained using three types of rabbit anti-human BAI2 polyclonal antibodies (LS-A981, A982, A984; Life Span) showed that neurons and astrocytes at each site of the amygdala, the hippocampus, the medulla, the thalamus, substantia nigra, the cerebral cortex, and some cells in the anterior pituitary, and the Herring body of the pituitary posterior lobe were commonly stained strongly. Figures 8-A and 8-B show the tissue immunostaining data using LS-A981 in protoplasmic astrocytes in the amygdala and neurons and glia in the amygdala, respectively. Figures 8-C and 8-D show the tissue immunostaining data using LS-A981 of neurons in the CA2 region and neurons in the CA1 region, respectively, of the hippocampus.

An expression analysis using the Genechip analysis data of BioExpress Database (Gene Logic) was performed for ph01207 (hBAI2), the CCK-A receptor (CCK-AR), the CCK-B receptor (CCK-BR), and CCK. The results are shown in Table 3.

It was shown that the ph01207 (hBAI2) gene was strongly expressed in brain tissues, in particular, localized in the cerebral cortex (the temporal pole of cerebrum, the motor cortex, etc.), the hippocampus, the amygdala, and the like (Table 3). This gene was rarely observed in the gastrointestinal tracts such as, for example, the pancreas, the small intestines, the stomach, and the gallbladder.

On the other hand, virtually no expression of the CCK-A receptor (denoted as CCK-AR in the table) was observed in brain tissues, and it was confirmed that it was expressed in the gastrointestinal tracts such as, for example, the pancreas, the stomach, and the gallbladder (Table 3). Furthermore, it was confirmed that the CCK-B receptor (denoted as CCK-BR in the table) was highly expressed in the pancreas and the small intestines, and also expressed in brain tissues such as, for example, the cerebral cortex, the hippocampus, and the amygdala (Table 3). The expression level of the ph01207 (hBAI2) gene in brain tissues was higher than that of the CCK-B receptor.

The analysis of tissue expression of CCK showed distributions similar to tissue expression of ph01207. Therefore, it was confirmed that ph01207 and CCK were strongly expressed at the same sites (Table 3).

**[Table 3]**

| | | CCK-AR | CCK-BR | ph01207 | | CCK |
|---|---|---|---|---|---|---|
| Brain | Cerebral cortex | 50 > | 100-200 | 300-700 | | 500-1500 |
| | Hippocampus | 50 > | 100 | 500 | | 500 |
| | Amygdala | 50 > | 100 | 500 | | 700 |
| Gastrointestinal tracts | Pancreas | 100 | 200 | 50 > | | 50 > |
| | Small intestines | 50 > | 50 > | 50 > | | 100-500 |
| | Stomach | 100 | 100-400 | 50 > | | 50 > |
| | Gallbladder | 100 | 50 > | 50 > | | 50 > |

### Example 11

The information about functions of the BAI2-knockout mouse was obtained from an information database about functions of knockout mice (Deltagen).

BAI2-knockout mice were prepared by gene disruption by homologous recombination. The gene disruption by homologous recombination was specifically achieved by preparing a vector targeting a region encoding the 876th aspartic acid (D) to the 917th leucine (L) (876D-917L region) in the mouse BAI2 in the nucleotide sequence of the mouse BAI2 gene (which encodes 1560 amino acid residues) and introducing the targeting vector into an ES cell derived from the 129/OlaHsd mouse. The targeting vector contains a LacZ-Neo gene cassette, which contains a genome sequence 0.8 kb upstream of an intron upstream of the 876D-917L region in the mouse BAI2 gene on the 5' end side (5' arm) of the cassette and a genome sequence 1.2 kb downstream of an intron downstream of the 876D-917L region of this gene on the 3' end side (3' arm). A LacZ-Neo fragment is inserted at a target site of the mouse BAI2 gene by homologous recombination using this targeting vector. ES cells were screened for those in which homologous recombination occurred by a selective medium containing G418, and chimeric mice were prepared by a usual method using C57BL/6 mice. First filial generation (F1) heterozygous mutant mice were prepared by mating the obtained chimeric mice and the C57BL/6 mice. Further, second filial generation (F2) homozygous mutant mice were prepared by mating between the F1 heterozygous mutant mice.

The genotypes of the F1 heterozygous mutant mice and the F2 homozygous mutant mice were confirmed by PCR and northern blotting analysis.

Whether a LacZ-Neo fragment was inserted at the target site in the mouse BAI2 gene or not was confirmed by a LacZ expression analysis in the F1 heterozygous mutant mice. As a result, strong expression of LacZ was observed in tissues, in particular, the hippocampus and the amygdala in the F1 heterozygous mutant mice (Figures 9-A and 9-B). From this result, it was confirmed that, a LacZ-Neo fragment was inserted at the target site in the mouse BAI2 gene, that is, the gene was disrupted in the F1 heterozygous mutant mice. This result also suggests that the mouse BAI2 gene was strongly expressed in brain tissues, in particular, the hippocampus and the amygdala in the F1 heterozygous mutant mice.

Functions of the BAI2-knockout mice were examined by behavioral examinations, physiological examinations, pathological examinations, and anatomical examinations.

In the behavioral examinations, the BAI2-knockout mice showed significant differences in the results of the fear conditioning test as compared with the wild-type mice (Figure 10). On the other hand, no significant difference was observed in many examination items such as physiological examinations, pathological examinations, and anatomical examinations as compared with wild-type mice.

A fear conditioning test is a test method in which an aversive stimulus is given to mice, and an immobility state (also referred to as a helplessness state) is measured in a condition without the aversive stimulus as a fear response after receiving the aversive stimulus, and is a technique commonly used as a test method to examine anxiety phenotypes. The fear conditioning test is used as a test system for examining association with an anxiety disorder in evaluation of anxiolytic agents or like (Yoshimizu, T. et al., "Psychopharmacology," 2006, Vol. 186, p. 587-593; Nakamura, K. et al., "European Journal of Pharmacology," 2001, Vol. 420, p. 33-43).

In the fear conditioning test, an anxiety condition is determined when the time showing an immobility state is longer in a condition without an aversive stimulus on the following day after the aversive stimulus was given, and an anti-anxiety condition is determined when the immobility time is shorter.

A fear conditioning test was performed using 10 BAI2-knockout mice. As a control, a fear conditioning test was similarly performed using 10 wild-type mice. All the mice used were 10 to 13 weeks old. Specifically, mice were placed in a feared condition measurement apparatus, and an electrical stimulus (a 0.3-mA electrical stimulus load for 1 sec every 15 sec) was given for 4 min as fear conditioning. On the following day, animals were placed in the faired condition measurement apparatus again, and the immobility time (also referred to as helplessness time) was measured under a condition without the electrical stimulus.

In a fear conditioning test, the immobility time in the BAI2-knockout mice was significantly shortened as compared with the wild-type mice (Figure 10). That is, while the wild-type mice were in a helplessness state from the initial stage of the experiment, no helplessness state was observed in the results of the BAI2-knockout mice. The results were expressed as mean ± standard error of the immobility time in each mouse in the BAI2-knockout mice group and the wild-type mice group. Significant differences were observed in the statistical processing by t test.

It can be interpreted from the results of the fear conditioning test that the BAI2-knockout mice are in a strong anti-anxiety condition. Since the BAI2-knockout mice show an anti-anxiety phenotype, the BAI2 gene appears to be associated with an anxiety condition and an anxiety disorder.

The BAI2 gene and splicing variants thereof are not expressed in the BAI2-knockout mice because the BAI2 gene is disrupted. Therefore, it is considered that not only the BAI2 gene, but splicing variants thereof are also involved in an anxiety condition and an anxiety disorder.

### Example 12

A compound that inhibits a response of the ph01207 gene product to a ligand was identified by an intracellular calcium concentration change assay system using the ph01207-expressing cell strain.

The ph01207-expressing cell strain was constructed as follows. First, a ph01207-expressing vector not containing an epitope tag was constructed by recombination between a ph01207 cDNA clone identified in Example 1 and pcDNA3.1 (Invitrogen) with Asp718I (Boehringer) and NotI (TAKARA). The ph01207-expressing vector was transfected into the CHO-K1 strain, a host cell, using Lipofectamine 2000 (Invitrogen), and the cells were screened for stably-expressing strains by a selective medium containing G418. Expression of the gene introduced into the cell was detected by the FCM analysis using an anti-ph01207 antibody.

As a result, a plurality of cell strains stably expressing ph01207 were obtained. A compound was identified using the ph01207#3F8-17 strain, a clone of these strains.

As a ligand, CCK-8S (Peptide Institute, Inc.) was used. 0.52 mg of CCK-8S (SEQ ID NO: 14) was dissolved in 4.5 mL of 1% NaHCO₃ (Wako) to prepare solutions at a concentration of 0.1 mM. As test compounds, the Soft Focus GPCR Target-Directed Library (BioFocus), a compound library, was used. 2 mg/mL DMSO solutions of compounds were all diluted 25-fold with PBS to adjust to a concentration of 80 µg/mL and used.

Compounds were specifically identified as follows. The ph01207#3F8-17 strain was seeded in a 96-well plate (with a black wall and a transparent bottom) at a cell count of 3 × 10⁴/100 µL of medium/well and cultured at 37°C in the presence of 5% CO₂. As a medium, DMEM/F12 (Gibco) containing 10% fetal calf serum (Moregate) was used. On the following day, 20 µL of 6X loading buffer was added to each well, and the mixture was reacted at 37°C for 1 h, so that a fluorescent dye contained in the loading buffer was taken up into the cell. The 6X loading buffer was prepared by dissolving component A in component B in the FLIPR Calcium 3 Assay Kit (Molecular Device) and adding probenecid (Sigma) at a final concentration of 15 mM. After reaction, CCK-8S (Peptide Institute, Inc.) and a compound was added, and changes over time in the fluorescence intensity in each well were measured for 80 sec. As a control, similar measurement was performed using a buffer instead of the compound. At 15 sec after the start of measurement, the compound or the buffer was added at a final concentration of 10 µg/mL, and 35 sec after addition of the compound or the buffer, CCK-8S was added at a final concentration of 10 nM. Changes over time in the fluorescence intensity were measured using the FLEX station (Molecular Device).

Of the compounds examined, several compounds inhibited a response of the ph01207#3F8-17 strain to CCK-8S. As representative examples, responses of the ph01207#3F8-17 strain to CCK-8S after addition of three compounds (compounds A, B, and C) are shown in Figures 11-A, 11-B, and Figure 11-C. Compounds A, B, and C are compounds represented by the above-mentioned formulas (I), (II), and (III), respectively.

When compound A or a buffer was added at 15 sec after the start of measurement, the fluorescence intensity did not change. When CCK-8S, a ligand, was added at 35 sec after addition of compound A or the buffer, the fluorescence intensity increased in wells to which the buffer was added, and a response of the ph01207#3F8-17 strain to the ligand was observed, but such a response was inhibited in wells to which compound A was added (Figure 11-A). This result suggests that compound A acts as an inhibitor of an interaction between CCK-8S and ph01207.

Compounds B and C inhibited a response of the ph01207#3F8-17 strain to a ligand as with compound A (Figures 11-B and 11-C). This suggests that both compounds B and C act as an inhibitor of an interaction between CCK-8S and ph01207.

The above results suggest that a compound that inhibits a response of ph01207 to a ligand thereof, for example, CCK-8S, can be identified by the intracellular calcium concentration change assay system using a ph01207-expressing cell strain.

### Industrial Applicability

According to the present invention, a method for identifying a compound having an anti-anxiety effect, an inhibitor of a function of a protein acting as a functional membrane protein receptor having a seven-transmembrane domain that appears to be a GPCR, can be provided. Furthermore, an agent and a method for improving an anxiety condition and a method and an agent for prophylactic and/or therapeutic treatment of an anxiety disorder can be provided. Furthermore, a measurement method for diagnosis and a diagnostic method of an anxiety disorder can be provided.

Thus, the present invention is useful and contributes to a wide range of fields from basic science to pharmaceutical development.

Sequence listing free text

### SEQ ID NO: 1: DNA encoding a functional membrane protein receptor.

SEQ ID NO: 2: Protein encoded by the DNA represented by the nucleotide sequence of SEQ ID NO: 1.
SEQ ID NO: 2: (297):(350) TSP-I domain.
SEQ ID NO: 2: (352):(405) TSP-I domain.
SEQ ID NO: 2: (408):(461) TSP-I domain.
SEQ ID NO: 2: (870):(890) Transmembrane domain.
SEQ ID NO: 2: (899):(919) Transmembrane domain.
SEQ ID NO: 2: (928):(948) Transmembrane domain.
SEQ ID NO: 2: (970):(990) Transmembrane domain.
SEQ ID NO: 2: (1012):(1032) Transmembrane domain.
SEQ ID NO: 2: (1087):(1107) Transmembrane domain.
SEQ ID NO: 2: (1114):(1134) Transmembrane domain.
SEQ ID NO: 3: Partial amino acid sequence existing in a region on the C terminus side in the peptide represented by the amino acid sequence of SEQ ID NO: 2, hBAI1, and hBAI2.
SEQ ID NO: 4: Oligonucleotide designed for primer.
SEQ ID NO: 5: Oligonucleotide designed for primer.
SEQ ID NO: 6: Oligonucleotide designed for primer.
SEQ ID NO: 7: Oligonucleotide designed for primer.
SEQ ID NO: 8: Oligonucleotide designed for primer.
SEQ ID NO: 9: Oligonucleotide designed for primer.
SEQ ID NO: 10: Oligonucleotide designed for primer.
SEQ ID NO: 11: Oligonucleotide designed for primer.
SEQ ID NO: 12: Synthetic oligonucleotide.
SEQ ID NO: 13: Synthetic oligonucleotide.
SEQ ID NO: 14: CCK-8S.
SEQ ID NO: 14: (2):(2) Sulfated.
SEQ ID NO: 15: Splicing variant of DNA of SEQ ID NO: 1.
SEQ ID NO: 16: Protein encoded by the DNA represented by the nucleotide sequence of SEQ ID NO: 15.
SEQ ID NO: 16: (297):(350) TSP-I domain.
SEQ ID NO: 16: (353):(406) TSP-I domain.
SEQ ID NO: 16: (815):(835) Transmembrane domain.
SEQ ID NO: 16: (844):(864) Transmembrane domain.
SEQ ID NO: 16: (873):(893) Transmembrane domain.
SEQ ID NO: 16: (915):(935) Transmembrane domain.
SEQ ID NO: 16: (957):(977) Transmembrane domain.
SEQ ID NO: 16: (1032):(1052) Transmembrane domain.
SEQ ID NO: 16: (1059):(1079) Transmembrane domain.
SEQ ID NO: 17: Splicing variant of the DNA of SEQ ID NO: 1.
SEQ ID NO: 18: Protein encoded by the DNA represented by the nucleotide sequence of SEQ ID NO: 17.
SEQ ID NO: 18: (297):(350) TSP-I domain.
SEQ ID NO: 18: (352):(405) TSP-I domain.
SEQ ID NO: 18: (408):(461) TSP-I domain.
SEQ ID NO: 18: (870):(890) Transmembrane domain.
SEQ ID NO: 18: (899):(919) Transmembrane domain.
SEQ ID NO: 18: (928):(948) Transmembrane domain.
SEQ ID NO: 18: (970):(990) Transmembrane domain.
SEQ ID NO: 18: (1012):(1032) Transmembrane domain.
SEQ ID NO: 18: (1087):(1107) Transmembrane domain.
SEQ ID NO: 18: (1114):(1134) Transmembrane domain.
SEQ ID NO: 19: Splicing variant of the DNA of SEQ ID NO: 1.
SEQ ID NO: 20: Protein encoded by the DNA represented by the nucleotide sequence of SEQ ID NO: 19.
SEQ ID NO: 20: (297):(350) TSP-I domain.
SEQ ID NO: 20: (352):(405) TSP-I domain.
SEQ ID NO: 20: (407):(460) TSP-I domain.
SEQ ID NO: 20: (463):(516) TSP-I domain.
SEQ ID NO: 20: (925):(945) Transmembrane domain.
SEQ ID NO: 20: (954):(974) Transmembrane domain.
SEQ ID NO: 20: (983):(1003) Transmembrane domain.
SEQ ID NO: 20: (1025):(1045) Transmembrane domain.
SEQ ID NO: 20: (1067):(1087) Transmembrane domain.
SEQ ID NO: 20: (1142):(1162) Transmembrane domain.
SEQ ID NO: 20: (1169):(1189) Transmembrane domain.
SEQ ID NO: 21: DNA encoding hBAI2 which is a splicing variant of the DNA of SEQ ID NO: 1.
SEQ ID NO: 22: Protein encoded by the DNA represented by the nucleotide sequence of SEQ ID NO: 21.
SEQ ID NO: 22: (297):(350) TSP-I domain.
SEQ ID NO: 22: (352):(405) TSP-I domain.
SEQ ID NO: 22: (407):(460) TSP-I domain.
SEQ ID NO: 22: (463):(516) TSP-I domain.
SEQ ID NO: 22: (925):(945) Transmembrane domain.
SEQ ID NO: 22: (954):(974) Transmembrane domain.
SEQ ID NO: 22: (983):(1003) Transmembrane domain.
SEQ ID NO: 22: (1025):(1045) Transmembrane domain.
SEQ ID NO: 22: (1067):(1087) Transmembrane domain.
SEQ ID NO: 22: (1142):(1162) Transmembrane domain.
SEQ ID NO: 22: (1169):(1189) Transmembrane domain.
SEQ ID NO: 23: Oligonucleotide designed as primer.
SEQ ID NO: 24: Oligonucleotide designed as primer.

## Claims

1. A method for identifying a compound having an anti-anxiety effect, an inhibitor of a function of a protein encoded by a DNA represented by the nucleotide sequence of SEQ ID NO: 1 in the sequence listing or a homologue protein thereof, **characterized by** using at least one selected from the group consisting of the DNA represented by the nucleotide sequence of SEQ ID NO: 1 in the sequence listing or a homologue DNA thereof, a protein encoded by the DNA, and a cell containing the DNA.

2. A method for identifying a compound having an anti-anxiety effect, an inhibitor of a function of a protein encoded by a DNA represented by the nucleotide sequence of SEQ ID NO: 1 in the sequence listing or a splicing variant of the protein, **characterized by** using at least one selected from the group consisting of a DNA having any one nucleotide sequence of the DNA represented by the nucleotide sequence of SEQ ID NO: 1 in the sequence listing and a splicing variant thereof, a protein encoded by the DNA, and a cell containing the DNA.

3. A method for identifying a compound having an anti-anxiety effect, an inhibitor of a function of a protein encoded by a DNA represented by the nucleotide sequence of SEQ ID NO: 1 in the sequence listing or a splicing variant of the protein, comprising bringing a cell containing a DNA represented by any one nucleotide sequence of the nucleotide sequences of SEQ ID NOS: 1, 15, 17, 19, and 21 in the sequence listing into contact with a test compound, measuring a function of a protein expressed on the cell membrane of the cell, and determining a test compound that decreases or eliminates the function of the protein as compared with when the cell and the test compound are not brought into contact with each other as a compound having an anti-anxiety effect.

4. The method according to claim 3, wherein the function of the protein expressed on the cell membrane of the cell is a function of inducing an increase in the intracellular calcium concentration by addition of a ligand of the protein.

5. The method according to claim 3, wherein the function of the protein expressed on the cell membrane of the cell is a function of inducing a change in the membrane potential by addition of a ligand of the protein.

6. The method according to claim 4 or 5, wherein the ligand used is a peptide selected from the group of the following:
(i) cholecystokinin octapeptide sulfated form (SEQ ID NO: 14, hereinafter, referred to as CCK-8S);
(ii) a peptide having a mutation such as deletion, substitution, or addition of one or several amino acids in the amino acid sequence of CCK-8S and having a function identical to that of CCK-8S; and
(iii) a peptide comprising the amino acid sequence of the peptide of the (i) or (ii) and having a function identical to that of CCK-8S.

7. The method according to any one of claims 1 to 6, further comprising evaluating an anti-anxiety effect of a compound in an experimental system using a nonhuman animal for measuring the anxiety condition.

8. The method according to claim 7, wherein the anti-anxiety effect of a compound in an experimental system using a nonhuman animal for measuring the anxiety condition is selected from the group consisting of fear conditioning tests, Vogel anti-conflict tests, Geller-Seifter anti-conflict tests, elevated maze tests, hole-board tests, light-dark tests, social interaction tests, and marble-burying tests.

9. A method for improving an anxiety condition, comprising inhibiting a function and/or the expression of a protein encoded by a DNA represented by the nucleotide sequence of SEQ ID NO: 1 in the sequence listing or a splicing variant of the protein.

10. A method for prophylactic and/or therapeutic treatment of an anxiety disorder, comprising using the method for improving an anxiety condition according to claim 9.

11. A method for measurement used for diagnosis of an anxiety disorder, comprising a quantitative or qualitative analysis using at least one DNA selected from a DNA represented by the nucleotide sequence of SEQ ID NO: 1 in the sequence listing and a splicing variant of the DNA and/or a protein encoded by the DNA as a marker.

12. A method for diagnosing an anxiety disorder, comprising a quantitative or qualitative analysis using at least one DNA selected from a DNA represented by the nucleotide sequence of SEQ ID NO: 1 in the sequence listing and a splicing variant of the DNA and/or a protein encoded by the DNA as a marker.


SEQUENCE LISTING

<110> DAIICHI SANKYO COMPANY, LIMITED
KAZUSA DNA RESEARCH INSTITUTE FOUNDATION

<120> Method for identification of compound having antianxiety effect

<130> 535-9

<140> EP 07 831 051.3
<141> 2007-11-01

<150> JP 2006-298814
<151> 2006-11-02

<160> 24

<170> PatentIn version 3.1

<210> 1
<211> 4557
<212> DNA
<213> homo sapiens

<220>
<221> misc_feature
<223> DNA encoding a novel functional membrane protein receptor

<400> 1
atgaccccag cctgtcccct cttactgtct gtgattctgt ccctgcgcct ggccaccgcc 60

ttcgaccccg cccccagtgc ctgctctgcc ctggcctcgg gtgtgctcta cggggccttc 120

tcgctgcagg acctctttcc taccatcgcc tcgggctgct cctggaccct ggagaaccct 180

gaccccacca agtactccct ctacctgcgc ttcaaccgcc aggagcaggt gtgcgcacac 240

tttgcccccc gcctgctgcc cctggaccac tacctggtca actttacctg cctgcggcct 300

agccccgagg aggcggtggc ccaggcggag tcagaggtgg ggcggccaga agaggaggag 360

gcagaggcgg cagcggggtt ggagctgtgc agcggctcag gcccctttac cttcctgcac 420

ttcgacaaga acttcgtgca gctgtgcctg tcggctgagc cctccgaggc cccgcgcctg 480

ctggcgcccg ctgccctagc cttccgcttt gtcgaggtct tgctcatcaa caacaacaac 540

tctagccaat tcacctgtgg tgtgctctgc cgctggagtg aggagtgtgg ccgcgctgcc 600

ggcagggcct gcggctttgc tcagccaggc tgcagctgcc ctggagaggc gggggccggc 660

tccaccacca ccacatctcc aggccctcct gctgcccaca ccctgtccaa tgccctggtg 720

cccgggggcc cagccccacc tgctgaggcc gatttgcact cggggagcag caatgatctg 780

ttcacaaccg agatgagata tggtgaggag ccggaagagg aaccgaaagt gaaaacccag 840

tggccgaggt ctgcagatga gcctgggcta tacatggcgc agacagtgca cggcgtgtgg 900

gaggagtggg ggtcctggag cctgtgctcc cgcagctgcg ggcgggggtc ccggagccgg 960

atgcggacct gcgtgccccc ccagcacggc ggcaaggcct gcgagggtcc tgagctgcag 1020

actaagctct gcagtatggc tgcctgcccg gtggaaggcc agtggttaga atggggtccc 1080

tggggcccat gctccacgtc ctgtgccaat gggacccaac agcgcagccg gaagtgcagc 1140

gtggcgggcc cagcctgggc cacatgcacg ggtgccctca ctgacacccg ggagtgcagc 1200
aacctcgagt gcccggccac tgatagcaag tgggggccat ggaatgcgtg gagcctgtgc 1260
tctaagacgt gtgacacagg ctggcagcgc cgcttccgca tgtgccaggc cacgggcacg 1320
cagggctacc cctgcgaggg caccggagag gaggtgaagc cttgtagtga gaagaggtgt 1380
ccagccttcc atgagatgtg cagggatgag tacgtgatgc tgatgacgtg gaagaaggca 1440
gctgctggcg agatcatcta caacaagtgc cccccgaatg cctcagggtc tgccagccgc 1500
cgctgtctcc tcagtgccca aggcgtggcg tactgggggc tgcccagctt tgctcgctgc 1560
atctcccatg agtaccgcta cctgtatctg tcacttaggg agcacctggc caaggggcag 1620
cgcatgctgg caggcgaggg catgtcgcag gtggtgcgca gcctgcagga gctactggcc 1680
cggcgcacct actatagtgg ggacctgctc ttctctgtgg acattctgag gaatgtcact 1740
gacaccttta agagggccac ctacgtgccc tcggctgatg atgtgcagcg cttcttccag 1800
gtggtgagct tcatggtgga tgcggaaaac aaggagaagt gggacgatgc tcagcaggtg 1860
tcccctggct ctgtgcacct gctccgtgtc gtggaggact tcattcacct ggtgggcgat 1920
gctctcaagg ccttccagag ctctctgatt gtcacagata atctagtgat cagcattcag 1980
cgagagcccg tctcagctgt gtccagtgac atcacgttcc ccatgcgggg ccgccggggc 2040
atgaaggact gggtgcggca ctcagaggac cgcctcttcc tgcccaagga ggtgctcagc 2100
ctctcctccc cagggaagcc agccacatct ggggcagcag gcagccctgg cagggggagg 2160
ggcccaggaa cggtgcctcc tggcccaggc cactcccacc agcgcctcct cccagcagac 2220
cctgatgagt cctcctactt tgtgatcggt gctgtactct accgcaccct tggcctcatc 2280
ctgccgcctc ccaggccccc gctggccgtc acatcccggg tgatgacagt gactgtgcgc 2340
ccccctaccc agcctccagc tgagcccctc atcactgtgg agctctccta catcatcaat 2400
gggaccacgg atccccattg cgccagctgg gactactcca gagcagatgc cagctcagga 2460
gactgggaca ctgaaaattg ccagaccctg gagacccagg cagctcacac ccgctgccag 2520
tgccagcacc tgtccacctt tgctgtacta gcccagccgc ccaaggacct gaccctggag 2580
ctggcgggct ccccctcggt ccccctggtg atcggctgtg cagtgtcgtg catggcgctg 2640
ctcaccctgc tcgccatcta tgccgccttt tggaggttca taaaatctga acgctccatc 2700
atcttgctga acttctgcct gtccatcttg gcatccaaca tcctgatcct cgtgggccag 2760
tcccgggtgc tgagcaaggg cgtgtgcacc atgacggctg ccttcctgca cttcttcttt 2820
ctctcctcct tttgctgggt gcttaccgag gcctggcagt cctacctggc tgtcattggg 2880
cggatgcgca cccgcctcgt tcgcaagcgc ttcctctgcc tgggctgggg tctgcctgcc 2940
ctggtggtgg ccgtgtctgt tggctttacc cgaacgaaag gatacggtac atccagctac 3000
tgctggctct ccctggaggg cggcctgctc tacgcctttg tgggccctgc agccgtcatt 3060
gtcctggtga acatgctcat cggaatcatc gtcttcaaca agctcatggc acgtgatggc 3120
atctccgaca aatccaagaa gcagagggcc gggtcggagc ggtgcccctg ggccagcctg 3180

ctcctcccct gctcagcgtg tggagcggtc cccagccccc tgctcagctc agcctcggcc 3240

aggaacgcca tggcctcact ctggagctcc tgcgtggtgc tgcccctgct ggcgctcacc 3300

tggatgtctg ccgtcctggc tatgacagac cgccgttccg tcctcttcca ggccctcttt 3360

gctgtcttca actccgcgca gggctttgtc atcactgctg tgcactgctt cctgcgccga 3420

gaggtccagg atgtggtgaa gtgccagatg ggggtgtgcc gggctgatga gagcgaagac 3480

tcccctgact cgtgtaagaa cgggcagctg cagatcctgt cagactttga aaaggatgtg 3540

gatctggctt gtcaaacagt gctgttcaag gaggtcaaca cttgcaaccc gtccaccatc 3600

acgggcacac tatcccgcct gtccctggat gaggatgagg agcccaagtc ctgcctcgtg 3660

ggccctgagg gcagcctcag cttctcacca ctgcctggga atatcctggt gcccatggca 3720

gcctcaccag ggctggggga gcctccgccc ccacaggagg ccaaccctgt ttacatgtgt 3780

ggggagggtg gcctgcggca gctggacctc acatggctgc ggcccactga gccaggctct 3840

gagggagact acatggtgct gccccggcgg actttgagcc tgcagcctgg cggtgggggt 3900

ggaggtggtg aggatgcccc cagggcccgg ccggagggga ccccccggcg agctgccaag 3960

acagtggccc acactgaagg ctaccccagc ttcctgtccg tggaccactc gggcctgggg 4020

ctgggccctg cctatggatc tctccagaat ccctatggaa tgaccttcca accgccaccg 4080

ccgacaccca gcgcccgcca agtgcccgag ccaggggagc gcagccggac catgcctcgc 4140

accgtgcccg gctctaccat gaagatgggc tccctggagc gaaagaaatt acggtattca 4200

gacctggact ttgagaaggt gatgcacacc cggaaacggc attcagaact ctaccacgag 4260

ctcaaccaga agttccacac tttcgaccgc taccgcagcc agtccacggc caagagggag 4320

aagcggtgga gtgtgtcctc gggtggggca gccgagcgga gcgtgtgcac cgataagccc 4380

agccctgggg agcgccccag cttgtcccaa catcggcgcc atcagagctg gagcaccttc 4440

aaatctatga cactgggctc gctgcccccc aagccccgag aacggctgac tctgcaccgg 4500

gcagcagcct gggagcccac agaaccaccg gatggtgact tccagacaga ggtgtga 4557

<210> 2
<211> 1518
<212> PRT
<213> homo sapiens

<220>
<221> MISC_FEATURE
<223> Protein encoded by the DNA having a nucleotide sequence of SEQ ID
 NO:1

<220>
<221> DOMAIN
<222> (297)..(350)
<223> TSP-I domain

<220>
<221> DOMAIN
<222> (352)..(405)
<223> TSP-I domain

<220>
<221>  DOMAIN
<222>  (408)..(461)
<223>  TSP-I domain


<220>
<221>  TRANSMEM
<222>  (870)..(890)
<223>  transmembrane domain


<220>
<221>  TRANSMEM
<222>  (899)..(919)
<223>  transmembrane domain


<220>
<221>  TRANSMEM
<222>  (928)..(948)
<223>  transmembrane domain


<220>
<221>  TRANSMEM
<222>  (970)..(990)
<223>  transmembrane domain


<220>
<221>  TRANSMEM
<222>  (1012)..(1032)
<223>  transmembrane domain


<220>
<221>  TRANSMEM
<222>  (1087)..(1107)
<223>  transmembrane domain


<220>
<221>  TRANSMEM
<222>  (1114)..(1134)
<223>  transmembrane domain


<400>  2

Met Thr Pro Ala Cys Pro Leu Leu Leu Ser Val Ile Leu Ser Leu Arg
1               5                   10                  15


Leu Ala Thr Ala Phe Asp Pro Ala Pro Ser Ala Cys Ser Ala Leu Ala
            20                  25                  30


Ser Gly Val Leu Tyr Gly Ala Phe Ser Leu Gln Asp Leu Phe Pro Thr
        35                  40                  45


Ile Ala Ser Gly Cys Ser Trp Thr Leu Glu Asn Pro Asp Pro Thr Lys
    50                  55                  60

Tyr Ser Leu Tyr Leu Arg Phe Asn Arg Gln Glu Gln Val Cys Ala His
65                  70                  75                  80

Phe Ala Pro Arg Leu Leu Pro Leu Asp His Tyr Leu Val Asn Phe Thr
                85                  90                  95

Cys Leu Arg Pro Ser Pro Glu Glu Ala Val Ala Gln Ala Glu Ser Glu
            100                 105                 110

Val Gly Arg Pro Glu Glu Glu Glu Ala Glu Ala Ala Ala Gly Leu Glu
        115                 120                 125

Leu Cys Ser Gly Ser Gly Pro Phe Thr Phe Leu His Phe Asp Lys Asn
    130                 135                 140

Phe Val Gln Leu Cys Leu Ser Ala Glu Pro Ser Glu Ala Pro Arg Leu
145                 150                 155                 160

Leu Ala Pro Ala Ala Leu Ala Phe Arg Phe Val Glu Val Leu Leu Ile
                165                 170                 175

Asn Asn Asn Asn Ser Ser Gln Phe Thr Cys Gly Val Leu Cys Arg Trp
            180                 185                 190

Ser Glu Glu Cys Gly Arg Ala Ala Gly Arg Ala Cys Gly Phe Ala Gln
        195                 200                 205

Pro Gly Cys Ser Cys Pro Gly Glu Ala Gly Ala Gly Ser Thr Thr Thr
    210                 215                 220

Thr Ser Pro Gly Pro Pro Ala Ala His Thr Leu Ser Asn Ala Leu Val
225                 230                 235                 240

Pro Gly Gly Pro Ala Pro Pro Ala Glu Ala Asp Leu His Ser Gly Ser
                245                 250                 255

Ser Asn Asp Leu Phe Thr Thr Glu Met Arg Tyr Gly Glu Glu Pro Glu
            260                 265                 270

Glu Glu Pro Lys Val Lys Thr Gln Trp Pro Arg Ser Ala Asp Glu Pro
        275                 280                 285

Gly Leu Tyr Met Ala Gln Thr Val His Gly Val Trp Glu Glu Trp Gly
    290                 295                 300

Ser Trp Ser Leu Cys Ser Arg Ser Cys Gly Arg Gly Ser Arg Ser Arg
305                 310                 315                 320

Met Arg Thr Cys Val Pro Pro Gln His Gly Gly Lys Ala Cys Glu Gly
                325                 330                 335

Pro Glu Leu Gln Thr Lys Leu Cys Ser Met Ala Ala Cys Pro Val Glu
            340                 345                 350

Gly Gln Trp Leu Glu Trp Gly Pro Trp Gly Pro Cys Ser Thr Ser Cys
        355                 360                 365

Ala Asn Gly Thr Gln Gln Arg Ser Arg Lys Cys Ser Val Ala Gly Pro
    370                 375                 380

Ala Trp Ala Thr Cys Thr Gly Ala Leu Thr Asp Thr Arg Glu Cys Ser
385                 390                 395                 400

Asn Leu Glu Cys Pro Ala Thr Asp Ser Lys Trp Gly Pro Trp Asn Ala
                405                 410                 415

Trp Ser Leu Cys Ser Lys Thr Cys Asp Thr Gly Trp Gln Arg Arg Phe
            420                 425                 430

Arg Met Cys Gln Ala Thr Gly Thr Gln Gly Tyr Pro Cys Glu Gly Thr
        435                 440                 445

Gly Glu Glu Val Lys Pro Cys Ser Glu Lys Arg Cys Pro Ala Phe His
    450                 455                 460

Glu Met Cys Arg Asp Glu Tyr Val Met Leu Met Thr Trp Lys Lys Ala
465                 470                 475                 480

Ala Ala Gly Glu Ile Ile Tyr Asn Lys Cys Pro Pro Asn Ala Ser Gly
                485                 490                 495

Ser Ala Ser Arg Arg Cys Leu Leu Ser Ala Gln Gly Val Ala Tyr Trp
            500                 505                 510

Gly Leu Pro Ser Phe Ala Arg Cys Ile Ser His Glu Tyr Arg Tyr Leu
        515                 520                 525

Tyr Leu Ser Leu Arg Glu His Leu Ala Lys Gly Gln Arg Met Leu Ala
    530                 535                 540

Gly Glu Gly Met Ser Gln Val Val Arg Ser Leu Gln Glu Leu Leu Ala
545                 550                 555                 560

Arg Arg Thr Tyr Tyr Ser Gly Asp Leu Leu Phe Ser Val Asp Ile Leu
                565                 570                 575

Arg Asn Val Thr Asp Thr Phe Lys Arg Ala Thr Tyr Val Pro Ser Ala
            580                 585                 590

Asp Asp Val Gln Arg Phe Phe Gln Val Val Ser Phe Met Val Asp Ala
        595                 600                 605

Glu Asn Lys Glu Lys Trp Asp Asp Ala Gln Gln Val Ser Pro Gly Ser
    610                 615                 620

Val His Leu Leu Arg Val Val Glu Asp Phe Ile His Leu Val Gly Asp
625                 630                 635                 640

Ala Leu Lys Ala Phe Gln Ser Ser Leu Ile Val Thr Asp Asn Leu Val
                645                 650                 655

Ile Ser Ile Gln Arg Glu Pro Val Ser Ala Val Ser Ser Asp Ile Thr
            660                 665                 670

Phe Pro Met Arg Gly Arg Arg Gly Met Lys Asp Trp Val Arg His Ser
        675                 680                 685

Glu Asp Arg Leu Phe Leu Pro Lys Glu Val Leu Ser Leu Ser Ser Pro
    690                 695                 700

Gly Lys Pro Ala Thr Ser Gly Ala Ala Gly Ser Pro Gly Arg Gly Arg
705                 710                 715                 720

Gly Pro Gly Thr Val Pro Pro Gly Pro Gly His Ser His Gln Arg Leu
                725                 730                 735

Leu Pro Ala Asp Pro Asp Glu Ser Ser Tyr Phe Val Ile Gly Ala Val
            740                 745                 750

Leu Tyr Arg Thr Leu Gly Leu Ile Leu Pro Pro Pro Arg Pro Pro Leu
        755                 760                 765

Ala Val Thr Ser Arg Val Met Thr Val Thr Val Arg Pro Pro Thr Gln
    770                 775                 780

Pro Pro Ala Glu Pro Leu Ile Thr Val Glu Leu Ser Tyr Ile Ile Asn
785                 790                 795                 800

Gly Thr Thr Asp Pro His Cys Ala Ser Trp Asp Tyr Ser Arg Ala Asp
                805                 810                 815

Ala Ser Ser Gly Asp Trp Asp Thr Glu Asn Cys Gln Thr Leu Glu Thr
            820                 825                 830

Gln Ala Ala His Thr Arg Cys Gln Cys Gln His Leu Ser Thr Phe Ala
        835                 840                 845

Val Leu Ala Gln Pro Pro Lys Asp Leu Thr Leu Glu Leu Ala Gly Ser
    850                 855                 860

Pro Ser Val Pro Leu Val Ile Gly Cys Ala Val Ser Cys Met Ala Leu
865                 870                 875                 880

Leu Thr Leu Leu Ala Ile Tyr Ala Ala Phe Trp Arg Phe Ile Lys Ser
                885                 890                 895

Glu Arg Ser Ile Ile Leu Leu Asn Phe Cys Leu Ser Ile Leu Ala Ser
            900                 905                 910

Asn Ile Leu Ile Leu Val Gly Gln Ser Arg Val Leu Ser Lys Gly Val
        915                 920                 925

Cys Thr Met Thr Ala Ala Phe Leu His Phe Phe Phe Leu Ser Ser Phe
    930                 935                 940

Cys Trp Val Leu Thr Glu Ala Trp Gln Ser Tyr Leu Ala Val Ile Gly
945                 950                 955                 960

Arg Met Arg Thr Arg Leu Val Arg Lys Arg Phe Leu Cys Leu Gly Trp
                965                 970                 975

Gly Leu Pro Ala Leu Val Val Ala Val Ser Val Gly Phe Thr Arg Thr
            980                 985                 990

Lys Gly Tyr Gly Thr Ser Ser Tyr  Cys Trp Leu Ser Leu  Glu Gly Gly
        995                 1000                 1005

Leu Leu  Tyr Ala Phe Val Gly  Pro Ala Ala Val Ile  Val Leu Val
    1010                 1015                 1020

Asn Met  Leu Ile Gly Ile Ile  Val Phe Asn Lys Leu  Met Ala Arg
    1025                 1030                 1035

Asp Gly  Ile Ser Asp Lys Ser  Lys Lys Gln Arg Ala  Gly Ser Glu
    1040                 1045                 1050

Arg Cys  Pro Trp Ala Ser Leu  Leu Leu Pro Cys Ser  Ala Cys Gly
    1055                 1060                 1065

Ala Val  Pro Ser Pro Leu Leu  Ser Ser Ala Ser Ala  Arg Asn Ala
    1070                 1075                 1080

Met Ala  Ser Leu Trp Ser Ser  Cys Val Val Leu Pro  Leu Leu Ala
    1085                 1090                 1095

Leu Thr  Trp Met Ser Ala Val  Leu Ala Met Thr Asp  Arg Arg Ser
    1100                 1105                 1110

Val Leu  Phe Gln Ala Leu Phe  Ala Val Phe Asn Ser  Ala Gln Gly
    1115                 1120                 1125

Phe Val  Ile Thr Ala Val His  Cys Phe Leu Arg Arg  Glu Val Gln
    1130                 1135                 1140

Asp Val Val Lys Cys Gln Met Gly Val Cys Arg Ala Asp Glu Ser
    1145                1150                1155

Glu Asp Ser Pro Asp Ser Cys Lys Asn Gly Gln Leu Gln Ile Leu
    1160                1165                1170

Ser Asp Phe Glu Lys Asp Val Asp Leu Ala Cys Gln Thr Val Leu
    1175                1180                1185

Phe Lys Glu Val Asn Thr Cys Asn Pro Ser Thr Ile Thr Gly Thr
    1190                1195                1200

Leu Ser Arg Leu Ser Leu Asp Glu Asp Glu Glu Pro Lys Ser Cys
    1205                1210                1215

Leu Val Gly Pro Glu Gly Ser Leu Ser Phe Ser Pro Leu Pro Gly
    1220                1225                1230

Asn Ile Leu Val Pro Met Ala Ala Ser Pro Gly Leu Gly Glu Pro
    1235                1240                1245

Pro Pro Pro Gln Glu Ala Asn Pro Val Tyr Met Cys Gly Glu Gly
    1250                1255                1260

Gly Leu Arg Gln Leu Asp Leu Thr Trp Leu Arg Pro Thr Glu Pro
    1265                1270                1275

Gly Ser Glu Gly Asp Tyr Met Val Leu Pro Arg Arg Thr Leu Ser
    1280                1285                1290

Leu Gln Pro Gly Gly Gly Gly Gly Gly Gly Glu Asp Ala Pro Arg
    1295                1300                1305

Ala Arg Pro Glu Gly Thr Pro Arg Arg Ala Ala Lys Thr Val Ala
    1310                1315                1320

His Thr Glu Gly Tyr Pro Ser Phe Leu Ser Val Asp His Ser Gly
    1325                1330                1335

Leu Gly Leu Gly Pro Ala Tyr Gly Ser Leu Gln Asn Pro Tyr Gly
    1340                1345                1350

Met Thr Phe Gln Pro Pro Pro Pro Thr Pro Ser Ala Arg Gln Val
    1355                1360                1365

Pro Glu Pro Gly Glu Arg Ser Arg Thr Met Pro Arg Thr Val Pro
    1370                1375                1380

Gly Ser Thr Met Lys Met Gly Ser Leu Glu Arg Lys Lys Leu Arg
    1385                1390                1395

Tyr Ser Asp Leu Asp Phe Glu Lys Val Met His Thr Arg Lys Arg
    1400                1405                1410

His Ser Glu Leu Tyr His Glu Leu Asn Gln Lys Phe His Thr Phe
    1415                1420                1425

Asp Arg Tyr Arg Ser Gln Ser Thr Ala Lys Arg Glu Lys Arg Trp
    1430                1435                1440

Ser Val Ser Ser Gly Gly Ala Ala Glu Arg Ser Val Cys Thr Asp
    1445                1450                1455

Lys Pro Ser Pro Gly Glu Arg Pro Ser Leu Ser Gln His Arg Arg
    1460                1465                1470

His Gln Ser Trp Ser Thr Phe Lys Ser Met Thr Leu Gly Ser Leu
    1475                1480                1485

Pro Pro Lys Pro Arg Glu Arg Leu Thr Leu His Arg Ala Ala Ala
    1490                1495                1500

Trp Glu Pro Thr Glu Pro Pro Asp Gly Asp Phe Gln Thr Glu Val
    1505                1510                1515

<210> 3
<211> 4
<212> PRT
<213> homo sapiens

<220>
<221> MISC_FEATURE
<222> (1)..(4)
<223> Partial amino acid sequence that exists in each C -terminal regio
n of the peptide having the amino acid sequence of SEQ ID NO:2, h
uman BAI1 and human BAI2,

<400> 3

Gln Thr Glu Val
1

<210> 4
<211> 28
<212> DNA
<213> Artificial

<220>
<223> Designed oligonucleotide for using as a primer

<400> 4
ggggacaagt ttgtacaaaa aagcaggc 28

<210> 5
<211> 28
<212> DNA
<213> Artificial

<220>
<223>  Designed oligonucleotide for using as a primer

<400>  5
ggggaccact ttgtacaaga aagctggg                                          28


<210>  6
<211>  31
<212>  DNA
<213>  Artificial

<220>
<223>  Designed oligonucleotide for using as a primer

<400>  6
aaaaagcagg ctggatgacc ccagcctgtc c                                      31


<210>  7
<211>  32
<212>  DNA
<213>  Artificial

<220>
<223>  Designed oligonucleotide for using as a primer

<400>  7
agaaagctgg gtgcactcac acctctgtct gg                                     32


<210>  8
<211>  36
<212>  DNA
<213>  Artificial

<220>
<223>  Designed oligonucleotide for using as a primer

<400>  8
tacaaaaaag caggcttcga ccccgccccc agtgcc                                 36


<210>  9
<211>  33
<212>  DNA
<213>  Artificial

<220>
<223>  Designed oligonucleotide for using as a primer

<400>  9
ggggtcgaag cctgcttttt tgtacaaagt tgg                                    33


<210>  10
<211>  25
<212>  DNA
<213>  Artificial

<220>
<223>  Designed oligonucleotide for using as a primer

<400>  10
tcgcgttaac gctagcatgg atctc                                             25


<210>  11

<211>  24
<212>  DNA
<213>  Artificial

<220>
<223>  Designed oligonucleotide for using as a primer

<400>  11
gtaacatcag agattttgag acac                                              24


<210>  12
<211>  125
<212>  DNA
<213>  Artificial

<220>
<223>  Synthesized oligonucleotide

<400>  12
gggggaattc accatggcct taccagtgac cgccttgctc ctgccgctgg ccttgctgct      60

ccacgccgcc aggccggatt acgcttctta cccatacgat gttccagatt acgctgtcga     120

cgggg                                                                 125


<210>  13
<211>  125
<212>  DNA
<213>  Artificial

<220>
<223>  Synthesized oligonucleotide

<400>  13
ccccgtcgac agcgtaatct ggaacatcgt atgggtaaga agcgtaatcc ggcctggcgg      60

cgtggagcag caaggccagc ggcaggagca aggcggtcac tggtaaggcc atggtgaatt     120

ccccc                                                                 125


<210>  14
<211>  8
<212>  PRT
<213>  Homo sapiens

<220>
<221>  MISC_FEATURE
<223>  CCK-8S


<220>
<221>  MOD_RES
<222>  (2)..(2)
<223>  SULFATATION


<400>  14


<210>  15
<211>  4389
<212>  DNA

<213>  homo sapiens

<220>
<221>  misc_feature
<223>  A splice variant of the DNA having the nucleotide sequence of SEQ
       ID NO:1

<400>  15
atgaccccag cctgtcccct cttactgtct gtgattctgt ccctgcgcct ggccaccgcc      60

ttcgaccccg cccccagtgc ctgctctgcc ctggcctcgg gtgtgctcta cggggccttc     120

tcgctgcagg acctctttcc taccatcgcc tcgggctgct cctggaccct ggagaaccct     180

gaccccacca agtactccct ctacctgcgc ttcaaccgcc aggagcaggt gtgcgcacac     240

tttgcccccc gcctgctgcc cctggaccac tacctggtca actttacctg cctgcggcct     300

agccccgagg aggcggtggc ccaggcggag tcagaggtgg ggcggccaga agaggaggag     360

gcagaggcgg cagcggggtt ggagctgtgc agcggctcag gcccctttac cttcctgcac     420

ttcgacaaga acttcgtgca gctgtgcctg tcggctgagc cctccgaggc cccgcgcctg     480

ctggcgcccg ctgccctagc cttccgcttt gtcgaggtct tgctcatcaa caacaacaac     540

tctagccaat tcacctgtgg tgtgctctgc cgctggagtg aggagtgtgg ccgcgctgcc     600

ggcagggcct gcggctttgc tcagccaggc tgcagctgcc ctggagaggc gggggccggc     660

tccaccacca ccacatctcc aggccctcct gctgcccaca ccctgtccaa tgccctggtg     720

cccgggggcc cagccccacc tgctgaggcc gatttgcact cggggagcag caatgatctg     780

ttcacaaccg agatgagata tggtgaggag ccggaagagg aaccgaaagt gaaaacccag     840

tggccgaggt ctgcagatga gcctgggcta tacatggcgc agacagtgga aggccagtgg     900

ttagaatggg gtccctgggg cccatgctcc acgtcctgtg ccaatgggac ccaacagcgc     960

agccggaagt gcagcgtggc gggcccagcc tgggccacat gcacgggtgc cctcactgac    1020

acccgggagt gcagcaacct cgagtgcccg gccactgata gcaagtgggg gccatggaat    1080

gcgtggagcc tgtgctctaa gacgtgtgac acaggctggc agcgccgctt ccgcatgtgc    1140

caggccacgg gcacgcaggg ctacccctgc gagggcaccg gagaggaggt gaagccttgt    1200

agtgagaaga ggtgtccagc cttccatgag atgtgcaggg atgagtacgt gatgctgatg    1260

acgtggaaga aggcagctgc tggcgagatc atctacaaca agtgcccccc gaatgcctca    1320

gggtctgcca gccgccgctg tctcctcagt gcccaaggcg tggcgtactg gggcctgccc    1380

agctttgctc gctgcatctc ccatgagtac cgctacctgt atctgtcact tagggagcac    1440

ctggccaagg ggcagcgcat gctggcaggc gagggcatgt cgcaggtggt gcgcagcctg    1500

caggagctac tggcccggcg cacctactat agtggggacc tgctcttctc tgtggacatt    1560

ctgaggaatg tcactgacac ctttaagagg gccacctacg tgccctcggc tgatgatgtg    1620

cagcgcttct tccaggtggt gagcttcatg gtggatgcgg aaaacaagga gaagtgggac    1680

gatgctcagc aggtgtcccc tggctctgtg cacctgctcc gtgtcgtgga ggacttcatt    1740

cacctggtgg gcgatgctct caaggccttc cagagctctc tgattgtcac agataatcta    1800

gtgatcagca ttcagcgaga gcccgtctca gctgtgtcca gtgacatcac gttccccatg 1860
cggggccgcc ggggcatgaa ggactgggtg cggcactcag aggaccgcct cttcctgccc 1920
aaggaggtgc tcagcctctc ctccccaggg aagccagcca catctggggc agcaggcagc 1980
cctggcaggg ggaggggccc aggaacggtg cctcctggcc caggccactc ccaccagcgc 2040
ctcctcccag cagaccctga tgagtcctcc tactttgtga tcggtgctgt actctaccgc 2100
acccttggcc tcatcctgcc gcctcccagg cccccgctgg ccgtcacatc ccgggtgatg 2160
acagtgactg tgcgcccccc tacccagcct ccagctgagc ccctcatcac tgtggagctc 2220
tcctacatca tcaatgggac cacggatccc cattgcgcca gctgggacta ctccagagca 2280
gatgccagct caggagactg ggacactgaa aattgccaga ccctggagac ccaggcagct 2340
cacacccgct gccagtgcca gcacctgtcc acctttgctg tactagccca gccgcccaag 2400
gacctgaccc tggagctggc gggctccccc tcggtccccc tggtgatcgg ctgtgcagtg 2460
tcgtgcatgg cgctgctcac cctgctcgcc atctatgccg ccttttggag gttcataaaa 2520
tctgaacgct ccatcatctt gctgaacttc tgcctgtcca tcttggcatc caacatcctg 2580
atcctcgtgg gccagtcccg ggtgctgagc aagggcgtgt gcaccatgac ggctgccttc 2640
ctgcacttct tctttctctc ctccttttgc tgggtgctta ccgaggcctg gcagtcctac 2700
ctggctgtca ttgggcggat gcgcacccgc ctcgttcgca agcgcttcct ctgcctgggc 2760
tggggtctgc ctgccctggt ggtggccgtg tctgttggct ttacccgaac gaaaggatac 2820
ggtacatcca gctactgctg gctctccctg gagggcggcc tgctctacgc ctttgtgggc 2880
cctgcagccg tcattgtcct ggtgaacatg ctcatcggaa tcatcgtctt caacaagctc 2940
atggcacgtg atggcatctc cgacaaatcc aagaagcaga gggccgggtc ggagcggtgc 3000
ccctgggcca gcctgctcct cccctgctca gcgtgtggag cggtccccag ccccctgctc 3060
agctcagcct cggccaggaa cgccatggcc tcactctgga gctcctgcgt ggtgctgccc 3120
ctgctggcgc tcacctggat gtctgccgtc ctggctatga cagaccgccg ttccgtcctc 3180
ttccaggccc tctttgctgt cttcaactcc gcgcagggct ttgtcatcac tgctgtgcac 3240
tgcttcctgc gccgagaggt ccaggatgtg gtgaagtgcc agatgggggt gtgccgggct 3300
gatgagagcg aagactcccc tgactcgtgt aagaacgggc agctgcagat cctgtcagac 3360
tttgaaaagg atgtggatct ggcttgtcaa acagtgctgt tcaaggaggt caacacttgc 3420
aacccgtcca ccatcacggg cacactatcc cgcctgtccc tggatgagga tgaggagccc 3480
aagtcctgcc tcgtgggccc tgagggcagc ctcagcttct caccactgcc tgggaatatc 3540
ctggtgccca tggcagcctc accagggctg ggggagcctc cgcccccaca ggaggccaac 3600
cctgtttaca tgtgtgggga gggtggcctg cggcagctgg acctcacatg gctgcggccc 3660
actgagccag gctctgaggg agactacatg gtgctgcccc ggcggacttt gagcctgcag 3720
cctggcggtg gggtggaggg tggtgaggat gcccccaggg cccggccgga ggggaccccc 3780
cggcgagctg ccaagacagt ggcccacact gaaggctacc ccagcttcct gtccgtggac 3840

cactcgggcc tggggctggg ccctgcctat ggatctctcc agaatcccta tggaatgacc 3900

ttccaaccgc caccgccgac acccagcgcc cgccaagtgc ccgagccagg ggagcgcagc 3960

cggaccatgc ctcgcaccgt gcccggctct accatgaaga tgggctccct ggagcgaaag 4020

aaattacggt attcagacct ggactttgag aaggtgatgc acacccggaa acggcattca 4080

gaactctacc acgagctcaa ccagaagttc cacactttcg accgctaccg cagccagtcc 4140

acggccaaga gggagaagcg gtggagtgtg tcctcgggtg gggcagccga gcggagcgtg 4200

tgcaccgata agcccagccc tggggagcgc cccagcttgt cccaacatcg gcgccatcag 4260

agctggagca ccttcaaatc tatgacactg ggctcgctgc cccccaagcc ccgagaacgg 4320

ctgactctgc accgggcagc agcctgggag cccacagaac caccggatgg tgacttccag 4380

acagaggtg 4389

<210> 16
<211> 1463
<212> PRT
<213> homo sapiens

<220>
<221> misc_feature
<223> Protein encoded by the DNA having a nucleotide sequence of SEQ ID
NO:15

<220>
<221> DOMAIN
<222> (297)..(350)
<223> TSP-I domain

<220>
<221> DOMAIN
<222> (353)..(406)
<223> TSP-I domain

<220>
<221> TRANSMEM
<222> (815)..(835)
<223> transmembrane domain

<220>
<221> TRANSMEM
<222> (844)..(864)
<223> transmembrane domain

<220>
<221> TRANSMEM
<222> (873)..(893)
<223> transmembrane domain

<220>
<221> TRANSMEM
<222> (915)..(935)
<223> transmembrane domain

<220>
<221>  TRANSMEM
<222>  (957)..(977)
<223>  transmembrane domain


<220>
<221>  TRANSMEM
<222>  (1032)..(1052)
<223>  transmembrane domain


<220>
<221>  TRANSMEM
<222>  (1059)..(1079)
<223>  transmembrane domain


<400>  16

Met Thr Pro Ala Cys Pro Leu Leu Leu Ser Val Ile Leu Ser Leu Arg
1               5                   10                  15

Leu Ala Thr Ala Phe Asp Pro Ala Pro Ser Ala Cys Ser Ala Leu Ala
            20                  25                  30

Ser Gly Val Leu Tyr Gly Ala Phe Ser Leu Gln Asp Leu Phe Pro Thr
        35                  40                  45

Ile Ala Ser Gly Cys Ser Trp Thr Leu Glu Asn Pro Asp Pro Thr Lys
    50                  55                  60

Tyr Ser Leu Tyr Leu Arg Phe Asn Arg Gln Glu Gln Val Cys Ala His
65                  70                  75                  80

Phe Ala Pro Arg Leu Leu Pro Leu Asp His Tyr Leu Val Asn Phe Thr
                85                  90                  95

Cys Leu Arg Pro Ser Pro Glu Glu Ala Val Ala Gln Ala Glu Ser Glu
            100                 105                 110

Val Gly Arg Pro Glu Glu Glu Glu Ala Glu Ala Ala Ala Gly Leu Glu
        115                 120                 125

Leu Cys Ser Gly Ser Gly Pro Phe Thr Phe Leu His Phe Asp Lys Asn
    130                 135                 140

Phe Val Gln Leu Cys Leu Ser Ala Glu Pro Ser Glu Ala Pro Arg Leu
145                 150                 155                 160

Leu Ala Pro Ala Ala Leu Ala Phe Arg Phe Val Glu Val Leu Leu Ile
                165                 170                 175

Asn Asn Asn Asn Ser Ser Gln Phe Thr Cys Gly Val Leu Cys Arg Trp
            180                 185                 190

Ser Glu Glu Cys Gly Arg Ala Ala Gly Arg Ala Cys Gly Phe Ala Gln
        195                 200                 205

Pro Gly Cys Ser Cys Pro Gly Glu Ala Gly Ala Gly Ser Thr Thr Thr
    210                 215                 220

Thr Ser Pro Gly Pro Pro Ala Ala His Thr Leu Ser Asn Ala Leu Val
225                 230                 235                 240

Pro Gly Gly Pro Ala Pro Pro Ala Glu Ala Asp Leu His Ser Gly Ser
                245                 250                 255

Ser Asn Asp Leu Phe Thr Thr Glu Met Arg Tyr Gly Glu Glu Pro Glu
            260                 265                 270

Glu Glu Pro Lys Val Lys Thr Gln Trp Pro Arg Ser Ala Asp Glu Pro
        275                 280                 285

Gly Leu Tyr Met Ala Gln Thr Val Glu Gly Gln Trp Leu Glu Trp Gly
    290                 295                 300

Pro Trp Gly Pro Cys Ser Thr Ser Cys Ala Asn Gly Thr Gln Gln Arg
305                 310                 315                 320

Ser Arg Lys Cys Ser Val Ala Gly Pro Ala Trp Ala Thr Cys Thr Gly
                325                 330                 335

Ala Leu Thr Asp Thr Arg Glu Cys Ser Asn Leu Glu Cys Pro Ala Thr
            340                 345                 350

Asp Ser Lys Trp Gly Pro Trp Asn Ala Trp Ser Leu Cys Ser Lys Thr
        355                 360                 365

Cys Asp Thr Gly Trp Gln Arg Arg Phe Arg Met Cys Gln Ala Thr Gly
    370                 375                 380

Thr Gln Gly Tyr Pro Cys Glu Gly Thr Gly Glu Glu Val Lys Pro Cys
385                 390                 395                 400

Ser Glu Lys Arg Cys Pro Ala Phe His Glu Met Cys Arg Asp Glu Tyr
                405                 410                 415

Val Met Leu Met Thr Trp Lys Lys Ala Ala Ala Gly Glu Ile Ile Tyr
            420                 425                 430

Asn Lys Cys Pro Pro Asn Ala Ser Gly Ser Ala Ser Arg Arg Cys Leu
        435                 440                 445

Leu Ser Ala Gln Gly Val Ala Tyr Trp Gly Leu Pro Ser Phe Ala Arg
    450                 455                 460

Cys Ile Ser His Glu Tyr Arg Tyr Leu Tyr Leu Ser Leu Arg Glu His
465                 470                 475                 480

Leu Ala Lys Gly Gln Arg Met Leu Ala Gly Glu Gly Met Ser Gln Val
                485                 490                 495

Val Arg Ser Leu Gln Glu Leu Leu Ala Arg Arg Thr Tyr Tyr Ser Gly
            500                 505                 510

Asp Leu Leu Phe Ser Val Asp Ile Leu Arg Asn Val Thr Asp Thr Phe
        515                 520                 525

Lys Arg Ala Thr Tyr Val Pro Ser Ala Asp Asp Val Gln Arg Phe Phe
    530                 535                 540

Gln Val Val Ser Phe Met Val Asp Ala Glu Asn Lys Glu Lys Trp Asp
545                 550                 555                 560

Asp Ala Gln Gln Val Ser Pro Gly Ser Val His Leu Leu Arg Val Val
                565                 570                 575

Glu Asp Phe Ile His Leu Val Gly Asp Ala Leu Lys Ala Phe Gln Ser
            580                 585                 590

Ser Leu Ile Val Thr Asp Asn Leu Val Ile Ser Ile Gln Arg Glu Pro
        595                 600                 605

Val Ser Ala Val Ser Ser Asp Ile Thr Phe Pro Met Arg Gly Arg Arg
    610                 615                 620

Gly Met Lys Asp Trp Val Arg His Ser Glu Asp Arg Leu Phe Leu Pro
625                 630                 635                 640

Lys Glu Val Leu Ser Leu Ser Ser Pro Gly Lys Pro Ala Thr Ser Gly
                645                 650                 655

Ala Ala Gly Ser Pro Gly Arg Gly Arg Gly Pro Gly Thr Val Pro Pro
            660                 665                 670

Gly Pro Gly His Ser His Gln Arg Leu Leu Pro Ala Asp Pro Asp Glu
        675                 680                 685

Ser Ser Tyr Phe Val Ile Gly Ala Val Leu Tyr Arg Thr Leu Gly Leu
    690                 695                 700

Ile Leu Pro Pro Pro Arg Pro Pro Leu Ala Val Thr Ser Arg Val Met
705                 710                 715                 720

Thr Val Thr Val Arg Pro Pro Thr Gln Pro Pro Ala Glu Pro Leu Ile
                725                 730                 735

Thr Val Glu Leu Ser Tyr Ile Ile Asn Gly Thr Thr Asp Pro His Cys
            740                 745                 750

Ala Ser Trp Asp Tyr Ser Arg Ala Asp Ala Ser Ser Gly Asp Trp Asp
        755                 760                 765

Thr Glu Asn Cys Gln Thr Leu Glu Thr Gln Ala Ala His Thr Arg Cys
    770                 775                 780

Gln Cys Gln His Leu Ser Thr Phe Ala Val Leu Ala Gln Pro Pro Lys
785                 790                 795                 800

Asp Leu Thr Leu Glu Leu Ala Gly Ser Pro Ser Val Pro Leu Val Ile
                805                 810                 815

Gly Cys Ala Val Ser Cys Met Ala Leu Leu Thr Leu Leu Ala Ile Tyr
            820                 825                 830

Ala Ala Phe Trp Arg Phe Ile Lys Ser Glu Arg Ser Ile Ile Leu Leu
        835                 840                 845

Asn Phe Cys Leu Ser Ile Leu Ala Ser Asn Ile Leu Ile Leu Val Gly
    850                 855                 860

Gln Ser Arg Val Leu Ser Lys Gly Val Cys Thr Met Thr Ala Ala Phe
865                 870                 875                 880

Leu His Phe Phe Phe Leu Ser Ser Phe Cys Trp Val Leu Thr Glu Ala
                885                 890                 895

Trp Gln Ser Tyr Leu Ala Val Ile Gly Arg Met Arg Thr Arg Leu Val
            900                 905                 910

Arg Lys Arg Phe Leu Cys Leu Gly Trp Gly Leu Pro Ala Leu Val Val
        915                 920                 925

Ala Val Ser Val Gly Phe Thr Arg Thr Lys Gly Tyr Gly Thr Ser Ser
    930                 935                 940

Tyr Cys Trp Leu Ser Leu Glu Gly Gly Leu Leu Tyr Ala Phe Val Gly
945                 950                 955                 960

Pro Ala Ala Val Ile Val Leu Val Asn Met Leu Ile Gly Ile Ile Val
                965                 970                 975

Phe Asn Lys Leu Met Ala Arg Asp Gly Ile Ser Asp Lys Ser Lys Lys
            980                 985                 990

Gln Arg Ala Gly Ser Glu Arg Cys  Pro Trp Ala Ser Leu  Leu Leu Pro
        995                 1000                1005

Cys Ser Ala Cys Gly Ala Val Pro Ser Pro Leu Leu Ser Ser Ala
    1010                1015                1020

Ser Ala Arg Asn Ala Met Ala Ser Leu Trp Ser Ser Cys Val Val
    1025                1030                1035

Leu Pro Leu Leu Ala Leu Thr Trp Met Ser Ala Val Leu Ala Met
    1040                1045                1050

Thr Asp Arg Arg Ser Val Leu Phe Gln Ala Leu Phe Ala Val Phe
    1055                1060                1065

Asn Ser Ala Gln Gly Phe Val Ile Thr Ala Val His Cys Phe Leu
    1070                1075                1080

Arg Arg Glu Val Gln Asp Val Val Lys Cys Gln Met Gly Val Cys
    1085                1090                1095

Arg Ala Asp Glu Ser Glu Asp Ser Pro Asp Ser Cys Lys Asn Gly
    1100                1105                1110

Gln Leu Gln Ile Leu Ser Asp Phe Glu Lys Asp Val Asp Leu Ala
    1115                1120                1125

Cys Gln Thr Val Leu Phe Lys Glu Val Asn Thr Cys Asn Pro Ser
    1130                1135                1140

Thr Ile Thr Gly Thr Leu Ser Arg Leu Ser Leu Asp Glu Asp Glu
    1145                1150                1155

Glu Pro Lys Ser Cys Leu Val Gly Pro Glu Gly Ser Leu Ser Phe
    1160                1165                1170

Ser Pro Leu Pro Gly Asn Ile Leu Val Pro Met Ala Ala Ser Pro
    1175                1180                1185

Gly Leu Gly Glu Pro Pro Pro Pro Gln Glu Ala Asn Pro Val Tyr
    1190                1195                1200

Met Cys Gly Glu Gly Gly Leu Arg Gln Leu Asp Leu Thr Trp Leu
    1205                1210                1215

Arg Pro Thr Glu Pro Gly Ser Glu Gly Asp Tyr Met Val Leu Pro
    1220                1225                1230

Arg Arg Thr Leu Ser Leu Gln Pro Gly Gly Gly Gly Gly Gly Gly
    1235                1240                1245

Glu Asp Ala Pro Arg Ala Arg Pro Glu Gly Thr Pro Arg Arg Ala
    1250                1255                1260

Ala Lys Thr Val Ala His Thr Glu Gly Tyr Pro Ser Phe Leu Ser
    1265                    1270                    1275

Val Asp His Ser Gly Leu Gly Leu Gly Pro Ala Tyr Gly Ser Leu
    1280                    1285                    1290

Gln Asn Pro Tyr Gly Met Thr Phe Gln Pro Pro Pro Pro Thr Pro
    1295                    1300                    1305

Ser Ala Arg Gln Val Pro Glu Pro Gly Glu Arg Ser Arg Thr Met
    1310                    1315                    1320

Pro Arg Thr Val Pro Gly Ser Thr Met Lys Met Gly Ser Leu Glu
    1325                    1330                    1335

Arg Lys Lys Leu Arg Tyr Ser Asp Leu Asp Phe Glu Lys Val Met
    1340                    1345                    1350

His Thr Arg Lys Arg His Ser Glu Leu Tyr His Glu Leu Asn Gln
    1355                    1360                    1365

Lys Phe His Thr Phe Asp Arg Tyr Arg Ser Gln Ser Thr Ala Lys
    1370                    1375                    1380

Arg Glu Lys Arg Trp Ser Val Ser Ser Gly Gly Ala Ala Glu Arg
    1385                    1390                    1395

Ser Val Cys Thr Asp Lys Pro Ser Pro Gly Glu Arg Pro Ser Leu
    1400                    1405                    1410

Ser Gln His Arg Arg His Gln Ser Trp Ser Thr Phe Lys Ser Met
    1415                    1420                    1425

Thr Leu Gly Ser Leu Pro Pro Lys Pro Arg Glu Arg Leu Thr Leu
    1430                    1435                    1440

His Arg Ala Ala Ala Trp Glu Pro Thr Glu Pro Pro Asp Gly Asp
    1445                    1450                    1455

Phe Gln Thr Glu Val
    1460

<210>  17
<211>  4554
<212>  DNA
<213>  homo sapiens

<220>
<221>  misc_feature
<223>  A splice variant of the DNA having the nucleotide sequence of SEQ
        ID NO:1

<400> 17
atgaccccag cctgtcccct cttactgtct gtgattctgt ccctgcgcct ggccaccgcc 60
ttcgaccccg cccccagtgc ctgctctgcc ctggcctcgg gtgtgctcta cggggccttc 120
tcgctgcagg acctctttcc taccatcgcc tcgggctgct cctggaccct ggagaaccct 180
gaccccacca agtactccct ctacctgcgc ttcaaccgcc aggagcaggt gtgcgcacac 240
tttgcccccc gcctgctgcc cctggaccac tacctggtca actttacctg cctgcggcct 300
agccccgagg aggcggtggc ccaggcggag tcagaggtgg ggcggccaga agaggaggag 360
gcagaggcgg cagcggggtt ggagctgtgc agcggctcag gcccctttac cttcctgcac 420
ttcgacaaga acttcgtgca gctgtgcctg tcggctgagc cctccgaggc cccgcgcctg 480
ctggcgcccg ctgccctagc cttccgcttt gtcgaggtct tgctcatcaa caacaacaac 540
tctagccaat tcacctgtgg tgtgctctgc cgctggagtg aggagtgtgg ccgcgctgcc 600
ggcagggcct gcggctttgc tcagccaggc tgcagctgcc ctggagaggc gggggccggc 660
tccaccacca ccacatctcc aggccctcct gctgcccaca ccctgtccaa tgccctggtg 720
cccgggggcc cagccccacc tgctgaggcc gatttgcact cggggagcag caatgatctg 780
ttcacaaccg agatgagata tggtgaggag ccggaagagg aaccgaaagt gaaaacccag 840
tggccgaggt ctgcagatga gcctgggcta tacatggcgc agacaggcga cccggcggct 900
gaggagtggt ccccgtggag cgtgtgttcc ctgacgtgtg ggcagggtct gcaggtgcgg 960
acccgctcct gtgtgtcctc cccctatggg accctgtgca gcgggcccct gcgggagacc 1020
aggccctgca acaattcagc cacctgccca gtggaaggcc agtggttaga atggggtccc 1080
tggggcccat gctccacgtc ctgtgccaat gggacccaac agcgcagccg gaagtgcagc 1140
gtggcgggcc cagcctgggc cacatgcacg ggtgccctca ctgacacccg ggagtgcagc 1200
aacctcgagt gcccggccac tgatagcaag tgggggccat ggaatgcgtg gagcctgtgc 1260
tctaagacgt gtgacacagg ctggcagcgc cgcttccgca tgtgccaggc cacgggcacg 1320
cagggctacc cctgcgaggg caccggagag gaggtgaagc cttgtagtga gaagaggtgt 1380
ccagccttcc atgagatgtg cagggatgag tacgtgatgc tgatgacgtg gaagaaggca 1440
gctgctggcg agatcatcta caacaagtgc cccccgaatg cctcagggtc tgccagccgc 1500
cgctgtctcc tcagtgccca aggcgtggcg tactgggggc tgcccagctt tgctcgctgc 1560
atctcccatg agtaccgcta cctgtatctg tcacttaggg agcacctggc caaggggcag 1620
cgcatgctgg caggcgaggg catgtcgcag gtggtgcgca gcctgcagga gctactggcc 1680
cggcgcacct actatagtgg ggacctgctc ttctctgtgg acattctgag gaatgtcact 1740
gacaccttta agagggccac ctacgtgccc tcggctgatg atgtgcagcg cttcttccag 1800
gtggtgagct tcatggtgga tgcggaaaac aaggagaagt gggacgatgc tcagcaggtg 1860
tcccctggct ctgtgcacct gctccgtgtc gtggaggact tcattcacct ggtgggcgat 1920
gctctcaagg ccttccagag ctctctgatt gtcacagata atctagtgat cagcattcag 1980
cgagagcccg tctcagctgt gtccagtgac atcacgttcc ccatgcgggg ccgccggggc 2040

atgaaggact gggtgcggca ctcagaggac cgcctcttcc tgcccaagga ggtgctcagc 2100
ctctcctccc cagggaagcc agccacatct ggggcagcag gcagccctgg cagggggagg 2160
ggcccaggaa cggtgcctcc tggcccaggc cactcccacc agcgcctcct cccagcagac 2220
cctgatgagt cctcctactt tgtgatcggt gctgtactct accgcaccct tggcctcatc 2280
ctgccgcctc ccaggccccc gctggccgtc acatcccggg tgatgacagt gactgtgcgc 2340
ccccctaccc agcctccagc tgagcccctc atcactgtgg agctctccta catcatcaat 2400
gggaccacgg atccccattg cgccagctgg gactactcca gagcagatgc cagctcagga 2460
gactgggaca ctgaaaattg ccagaccctg gagacccagg cagctcacac ccgctgccag 2520
tgccagcacc tgtccacctt tgctgtacta gcccagccgc ccaaggacct gaccctggag 2580
ctggcgggct ccccctcggt ccccctggtg atcggctgtg cagtgtcgtg catggcgctg 2640
ctcaccctgc tcgccatcta tgccgccttt tggaggttca taaaatctga acgctccatc 2700
atcttgctga acttctgcct gtccatcttg gcatccaaca tcctgatcct cgtgggccag 2760
tcccgggtgc tgagcaaggg cgtgtgcacc atgacggctg ccttcctgca cttcttcttt 2820
ctctcctcct tttgctgggt gcttaccgag gcctggcagt cctacctggc tgtcattggg 2880
cggatgcgca cccgcctcgt tcgcaagcgc ttcctctgcc tgggctgggg tctgcctgcc 2940
ctggtggtgg ccgtgtctgt tggctttacc cgaacgaaag gatacggtac atccagctac 3000
tgctggctct ccctggaggg cggcctgctc tacgcctttg tgggccctgc agccgtcatt 3060
gtcctggtga acatgctcat cggaatcatc gtcttcaaca agctcatggc acgtgatggc 3120
atctccgaca aatccaagaa gcagagggcc gggtcggagc ggtgcccctg ggccagcctg 3180
ctcctcccct gctcagcgtg tggagcggtc cccagccccc tgctcagctc agcctcggcc 3240
aggaacgcca tggcctcact ctggagctcc tgcgtggtgc tgcccctgct ggcgctcacc 3300
tggatgtctg ccgtcctggc tatgacagac cgccgttccg tcctcttcca ggccctcttt 3360
gctgtcttca actccgcgca gggctttgtc atcactgctg tgcactgctt cctgcgccga 3420
gaggtccagg atgtggtgaa gtgccagatg ggggtgtgcc gggctgatga gagcgaagac 3480
tcccctgact cgtgtaagaa cgggcagctg cagatcctgt cagactttga aaaggatgtg 3540
gatctggctt gtcaaacagt gctgttcaag gaggtcaaca cttgcaaccc gtccaccatc 3600
acgggcacac tatcccgcct gtccctggat gaggatgagg agcccaagtc ctgcctcgtg 3660
ggccctgagg gcagcctcag cttctcacca ctgcctggga atatcctggt gcccatggca 3720
gcctcaccag ggctggggga gcctccgccc ccacaggagg ccaaccctgt ttacatgtgt 3780
ggggagggtg gcctgcggca gctggacctc acatggctgc ggcccactga gccaggctct 3840
gagggagact acatggtgct gccccggcgg actttgagcc tgcagcctgg cggtgggggt 3900
ggaggtggtg aggatgcccc cagggcccgg ccggagggga ccccccggcg agctgccaag 3960
acagtggccc acactgaagg ctaccccagc ttcctgtccg tggaccactc gggcctgggg 4020
ctgggccctg cctatggatc tctccagaat ccctatggaa tgaccttcca accgccaccg 4080

ccgacaccca gcgcccgcca agtgcccgag ccaggggagc gcagccggac catgcctcgc 4140

accgtgcccg gctctaccat gaagatgggc tccctggagc gaaagaaatt acggtattca 4200

gacctggact ttgagaaggt gatgcacacc cggaaacggc attcagaact ctaccacgag 4260

ctcaaccaga agttccacac tttcgaccgc taccgcagcc agtccacggc caagagggag 4320

aagcggtgga gtgtgtcctc gggtggggca gccgagcgga gcgtgtgcac cgataagccc 4380

agccctgggg agcgccccag cttgtcccaa catcggcgcc atcagagctg gagcaccttc 4440

aaatctatga cactgggctc gctgcccccc aagccccgag aacggctgac tctgcaccgg 4500

gcagcagcct gggagcccac agaaccaccg gatggtgact tccagacaga ggtg 4554

<210> 18
<211> 1518
<212> PRT
<213> homo sapiens

<220>
<221> misc_feature
<223> Protein encoded by the DNA having a nucleotide sequence of SEQ ID
 NO:17

<220>
<221> DOMAIN
<222> (297)..(350)
<223> TSP-I domain

<220>
<221> DOMAIN
<222> (352)..(405)
<223> TSP-I domain

<220>
<221> DOMAIN
<222> (408)..(461)
<223> TSP-I domain

<220>
<221> TRANSMEM
<222> (870)..(890)
<223> transmembrane domain

<220>
<221> TRANSMEM
<222> (899)..(919)
<223> transmembrane domain

<220>
<221> TRANSMEM
<222> (928)..(948)
<223> transmembrane domain

<220>
<221> TRANSMEM
<222> (970)..(990)

<223>  transmembrane domain

<220>
<221>  TRANSMEM
<222>  (1012)..(1032)
<223>  transmembrane domain

<220>
<221>  TRANSMEM
<222>  (1087)..(1107)
<223>  transmembrane domain

<220>
<221>  TRANSMEM
<222>  (1114)..(1134)
<223>  transmembrane domain

<400>  18

Met Thr Pro Ala Cys Pro Leu Leu Leu Ser Val Ile Leu Ser Leu Arg
1               5                   10                  15

Leu Ala Thr Ala Phe Asp Pro Ala Pro Ser Ala Cys Ser Ala Leu Ala
            20                  25                  30

Ser Gly Val Leu Tyr Gly Ala Phe Ser Leu Gln Asp Leu Phe Pro Thr
        35                  40                  45

Ile Ala Ser Gly Cys Ser Trp Thr Leu Glu Asn Pro Asp Pro Thr Lys
    50                  55                  60

Tyr Ser Leu Tyr Leu Arg Phe Asn Arg Gln Glu Gln Val Cys Ala His
65                  70                  75                  80

Phe Ala Pro Arg Leu Leu Pro Leu Asp His Tyr Leu Val Asn Phe Thr
                85                  90                  95

Cys Leu Arg Pro Ser Pro Glu Glu Ala Val Ala Gln Ala Glu Ser Glu
            100                 105                 110

Val Gly Arg Pro Glu Glu Glu Glu Ala Glu Ala Ala Ala Gly Leu Glu
        115                 120                 125

Leu Cys Ser Gly Ser Gly Pro Phe Thr Phe Leu His Phe Asp Lys Asn
    130                 135                 140

Phe Val Gln Leu Cys Leu Ser Ala Glu Pro Ser Glu Ala Pro Arg Leu
145                 150                 155                 160

Leu Ala Pro Ala Ala Leu Ala Phe Arg Phe Val Glu Val Leu Leu Ile
                165                 170                 175

Asn Asn Asn Asn Ser Ser Gln Phe Thr Cys Gly Val Leu Cys Arg Trp

180                 185                 190

Ser Glu Glu Cys Gly Arg Ala Ala Gly Arg Ala Cys Gly Phe Ala Gln
        195                 200                 205

Pro Gly Cys Ser Cys Pro Gly Glu Ala Gly Ala Gly Ser Thr Thr Thr
    210                 215                 220

Thr Ser Pro Gly Pro Pro Ala Ala His Thr Leu Ser Asn Ala Leu Val
225                 230                 235                 240

Pro Gly Gly Pro Ala Pro Pro Ala Glu Ala Asp Leu His Ser Gly Ser
                245                 250                 255

Ser Asn Asp Leu Phe Thr Thr Glu Met Arg Tyr Gly Glu Glu Pro Glu
            260                 265                 270

Glu Glu Pro Lys Val Lys Thr Gln Trp Pro Arg Ser Ala Asp Glu Pro
        275                 280                 285

Gly Leu Tyr Met Ala Gln Thr Gly Asp Pro Ala Ala Glu Glu Trp Ser
    290                 295                 300

Pro Trp Ser Val Cys Ser Leu Thr Cys Gly Gln Gly Leu Gln Val Arg
305                 310                 315                 320

Thr Arg Ser Cys Val Ser Ser Pro Tyr Gly Thr Leu Cys Ser Gly Pro
                325                 330                 335

Leu Arg Glu Thr Arg Pro Cys Asn Asn Ser Ala Thr Cys Pro Val Glu
            340                 345                 350

Gly Gln Trp Leu Glu Trp Gly Pro Trp Gly Pro Cys Ser Thr Ser Cys
        355                 360                 365

Ala Asn Gly Thr Gln Gln Arg Ser Arg Lys Cys Ser Val Ala Gly Pro
    370                 375                 380

Ala Trp Ala Thr Cys Thr Gly Ala Leu Thr Asp Thr Arg Glu Cys Ser
385                 390                 395                 400

Asn Leu Glu Cys Pro Ala Thr Asp Ser Lys Trp Gly Pro Trp Asn Ala
                405                 410                 415

Trp Ser Leu Cys Ser Lys Thr Cys Asp Thr Gly Trp Gln Arg Arg Phe
            420                 425                 430

Arg Met Cys Gln Ala Thr Gly Thr Gln Gly Tyr Pro Cys Glu Gly Thr
        435                 440                 445

Gly Glu Glu Val Lys Pro Cys Ser Glu Lys Arg Cys Pro Ala Phe His

450                 455                 460

Glu Met Cys Arg Asp Glu Tyr Val Met Leu Met Thr Trp Lys Lys Ala
465                 470                 475                 480

Ala Ala Gly Glu Ile Ile Tyr Asn Lys Cys Pro Pro Asn Ala Ser Gly
                485                 490                 495

Ser Ala Ser Arg Arg Cys Leu Leu Ser Ala Gln Gly Val Ala Tyr Trp
            500                 505                 510

Gly Leu Pro Ser Phe Ala Arg Cys Ile Ser His Glu Tyr Arg Tyr Leu
        515                 520                 525

Tyr Leu Ser Leu Arg Glu His Leu Ala Lys Gly Gln Arg Met Leu Ala
    530                 535                 540

Gly Glu Gly Met Ser Gln Val Val Arg Ser Leu Gln Glu Leu Leu Ala
545                 550                 555                 560

Arg Arg Thr Tyr Tyr Ser Gly Asp Leu Leu Phe Ser Val Asp Ile Leu
                565                 570                 575

Arg Asn Val Thr Asp Thr Phe Lys Arg Ala Thr Tyr Val Pro Ser Ala
            580                 585                 590

Asp Asp Val Gln Arg Phe Phe Gln Val Val Ser Phe Met Val Asp Ala
        595                 600                 605

Glu Asn Lys Glu Lys Trp Asp Asp Ala Gln Gln Val Ser Pro Gly Ser
    610                 615                 620

Val His Leu Leu Arg Val Val Glu Asp Phe Ile His Leu Val Gly Asp
625                 630                 635                 640

Ala Leu Lys Ala Phe Gln Ser Ser Leu Ile Val Thr Asp Asn Leu Val
                645                 650                 655

Ile Ser Ile Gln Arg Glu Pro Val Ser Ala Val Ser Ser Asp Ile Thr
            660                 665                 670

Phe Pro Met Arg Gly Arg Arg Gly Met Lys Asp Trp Val Arg His Ser
        675                 680                 685

Glu Asp Arg Leu Phe Leu Pro Lys Glu Val Leu Ser Leu Ser Ser Pro
    690                 695                 700

Gly Lys Pro Ala Thr Ser Gly Ala Ala Gly Ser Pro Gly Arg Gly Arg
705                 710                 715                 720

Gly Pro Gly Thr Val Pro Pro Gly Pro Gly His Ser His Gln Arg Leu

725                     730                     735

Leu Pro Ala Asp Pro Asp Glu Ser Ser Tyr Phe Val Ile Gly Ala Val
            740                 745                 750

Leu Tyr Arg Thr Leu Gly Leu Ile Leu Pro Pro Pro Arg Pro Pro Leu
        755                 760                 765

Ala Val Thr Ser Arg Val Met Thr Val Thr Val Arg Pro Pro Thr Gln
    770                 775                 780

Pro Pro Ala Glu Pro Leu Ile Thr Val Glu Leu Ser Tyr Ile Ile Asn
785                 790                 795                 800

Gly Thr Thr Asp Pro His Cys Ala Ser Trp Asp Tyr Ser Arg Ala Asp
                805                 810                 815

Ala Ser Ser Gly Asp Trp Asp Thr Glu Asn Cys Gln Thr Leu Glu Thr
            820                 825                 830

Gln Ala Ala His Thr Arg Cys Gln Cys Gln His Leu Ser Thr Phe Ala
        835                 840                 845

Val Leu Ala Gln Pro Pro Lys Asp Leu Thr Leu Glu Leu Ala Gly Ser
    850                 855                 860

Pro Ser Val Pro Leu Val Ile Gly Cys Ala Val Ser Cys Met Ala Leu
865                 870                 875                 880

Leu Thr Leu Leu Ala Ile Tyr Ala Ala Phe Trp Arg Phe Ile Lys Ser
                885                 890                 895

Glu Arg Ser Ile Ile Leu Leu Asn Phe Cys Leu Ser Ile Leu Ala Ser
            900                 905                 910

Asn Ile Leu Ile Leu Val Gly Gln Ser Arg Val Leu Ser Lys Gly Val
        915                 920                 925

Cys Thr Met Thr Ala Ala Phe Leu His Phe Phe Phe Leu Ser Ser Phe
    930                 935                 940

Cys Trp Val Leu Thr Glu Ala Trp Gln Ser Tyr Leu Ala Val Ile Gly
945                 950                 955                 960

Arg Met Arg Thr Arg Leu Val Arg Lys Arg Phe Leu Cys Leu Gly Trp
                965                 970                 975

Gly Leu Pro Ala Leu Val Val Ala Val Ser Val Gly Phe Thr Arg Thr
            980                 985                 990

Lys Gly Tyr Gly Thr Ser Ser Tyr  Cys Trp Leu Ser Leu  Glu Gly Gly

995                    1000                    1005

Leu Leu Tyr Ala Phe Val Gly Pro Ala Ala Val Ile Val Leu Val
    1010                    1015                    1020

Asn Met Leu Ile Gly Ile Ile Val Phe Asn Lys Leu Met Ala Arg
    1025                    1030                    1035

Asp Gly Ile Ser Asp Lys Ser Lys Lys Gln Arg Ala Gly Ser Glu
    1040                    1045                    1050

Arg Cys Pro Trp Ala Ser Leu Leu Leu Pro Cys Ser Ala Cys Gly
    1055                    1060                    1065

Ala Val Pro Ser Pro Leu Leu Ser Ser Ala Ser Ala Arg Asn Ala
    1070                    1075                    1080

Met Ala Ser Leu Trp Ser Ser Cys Val Val Leu Pro Leu Leu Ala
    1085                    1090                    1095

Leu Thr Trp Met Ser Ala Val Leu Ala Met Thr Asp Arg Arg Ser
    1100                    1105                    1110

Val Leu Phe Gln Ala Leu Phe Ala Val Phe Asn Ser Ala Gln Gly
    1115                    1120                    1125

Phe Val Ile Thr Ala Val His Cys Phe Leu Arg Arg Glu Val Gln
    1130                    1135                    1140

Asp Val Val Lys Cys Gln Met Gly Val Cys Arg Ala Asp Glu Ser
    1145                    1150                    1155

Glu Asp Ser Pro Asp Ser Cys Lys Asn Gly Gln Leu Gln Ile Leu
    1160                    1165                    1170

Ser Asp Phe Glu Lys Asp Val Asp Leu Ala Cys Gln Thr Val Leu
    1175                    1180                    1185

Phe Lys Glu Val Asn Thr Cys Asn Pro Ser Thr Ile Thr Gly Thr
    1190                    1195                    1200

Leu Ser Arg Leu Ser Leu Asp Glu Asp Glu Glu Pro Lys Ser Cys
    1205                    1210                    1215

Leu Val Gly Pro Glu Gly Ser Leu Ser Phe Ser Pro Leu Pro Gly
    1220                    1225                    1230

Asn Ile Leu Val Pro Met Ala Ala Ser Pro Gly Leu Gly Glu Pro
    1235                    1240                    1245

Pro Pro Pro Gln Glu Ala Asn Pro Val Tyr Met Cys Gly Glu Gly

1250                1255                1260

Gly Leu Arg Gln Leu Asp Leu Thr Trp Leu Arg Pro Thr Glu Pro
    1265                1270                1275

Gly Ser Glu Gly Asp Tyr Met Val Leu Pro Arg Arg Thr Leu Ser
    1280                1285                1290

Leu Gln Pro Gly Gly Gly Gly Gly Gly Gly Glu Asp Ala Pro Arg
    1295                1300                1305

Ala Arg Pro Glu Gly Thr Pro Arg Arg Ala Ala Lys Thr Val Ala
    1310                1315                1320

His Thr Glu Gly Tyr Pro Ser Phe Leu Ser Val Asp His Ser Gly
    1325                1330                1335

Leu Gly Leu Gly Pro Ala Tyr Gly Ser Leu Gln Asn Pro Tyr Gly
    1340                1345                1350

Met Thr Phe Gln Pro Pro Pro Pro Thr Pro Ser Ala Arg Gln Val
    1355                1360                1365

Pro Glu Pro Gly Glu Arg Ser Arg Thr Met Pro Arg Thr Val Pro
    1370                1375                1380

Gly Ser Thr Met Lys Met Gly Ser Leu Glu Arg Lys Lys Leu Arg
    1385                1390                1395

Tyr Ser Asp Leu Asp Phe Glu Lys Val Met His Thr Arg Lys Arg
    1400                1405                1410

His Ser Glu Leu Tyr His Glu Leu Asn Gln Lys Phe His Thr Phe
    1415                1420                1425

Asp Arg Tyr Arg Ser Gln Ser Thr Ala Lys Arg Glu Lys Arg Trp
    1430                1435                1440

Ser Val Ser Ser Gly Gly Ala Ala Glu Arg Ser Val Cys Thr Asp
    1445                1450                1455

Lys Pro Ser Pro Gly Glu Arg Pro Ser Leu Ser Gln His Arg Arg
    1460                1465                1470

His Gln Ser Trp Ser Thr Phe Lys Ser Met Thr Leu Gly Ser Leu
    1475                1480                1485

Pro Pro Lys Pro Arg Glu Arg Leu Thr Leu His Arg Ala Ala Ala
    1490                1495                1500

Trp Glu Pro Thr Glu Pro Pro Asp Gly Asp Phe Gln Thr Glu Val

1505                1510                1515

<210>  19
<211>  4719
<212>  DNA
<213>  homo sapiens

<220>
<221>  misc_feature
<223>  A splice variant of the DNA having the nucleotide sequence of SEQ
        ID NO:1.

<220>
<221>  misc_feature
<223>  A splice variant of the DNA having the nucleotide sequence of SEQ
        ID NO:1

<400>  19
   atgaccccag cctgtcccct cttactgtct gtgattctgt ccctgcgcct ggccaccgcc      60
   ttcgaccccg cccccagtgc ctgctctgcc ctggcctcgg gtgtgctcta cggggccttc     120
   tcgctgcagg acctctttcc taccatcgcc tcgggctgct cctggaccct ggagaaccct     180
   gaccccacca agtactccct ctacctgcgc ttcaaccgcc aggagcaggt gtgcgcacac     240
   tttgcccccc gcctgctgcc cctggaccac tacctggtca actttacctg cctgcggcct     300
   agccccgagg aggcggtggc ccaggcggag tcagaggtgg ggcggccaga agaggaggag     360
   gcagaggcgg cagcggggtt ggagctgtgc agcggctcag gcccctttac cttcctgcac     420
   ttcgacaaga acttcgtgca gctgtgcctg tcggctgagc cctccgaggc cccgcgcctg     480
   ctggcgcccg ctgccctagc cttccgcttt gtcgaggtct tgctcatcaa caacaacaac     540
   tctagccaat tcacctgtgg tgtgctctgc cgctggagtg aggagtgtgg ccgcgctgcc     600
   ggcagggcct gcggctttgc tcagccaggc tgcagctgcc ctggagaggc gggggccggc     660
   tccaccacca ccacatctcc aggccctcct gctgcccaca ccctgtccaa tgccctggtg     720
   cccgggggcc cagccccacc tgctgaggcc gatttgcact cggggagcag caatgatctg     780
   ttcacaaccg agatgagata tggtgaggag ccggaagagg aaccgaaagt gaaaacccag     840
   tggccgaggt ctgcagatga gcctgggcta tacatggcgc agacaggcga cccggcggct     900
   gaggagtggt ccccgtggag cgtgtgttcc ctgacgtgtg ggcagggtct gcaggtgcgg     960
   acccgctcct gtgtgtcctc cccctatggg accctgtgca gcgggcccct gcgggagacc    1020
   aggccctgca acaattcagc cacctgccca gtgcacggcg tgtgggagga gtgggggtcc    1080
   tggagcctgt gctcccgcag ctgcgggcgg gggtcccgga gccggatgcg gacctgcgtg    1140
   cccccccagc acggcggcaa ggcctgcgag ggtcctgagc tgcagactaa gctctgcagt    1200
   atggctgcct gcccggtgga aggccagtgg ttagaatggg gtccctgggg cccatgctcc    1260
   acgtcctgtg ccaatgggac ccaacagcgc agccggaagt gcagcgtggc gggcccagcc    1320
   tgggccacat gcacgggtgc cctcactgac acccgggagt gcagcaacct cgagtgcccg    1380
   gccactgata gcaagtgggg gccatggaat gcgtggagcc tgtgctctaa gacgtgtgac    1440
   acaggctggc agcgccgctt ccgcatgtgc caggccacgg gcacgcaggg ctacccctgc 1500
   gagggcaccg gagaggaggt gaagccttgt agtgagaaga ggtgtccagc cttccatgag 1560
   atgtgcaggg atgagtacgt gatgctgatg acgtggaaga aggcagctgc tggcgagatc 1620
   atctacaaca agtgcccccc gaatgcctca gggtctgcca gccgccgctg tctcctcagt 1680
   gcccaaggcg tggcgtactg ggggctgccc agctttgctc gctgcatctc ccatgagtac 1740
   cgctacctgt atctgtcact tagggagcac ctggccaagg ggcagcgcat gctggcaggc 1800
   gagggcatgt cgcaggtggt gcgcagcctg caggagctac tggcccggcg cacctactat 1860
   agtggggacc tgctcttctc tgtggacatt ctgaggaatg tcactgacac ctttaagagg 1920
   gccacctacg tgccctcggc tgatgatgtg cagcgcttct tccaggtggt gagcttcatg 1980
   gtggatgcgg aaaacaagga gaagtgggac gatgctcagc aggtgtcccc tggctctgtg 2040
   cacctgctcc gtgtcgtgga ggacttcatt cacctggtgg gcgatgctct caaggccttc 2100
   cagagctctc tgattgtcac agataatcta gtgatcagca ttcagcgaga gcccgtctca 2160
   gctgtgtcca gtgacatcac gttccccatg cggggccgcc ggggcatgaa ggactgggtg 2220
   cggcactcag aggaccgcct cttcctgccc aaggaggtgc tcagcctctc ctccccaggg 2280
   aagccagcca catctggggc agcaggcagc cctggcaggg ggagggggcc aggaacggtg 2340
   cctcctggcc caggccactc ccaccagcgc ctcctcccag cagaccctga tgagtcctcc 2400
   tactttgtga tcggtgctgt actctaccgc acccttggcc tcatcctgcc gcctcccagg 2460
   cccccgctgg ccgtcacatc ccgggtgatg acagtgactg tgcgcccccc tacccagcct 2520
   ccagctgagc ccctcatcac tgtggagctc tcctacatca tcaatgggac cacggatccc 2580
   cattgcgcca gctgggacta ctccagagca gatgccagct caggagactg ggacactgaa 2640
   aattgccaga ccctggagac ccaggcagct cacacccgct gccagtgcca gcacctgtcc 2700
   acctttgctg tactagccca gccgcccaag gacctgaccc tggagctggc gggctccccc 2760
   tcggtccccc tggtgatcgg ctgtgcagtg tcgtgcatgg cgctgctcac cctgctcgcc 2820
   atctatgccg ccttttggag gttcataaaa tctgaacgct ccatcatctt gctgaacttc 2880
   tgcctgtcca tcttggcatc caacatcctg atcctcgtgg gccagtcccg ggtgctgagc 2940
   aagggcgtgt gcaccatgac ggctgccttc ctgcacttct tctttctctc ctccttttgc 3000
   tgggtgctta ccgaggcctg gcagtcctac ctggctgtca ttgggcggat gcgcacccgc 3060
   ctcgttcgca agcgcttcct ctgcctgggc tggggtctgc ctgccctggt ggtggccgtg 3120
   tctgttggct ttacccgaac gaaaggatac ggtacatcca gctactgctg gctctccctg 3180
   gagggcggcc tgctctacgc ctttgtgggc cctgcagccg tcattgtcct ggtgaacatg 3240
   ctcatcggaa tcatcgtctt caacaagctc atggcacgtg atggcatctc cgacaaatcc 3300
   aagaagcaga gggccgggtc ggagcggtgc ccctgggcca gcctgctcct cccctgctca 3360
   gcgtgtggag cggtccccag ccccctgctc agctcagcct cggccaggaa cgccatggcc 3420
   tcactctgga gctcctgcgt ggtgctgccc ctgctggcgc tcacctggat gtctgccgtc 3480
   ctggctatga cagaccgccg ttccgtcctc ttccaggccc tctttgctgt cttcaactcc 3540
   gcgcagggct ttgtcatcac tgctgtgcac tgcttcctgc gccgagaggt ccaggatgtg 3600
   gtgaagtgcc agatgggggt gtgccgggct gatgagagcg aagactcccc tgactcgtgt 3660
   aagaacgggc agctgcagat cctgtcagac tttgaaaagg atgtggatct ggcttgtcaa 3720
   acagtgctgt tcaaggaggt caacacttgc aacccgtcca ccatcacggg cacactatcc 3780
   cgcctgtccc tggatgagga tgaggagccc aagtcctgcc tcgtgggccc tgagggcagc 3840
   ctcagcttct caccactgcc tgggaatatc ctggtgccca tggcagcctc accagggctg 3900
   ggggagcctc cgcccccaca ggaggccaac cctgtttaca tgtgtgggga gggtggcctg 3960
   cggcagctgg acctcacatg gctgcggccc actgagccag gctctgaggg agactacatg 4020
   gtgctgcccc ggcggacttt gagcctgcag cctggcggtg ggggtggagg tggtgaggat 4080
   gcccccaggg cccggccgga ggggaccccc cggcgagctg ccaagacagt ggcccacact 4140
   gaaggctacc ccagcttcct gtccgtggac cactcgggcc tggggctggg ccctgcctat 4200
   ggatctctcc agaatcccta tggaatgacc ttccaaccgc caccgccgac acccagcgcc 4260
   cgccaagtgc ccgagccagg ggagcgcagc cggaccatgc ctcgcaccgt gcccggctct 4320
   accatgaaga tgggctccct ggagcgaaag aaattacggt attcagacct ggactttgag 4380
   aaggtgatgc acacccggaa acggcattca gaactctacc acgagctcaa ccagaagttc 4440
   cacactttcg accgctaccg cagccagtcc acggccaaga gggagaagcg gtggagtgtg 4500
   tcctcgggtg gggcagccga gcggagcgtg tgcaccgata agcccagccc tggggagcgc 4560
   cccagcttgt cccaacatcg gcgccatcag agctggagca ccttcaaatc tatgacactg 4620
   ggctcgctgc cccccaagcc ccgagaacgg ctgactctgc accgggcagc agcctgggag 4680
   cccacagaac caccggatgg tgacttccag acagaggtg 4719

<210> 20
<211> 1573
<212> PRT
<213> homo sapiens

<220>
<221> misc_feature
<223> Protein encoded by the DNA having a nucleotide sequence of SEQ ID
 NO:19

<220>
<221> DOMAIN
<222> (297)..(350)
<223> TSP-I domain

<220>
<221> DOMAIN
<222> (352)..(405)
<223> TSP-I domain

<220>

<221>  DOMAIN
<222>  (407)..(460)
<223>  TSP-I domain


<220>
<221>  DOMAIN
<222>  (463)..(516)
<223>  TSP-I domain


<220>
<221>  TRANSMEM
<222>  (925)..(945)
<223>  transmembrane domain


<220>
<221>  TRANSMEM
<222>  (954)..(974)
<223>  transmembrane domain


<220>
<221>  TRANSMEM
<222>  (983)..(1003)
<223>  transmembrane domain


<220>
<221>  TRANSMEM
<222>  (1025)..(1045)
<223>  transmembrane domain


<220>
<221>  TRANSMEM
<222>  (1067)..(1087)
<223>  transmembrane domain


<220>
<221>  TRANSMEM
<222>  (1142)..(1162)
<223>  transmembrane domain


<220>
<221>  TRANSMEM
<222>  (1169)..(1189)
<223>  transmembrane domain


<400>  20

Met Thr Pro Ala Cys Pro Leu Leu Leu Ser Val Ile Leu Ser Leu Arg
1               5                   10                  15


Leu Ala Thr Ala Phe Asp Pro Ala Pro Ser Ala Cys Ser Ala Leu Ala
            20                  25                  30


Ser Gly Val Leu Tyr Gly Ala Phe Ser Leu Gln Asp Leu Phe Pro Thr
        35                  40                  45


Ile Ala Ser Gly Cys Ser Trp Thr Leu Glu Asn Pro Asp Pro Thr Lys

50                  55                  60

Tyr Ser Leu Tyr Leu Arg Phe Asn Arg Gln Glu Gln Val Cys Ala His
65                  70                  75                  80

Phe Ala Pro Arg Leu Leu Pro Leu Asp His Tyr Leu Val Asn Phe Thr
                85                  90                  95

Cys Leu Arg Pro Ser Pro Glu Glu Ala Val Ala Gln Ala Glu Ser Glu
            100                 105                 110

Val Gly Arg Pro Glu Glu Glu Glu Ala Glu Ala Ala Ala Gly Leu Glu
        115                 120                 125

Leu Cys Ser Gly Ser Gly Pro Phe Thr Phe Leu His Phe Asp Lys Asn
    130                 135                 140

Phe Val Gln Leu Cys Leu Ser Ala Glu Pro Ser Glu Ala Pro Arg Leu
145                 150                 155                 160

Leu Ala Pro Ala Ala Leu Ala Phe Arg Phe Val Glu Val Leu Leu Ile
                165                 170                 175

Asn Asn Asn Asn Ser Ser Gln Phe Thr Cys Gly Val Leu Cys Arg Trp
            180                 185                 190

Ser Glu Glu Cys Gly Arg Ala Ala Gly Arg Ala Cys Gly Phe Ala Gln
        195                 200                 205

Pro Gly Cys Ser Cys Pro Gly Glu Ala Gly Ala Gly Ser Thr Thr Thr
    210                 215                 220

Thr Ser Pro Gly Pro Pro Ala Ala His Thr Leu Ser Asn Ala Leu Val
225                 230                 235                 240

Pro Gly Gly Pro Ala Pro Pro Ala Glu Ala Asp Leu His Ser Gly Ser
                245                 250                 255

Ser Asn Asp Leu Phe Thr Thr Glu Met Arg Tyr Gly Glu Glu Pro Glu
            260                 265                 270

Glu Glu Pro Lys Val Lys Thr Gln Trp Pro Arg Ser Ala Asp Glu Pro
        275                 280                 285

Gly Leu Tyr Met Ala Gln Thr Gly Asp Pro Ala Ala Glu Glu Trp Ser
    290                 295                 300

Pro Trp Ser Val Cys Ser Leu Thr Cys Gly Gln Gly Leu Gln Val Arg
305                 310                 315                 320

Thr Arg Ser Cys Val Ser Ser Pro Tyr Gly Thr Leu Cys Ser Gly Pro

325                 330                 335

Leu Arg Glu Thr Arg Pro Cys Asn Asn Ser Ala Thr Cys Pro Val His
            340                 345                 350

Gly Val Trp Glu Glu Trp Gly Ser Trp Ser Leu Cys Ser Arg Ser Cys
        355                 360                 365

Gly Arg Gly Ser Arg Ser Arg Met Arg Thr Cys Val Pro Pro Gln His
    370                 375                 380

Gly Gly Lys Ala Cys Glu Gly Pro Glu Leu Gln Thr Lys Leu Cys Ser
385                 390                 395                 400

Met Ala Ala Cys Pro Val Glu Gly Gln Trp Leu Glu Trp Gly Pro Trp
                405                 410                 415

Gly Pro Cys Ser Thr Ser Cys Ala Asn Gly Thr Gln Gln Arg Ser Arg
            420                 425                 430

Lys Cys Ser Val Ala Gly Pro Ala Trp Ala Thr Cys Thr Gly Ala Leu
        435                 440                 445

Thr Asp Thr Arg Glu Cys Ser Asn Leu Glu Cys Pro Ala Thr Asp Ser
    450                 455                 460

Lys Trp Gly Pro Trp Asn Ala Trp Ser Leu Cys Ser Lys Thr Cys Asp
465                 470                 475                 480

Thr Gly Trp Gln Arg Arg Phe Arg Met Cys Gln Ala Thr Gly Thr Gln
                485                 490                 495

Gly Tyr Pro Cys Glu Gly Thr Gly Glu Glu Val Lys Pro Cys Ser Glu
            500                 505                 510

Lys Arg Cys Pro Ala Phe His Glu Met Cys Arg Asp Glu Tyr Val Met
        515                 520                 525

Leu Met Thr Trp Lys Lys Ala Ala Ala Gly Glu Ile Ile Tyr Asn Lys
    530                 535                 540

Cys Pro Pro Asn Ala Ser Gly Ser Ala Ser Arg Arg Cys Leu Leu Ser
545                 550                 555                 560

Ala Gln Gly Val Ala Tyr Trp Gly Leu Pro Ser Phe Ala Arg Cys Ile
                565                 570                 575

Ser His Glu Tyr Arg Tyr Leu Tyr Leu Ser Leu Arg Glu His Leu Ala
            580                 585                 590

Lys Gly Gln Arg Met Leu Ala Gly Glu Gly Met Ser Gln Val Val Arg

595                 600                 605

Ser Leu Gln Glu Leu Leu Ala Arg Arg Thr Tyr Tyr Ser Gly Asp Leu
    610                 615                 620

Leu Phe Ser Val Asp Ile Leu Arg Asn Val Thr Asp Thr Phe Lys Arg
625                 630                 635                 640

Ala Thr Tyr Val Pro Ser Ala Asp Asp Val Gln Arg Phe Phe Gln Val
                645                 650                 655

Val Ser Phe Met Val Asp Ala Glu Asn Lys Glu Lys Trp Asp Asp Ala
            660                 665                 670

Gln Gln Val Ser Pro Gly Ser Val His Leu Leu Arg Val Val Glu Asp
        675                 680                 685

Phe Ile His Leu Val Gly Asp Ala Leu Lys Ala Phe Gln Ser Ser Leu
    690                 695                 700

Ile Val Thr Asp Asn Leu Val Ile Ser Ile Gln Arg Glu Pro Val Ser
705                 710                 715                 720

Ala Val Ser Ser Asp Ile Thr Phe Pro Met Arg Gly Arg Arg Gly Met
                725                 730                 735

Lys Asp Trp Val Arg His Ser Glu Asp Arg Leu Phe Leu Pro Lys Glu
            740                 745                 750

Val Leu Ser Leu Ser Ser Pro Gly Lys Pro Ala Thr Ser Gly Ala Ala
        755                 760                 765

Gly Ser Pro Gly Arg Gly Arg Gly Pro Gly Thr Val Pro Pro Gly Pro
    770                 775                 780

Gly His Ser His Gln Arg Leu Leu Pro Ala Asp Pro Asp Glu Ser Ser
785                 790                 795                 800

Tyr Phe Val Ile Gly Ala Val Leu Tyr Arg Thr Leu Gly Leu Ile Leu
                805                 810                 815

Pro Pro Pro Arg Pro Pro Leu Ala Val Thr Ser Arg Val Met Thr Val
            820                 825                 830

Thr Val Arg Pro Pro Thr Gln Pro Pro Ala Glu Pro Leu Ile Thr Val
        835                 840                 845

Glu Leu Ser Tyr Ile Ile Asn Gly Thr Thr Asp Pro His Cys Ala Ser
    850                 855                 860

Trp Asp Tyr Ser Arg Ala Asp Ala Ser Ser Gly Asp Trp Asp Thr Glu

865                 870                 875                 880

Asn Cys Gln Thr Leu Glu Thr Gln Ala Ala His Thr Arg Cys Gln Cys
                885                 890                 895

Gln His Leu Ser Thr Phe Ala Val Leu Ala Gln Pro Pro Lys Asp Leu
            900                 905                 910

Thr Leu Glu Leu Ala Gly Ser Pro Ser Val Pro Leu Val Ile Gly Cys
        915                 920                 925

Ala Val Ser Cys Met Ala Leu Leu Thr Leu Leu Ala Ile Tyr Ala Ala
    930                 935                 940

Phe Trp Arg Phe Ile Lys Ser Glu Arg Ser Ile Ile Leu Leu Asn Phe
945                 950                 955                 960

Cys Leu Ser Ile Leu Ala Ser Asn Ile Leu Ile Leu Val Gly Gln Ser
                965                 970                 975

Arg Val Leu Ser Lys Gly Val Cys Thr Met Thr Ala Ala Phe Leu His
            980                 985                 990

Phe Phe Phe Leu Ser Ser Phe Cys  Trp Val Leu Thr Glu  Ala Trp Gln
        995                 1000                1005

Ser Tyr  Leu Ala Val Ile Gly  Arg Met Arg Thr Arg  Leu Val Arg
    1010                1015                1020

Lys Arg  Phe Leu Cys Leu Gly  Trp Gly Leu Pro Ala  Leu Val Val
    1025                1030                1035

Ala Val  Ser Val Gly Phe Thr  Arg Thr Lys Gly Tyr  Gly Thr Ser
    1040                1045                1050

Ser Tyr  Cys Trp Leu Ser Leu  Glu Gly Gly Leu Leu  Tyr Ala Phe
    1055                1060                1065

Val Gly  Pro Ala Ala Val Ile  Val Leu Val Asn Met  Leu Ile Gly
    1070                1075                1080

Ile Ile  Val Phe Asn Lys Leu  Met Ala Arg Asp Gly  Ile Ser Asp
    1085                1090                1095

Lys Ser  Lys Lys Gln Arg Ala  Gly Ser Glu Arg Cys  Pro Trp Ala
    1100                1105                1110

Ser Leu  Leu Leu Pro Cys Ser  Ala Cys Gly Ala Val  Pro Ser Pro
    1115                1120                1125

Leu Leu  Ser Ser Ala Ser Ala  Arg Asn Ala Met Ala  Ser Leu Trp

1130                 1135                 1140

Ser Ser Cys Val Val Leu Pro Leu Leu Ala Leu Thr Trp Met Ser
    1145                1150                1155

Ala Val Leu Ala Met Thr Asp Arg Arg Ser Val Leu Phe Gln Ala
    1160                1165                1170

Leu Phe Ala Val Phe Asn Ser Ala Gln Gly Phe Val Ile Thr Ala
    1175                1180                1185

Val His Cys Phe Leu Arg Arg Glu Val Gln Asp Val Val Lys Cys
    1190                1195                1200

Gln Met Gly Val Cys Arg Ala Asp Glu Ser Glu Asp Ser Pro Asp
    1205                1210                1215

Ser Cys Lys Asn Gly Gln Leu Gln Ile Leu Ser Asp Phe Glu Lys
    1220                1225                1230

Asp Val Asp Leu Ala Cys Gln Thr Val Leu Phe Lys Glu Val Asn
    1235                1240                1245

Thr Cys Asn Pro Ser Thr Ile Thr Gly Thr Leu Ser Arg Leu Ser
    1250                1255                1260

Leu Asp Glu Asp Glu Glu Pro Lys Ser Cys Leu Val Gly Pro Glu
    1265                1270                1275

Gly Ser Leu Ser Phe Ser Pro Leu Pro Gly Asn Ile Leu Val Pro
    1280                1285                1290

Met Ala Ala Ser Pro Gly Leu Gly Glu Pro Pro Pro Pro Gln Glu
    1295                1300                1305

Ala Asn Pro Val Tyr Met Cys Gly Glu Gly Gly Leu Arg Gln Leu
    1310                1315                1320

Asp Leu Thr Trp Leu Arg Pro Thr Glu Pro Gly Ser Glu Gly Asp
    1325                1330                1335

Tyr Met Val Leu Pro Arg Arg Thr Leu Ser Leu Gln Pro Gly Gly
    1340                1345                1350

Gly Gly Gly Gly Gly Glu Asp Ala Pro Arg Ala Arg Pro Glu Gly
    1355                1360                1365

Thr Pro Arg Arg Ala Ala Lys Thr Val Ala His Thr Glu Gly Tyr
    1370                1375                1380

Pro Ser Phe Leu Ser Val Asp His Ser Gly Leu Gly Leu Gly Pro

1385                    1390                    1395

Ala Tyr Gly Ser Leu Gln Asn Pro Tyr Gly Met Thr Phe Gln Pro
    1400                    1405                    1410

Pro Pro Pro Thr Pro Ser Ala Arg Gln Val Pro Glu Pro Gly Glu
    1415                    1420                    1425

Arg Ser Arg Thr Met Pro Arg Thr Val Pro Gly Ser Thr Met Lys
    1430                    1435                    1440

Met Gly Ser Leu Glu Arg Lys Lys Leu Arg Tyr Ser Asp Leu Asp
    1445                    1450                    1455

Phe Glu Lys Val Met His Thr Arg Lys Arg His Ser Glu Leu Tyr
    1460                    1465                    1470

His Glu Leu Asn Gln Lys Phe His Thr Phe Asp Arg Tyr Arg Ser
    1475                    1480                    1485

Gln Ser Thr Ala Lys Arg Glu Lys Arg Trp Ser Val Ser Ser Gly
    1490                    1495                    1500

Gly Ala Ala Glu Arg Ser Val Cys Thr Asp Lys Pro Ser Pro Gly
    1505                    1510                    1515

Glu Arg Pro Ser Leu Ser Gln His Arg Arg His Gln Ser Trp Ser
    1520                    1525                    1530

Thr Phe Lys Ser Met Thr Leu Gly Ser Leu Pro Pro Lys Pro Arg
    1535                    1540                    1545

Glu Arg Leu Thr Leu His Arg Ala Ala Ala Trp Glu Pro Thr Glu
    1550                    1555                    1560

Pro Pro Asp Gly Asp Phe Gln Thr Glu Val
    1565                    1570

<210>  21
<211>  5399
<212>  DNA
<213>  homo sapiens

<220>
<221>  misc_feature
<223>  A splice variant of the DNA having the nucleotide sequence of SEQ
       ID NO:1, which encodes hBAI2

<400>  21
gccgcgcggg agagcgggag cctcggccct ccgcgcggct gcagctacct accctgcgcc      60

cggccaggtc cccgacttag ggatggcaaa cttgcgcccc gtggccgccc ccgccagcgc     120

cggcccccgc tcctgctgct gacggcgccc aggaaatcca cagcagtgat acatgtgacg     180

tccacactga cagtgccctc ctgtgggcat ggctcaggtt gtgcgcagtt cctggcacac 240
tggctgtaac tccgcccctt tctctccctc tcagtaaagc aagattacgc ggtgacatgc 300
ctcacagctg atcacgacac acggggatgg agagcaagag ttatggagaa tacaggttgg 360
atgggcaagg gacataggat gaccccagcc tgtcccctct tactgtctgt gattctgtcc 420
ctgcgcctgg ccaccgcctt cgaccccgcc cccagtgcct gctctgccct ggcctcgggt 480
gtgctctacg gggccttctc gctgcaggac ctctttccta ccatcgcctc gggctgctcc 540
tggaccctgg agaaccctga ccccaccaag tactccctct acctgcgctt caaccgccag 600
gagcaggtgt gcgcacactt tgccccccgc ctgctgcccc tggaccacta cctggtcaac 660
tttacctgcc tgcggcctag ccccgaggag gcggtggccc aggcggagtc agaggtgggg 720
cggccagaag aggaggaggc agaggcggca gcggggttgg agctgtgcag cggctcaggc 780
ccctttacct tcctgcactt cgacaagaac ttcgtgcagc tgtgcctgtc ggctgagccc 840
tccgaggccc cgcgcctgct ggcgcccgct gccctagcct tccgctttgt cgaggtcttg 900
ctcatcaaca acaacaactc tagccaattc acctgtggtg tgctctgccg ctggagtgag 960
gagtgtggcc gcgctgccgg cagggcctgc ggctttgctc agccaggctg cagctgccct 1020
ggagaggcgg gggccggctc caccaccacc acatctccag gccctcctgc tgcccacacc 1080
ctgtccaatg ccctggtgcc cgggggccca gccccacctg ctgaggccga tttgcactcg 1140
gggagcagca atgatctgtt cacaaccgag atgagatatg gtgaggagcc ggaagaggaa 1200
ccgaaagtga aaacccagtg gccgaggtct gcagatgagc ctgggctata catggcgcag 1260
acaggcgacc cggcggctga ggagtggtcc ccgtggagcg tgtgttccct gacgtgtggg 1320
cagggtctgc aggtgcggac ccgctcctgt gtgtcctccc cctatgggac cctgtgcagc 1380
gggcccctgc gggagaccag gccctgcaac aattcagcca cctgcccagt gcacggcgtg 1440
tgggaggagt gggggtcctg gagcctgtgc tcccgcagct gcgggcgggg gtcccggagc 1500
cggatgcgga cctgcgtgcc cccccagcac ggcggcaagg cctgcgaggg tcctgagctg 1560
cagactaagc tctgcagtat ggctgcctgc ccggtggaag gccagtggtt agaatggggt 1620
ccctggggcc catgctccac gtcctgtgcc aatgggaccc aacagcgcag ccggaagtgc 1680
agcgtggcgg gcccagcctg ggccacatgc acgggtgccc tcactgacac ccgggagtgc 1740
agcaacctcg agtgcccggc cactgatagc aagtgggggc catggaatgc gtggagcctg 1800
tgctctaaga cgtgtgacac aggctggcag cgccgcttcc gcatgtgcca ggccacgggc 1860
acgcagggct acccctgcga gggcaccgga gaggaggtga agccttgtag tgagaagagg 1920
tgtccagcct tccatgagat gtgcagggat gagtacgtga tgctgatgac gtggaagaag 1980
gcagctgctg gcgagatcat ctacaacaag tgccccccga atgcctcagg gtctgccagc 2040
cgccgctgtc tcctcagtgc ccaaggcgtg gcgtactggg ggctgcccag ctttgctcgc 2100
tgcatctccc atgagtaccg ctacctgtat ctgtcactta gggagcacct ggccaagggg 2160
cagcgcatgc tggcaggcga gggcatgtcg caggtggtgc gcagcctgca ggagctactg 2220

gcccggcgca cctactatag tggggacctg ctcttctctg tggacattct gaggaatgtc 2280
actgacacct ttaagagggc cacctacgtg ccctcggctg atgatgtgca gcgcttcttc 2340
caggtggtga gcttcatggt ggatgcggaa aacaaggaga agtgggacga tgctcagcag 2400
gtgtcccctg gctctgtgca cctgctccgt gtcgtggagg acttcattca cctggtgggc 2460
gatgctctca aggccttcca gagctctctg attgtcacag ataatctagt gatcagcatt 2520
cagcgagagc ccgtctcagc tgtgtccagt gacatcacgt tccccatgcg gggccgccgg 2580
ggcatgaagg actgggtgcg gcactcagag gaccgcctct tcctgcccaa ggaggtgctc 2640
agcctctcct ccccagggaa gccagccaca tctggggcag caggcagccc tggcaggggg 2700
aggggcccag gaacggtgcc tcctggccca ggccactccc accagcgcct cctcccagca 2760
gaccctgatg agtcctccta ctttgtgatc ggtgctgtac tctaccgcac ccttggcctc 2820
atcctgccgc ctcccaggcc cccgctggcc gtcacatccc gggtgatgac agtgactgtg 2880
cgccccccta cccagcctcc agctgagccc ctcatcactg tggagctctc ctacatcatc 2940
aatgggacca cggatcccca ttgcgccagc tgggactact ccagagcaga tgccagctca 3000
ggagactggg acactgaaaa ttgccagacc ctggagaccc aggcagctca cacccgctgc 3060
cagtgccagc acctgtccac ctttgctgta ctagcccagc cgcccaagga cctgaccctg 3120
gagctggcgg gctccccctc ggtccccctg gtgatcggct gtgcagtgtc gtgcatggcg 3180
ctgctcaccc tgctcgccat ctatgccgcc ttttggaggt tcataaaatc tgaacgctcc 3240
atcatcttgc tgaacttctg cctgtccatc ttggcatcca acatcctgat cctcgtgggc 3300
cagtcccggg tgctgagcaa gggcgtgtgc accatgacgg ctgccttcct gcacttcttc 3360
tttctctcct ccttttgctg ggtgcttacc gaggcctggc agtcctacct ggctgtcatt 3420
gggcggatgc gcacccgcct cgttcgcaag cgcttcctct gcctgggctg gggtctgcct 3480
gccctggtgg tggccgtgtc tgttggcttt acccgaacga aaggatacgg tacatccagc 3540
tactgctggc tctccctgga gggcggcctg ctctacgcct ttgtgggccc tgcagccgtc 3600
attgtcctgg tgaacatgct catcggaatc atcgtcttca acaagctcat ggcacgtgat 3660
ggcatctccg acaaatccaa gaagcagagg gccgggtcgg agcggtgccc ctgggccagc 3720
ctgctcctcc cctgctcagc gtgtggagcg gtccccagcc ccctgctcag ctcagcctcg 3780
gccaggaacg ccatggcctc actctggagc tcctgcgtgg tgctgcccct gctggcgctc 3840
acctggatgt ctgccgtcct ggctatgaca gaccgccgtt ccgtcctctt ccaggccctc 3900
tttgctgtct tcaactccgc gcagggcttt gtcatcactg ctgtgcactg cttcctgcgc 3960
cgagaggtcc aggatgtggt gaagtgccag atgggggtgt gccgggctga tgagagcgaa 4020
gactcccctg actcgtgtaa gaacgggcag ctgcagatcc tgtcagactt tgaaaaggat 4080
gtggatctgg cttgtcaaac agtgctgttc aaggaggtca acacttgcaa cccgtccacc 4140
atcacgggca cactatcccg cctgtccctg gatgaggatg aggagcccaa gtcctgcctc 4200
gtgggccctg agggcagcct cagcttctca ccactgcctg ggaatatcct ggtgcccatg 4260

gcagcctcac cagggctggg ggagcctccg cccccacagg aggccaaccc tgtttacatg 4320

tgtggggagg gtggcctgcg gcagctggac ctcacatggc tgcggcccac tgagccaggc 4380

tctgagggag actacatggt gctgccccgg cggactttga gcctgcagcc tggcggtggg 4440

ggtggaggtg gtgaggatgc ccccagggcc cggcccgagg ggaccccccg gcgagctgcc 4500

aagacagtgg cccacactga aggctacccc agcttcctgt ccgtggacca ctcgggcctg 4560

gggctgggcc ctgcctatgg atctctccag aatccctatg gaatgacctt ccaaccgcca 4620

ccgccgacac ccagcgcccg ccaagtgccc gagccagggg agcgcagccg gaccatgcct 4680

cgcaccgtgc ccggctctac catgaagatg ggctccctgg agcgaaagaa attacggtat 4740

tcagacctgg actttgaggt gatgcacacc cggaaacggc attcagaact ctaccacgag 4800

ctcaaccaga agttccacac tttcgaccgc taccgcagcc agtccacggc caagagggag 4860

aagcggtgga gtgtgtcctc gggtggggcg gccgagcgga gcgtgtgcac cgataagccc 4920

agccctgggg agcgccccag cttgtcccaa catcggcgcc atcagagctg gagcaccttc 4980

aaatctatga cactgggctc gctgcccccc aagccccgag aacggctgac tctgcaccgg 5040

gcagcagcct gggagcccac agaaccaccg gatggtgact tccagacaga ggtgtgagtg 5100

ccacgctgga ctgcccactg catataaata tatatatctc tctattttca cactccactt 5160

tggaactacc caggagccag cgccctctcc cctctcccga gggctgggca gggaggcgcc 5220

gtggactcag ccaggctggg ggagccggac atggcttggc ctggggtccc agggcccttc 5280

cttgtttctc agaggcccct cagccactgg aaccccatct tcagcccagc ctgtccgtcc 5340

ctgtcccggg ctggggaggg gggaggggaa ctttgttggg aataaacttc actctgtgg 5399

<210> 22
<211> 1572
<212> PRT
<213> homo sapiens

<220>
<221> misc_feature
<223> Protein encoded by the DNA having a nucleotide sequence of SEQ ID
NO:21

<220>
<221> DOMAIN
<222> (297)..(350)
<223> TSP-I domain

<220>
<221> DOMAIN
<222> (352)..(405)
<223> TSP-I domain

<220>
<221> DOMAIN
<222> (407)..(460)
<223> TSP-I domain

<220>
<221>  DOMAIN
<222>  (463)..(516)
<223>  TSP-I domain


<220>
<221>  TRANSMEM
<222>  (925)..(945)
<223>  transmembrane domain


<220>
<221>  TRANSMEM
<222>  (954)..(974)
<223>  transmembrane domain


<220>
<221>  TRANSMEM
<222>  (983)..(1003)
<223>  transmembrane domain


<220>
<221>  TRANSMEM
<222>  (1025)..(1045)
<223>  transmembrane domain


<220>
<221>  TRANSMEM
<222>  (1067)..(1087)
<223>  transmembrane domain


<220>
<221>  TRANSMEM
<222>  (1142)..(1162)
<223>  transmembrane domain


<220>
<221>  TRANSMEM
<222>  (1169)..(1189)
<223>  transmembrane domain


<400>  22

Met Thr Pro Ala Cys Pro Leu Leu Leu Ser Val Ile Leu Ser Leu Arg
1               5                   10                  15


Leu Ala Thr Ala Phe Asp Pro Ala Pro Ser Ala Cys Ser Ala Leu Ala
            20                  25                  30


Ser Gly Val Leu Tyr Gly Ala Phe Ser Leu Gln Asp Leu Phe Pro Thr
        35                  40                  45


Ile Ala Ser Gly Cys Ser Trp Thr Leu Glu Asn Pro Asp Pro Thr Lys
    50                  55                  60


Tyr Ser Leu Tyr Leu Arg Phe Asn Arg Gln Glu Gln Val Cys Ala His

65                  70                  75                  80

Phe Ala Pro Arg Leu Leu Pro Leu Asp His Tyr Leu Val Asn Phe Thr
                85                  90                  95

Cys Leu Arg Pro Ser Pro Glu Glu Ala Val Ala Gln Ala Glu Ser Glu
            100                 105                 110

Val Gly Arg Pro Glu Glu Glu Glu Ala Glu Ala Ala Ala Gly Leu Glu
        115                 120                 125

Leu Cys Ser Gly Ser Gly Pro Phe Thr Phe Leu His Phe Asp Lys Asn
    130                 135                 140

Phe Val Gln Leu Cys Leu Ser Ala Glu Pro Ser Glu Ala Pro Arg Leu
145                 150                 155                 160

Leu Ala Pro Ala Ala Leu Ala Phe Arg Phe Val Glu Val Leu Leu Ile
                165                 170                 175

Asn Asn Asn Asn Ser Ser Gln Phe Thr Cys Gly Val Leu Cys Arg Trp
            180                 185                 190

Ser Glu Glu Cys Gly Arg Ala Ala Gly Arg Ala Cys Gly Phe Ala Gln
        195                 200                 205

Pro Gly Cys Ser Cys Pro Gly Glu Ala Gly Ala Gly Ser Thr Thr Thr
    210                 215                 220

Thr Ser Pro Gly Pro Pro Ala Ala His Thr Leu Ser Asn Ala Leu Val
225                 230                 235                 240

Pro Gly Gly Pro Ala Pro Pro Ala Glu Ala Asp Leu His Ser Gly Ser
                245                 250                 255

Ser Asn Asp Leu Phe Thr Thr Glu Met Arg Tyr Gly Glu Glu Pro Glu
            260                 265                 270

Glu Glu Pro Lys Val Lys Thr Gln Trp Pro Arg Ser Ala Asp Glu Pro
        275                 280                 285

Gly Leu Tyr Met Ala Gln Thr Gly Asp Pro Ala Ala Glu Glu Trp Ser
    290                 295                 300

Pro Trp Ser Val Cys Ser Leu Thr Cys Gly Gln Gly Leu Gln Val Arg
305                 310                 315                 320

Thr Arg Ser Cys Val Ser Ser Pro Tyr Gly Thr Leu Cys Ser Gly Pro
                325                 330                 335

Leu Arg Glu Thr Arg Pro Cys Asn Asn Ser Ala Thr Cys Pro Val His

340                  345 [?]                  350

Gly Val Trp Glu Glu Trp Gly Ser Trp Ser Leu Cys Ser Arg Ser Cys
        355                 360                 365

Gly Arg Gly Ser Arg Ser Arg Met Arg Thr Cys Val Pro Pro Gln His
    370                 375                 380

Gly Gly Lys Ala Cys Glu Gly Pro Glu Leu Gln Thr Lys Leu Cys Ser
385                 390                 395                 400

Met Ala Ala Cys Pro Val Glu Gly Gln Trp Leu Glu Trp Gly Pro Trp
                405                 410                 415

Gly Pro Cys Ser Thr Ser Cys Ala Asn Gly Thr Gln Gln Arg Ser Arg
            420                 425                 430

Lys Cys Ser Val Ala Gly Pro Ala Trp Ala Thr Cys Thr Gly Ala Leu
        435                 440                 445

Thr Asp Thr Arg Glu Cys Ser Asn Leu Glu Cys Pro Ala Thr Asp Ser
    450                 455                 460

Lys Trp Gly Pro Trp Asn Ala Trp Ser Leu Cys Ser Lys Thr Cys Asp
465                 470                 475                 480

Thr Gly Trp Gln Arg Arg Phe Arg Met Cys Gln Ala Thr Gly Thr Gln
                485                 490                 495

Gly Tyr Pro Cys Glu Gly Thr Gly Glu Glu Val Lys Pro Cys Ser Glu
            500                 505                 510

Lys Arg Cys Pro Ala Phe His Glu Met Cys Arg Asp Glu Tyr Val Met
        515                 520                 525

Leu Met Thr Trp Lys Lys Ala Ala Ala Gly Glu Ile Ile Tyr Asn Lys
    530                 535                 540

Cys Pro Pro Asn Ala Ser Gly Ser Ala Ser Arg Arg Cys Leu Leu Ser
545                 550                 555                 560

Ala Gln Gly Val Ala Tyr Trp Gly Leu Pro Ser Phe Ala Arg Cys Ile
                565                 570                 575

Ser His Glu Tyr Arg Tyr Leu Tyr Leu Ser Leu Arg Glu His Leu Ala
            580                 585                 590

Lys Gly Gln Arg Met Leu Ala Gly Glu Gly Met Ser Gln Val Val Arg
        595                 600                 605

Ser Leu Gln Glu Leu Leu Ala Arg Arg Thr Tyr Tyr Ser Gly Asp Leu

610                 615                 620

Leu Phe Ser Val Asp Ile Leu Arg Asn Val Thr Asp Thr Phe Lys Arg
625                 630                 635                 640

Ala Thr Tyr Val Pro Ser Ala Asp Asp Val Gln Arg Phe Phe Gln Val
                645                 650                 655

Val Ser Phe Met Val Asp Ala Glu Asn Lys Glu Lys Trp Asp Asp Ala
            660                 665                 670

Gln Gln Val Ser Pro Gly Ser Val His Leu Leu Arg Val Val Glu Asp
        675                 680                 685

Phe Ile His Leu Val Gly Asp Ala Leu Lys Ala Phe Gln Ser Ser Leu
    690                 695                 700

Ile Val Thr Asp Asn Leu Val Ile Ser Ile Gln Arg Glu Pro Val Ser
705                 710                 715                 720

Ala Val Ser Ser Asp Ile Thr Phe Pro Met Arg Gly Arg Arg Gly Met
                725                 730                 735

Lys Asp Trp Val Arg His Ser Glu Asp Arg Leu Phe Leu Pro Lys Glu
            740                 745                 750

Val Leu Ser Leu Ser Ser Pro Gly Lys Pro Ala Thr Ser Gly Ala Ala
        755                 760                 765

Gly Ser Pro Gly Arg Gly Arg Gly Pro Gly Thr Val Pro Pro Gly Pro
    770                 775                 780

Gly His Ser His Gln Arg Leu Leu Pro Ala Asp Pro Asp Glu Ser Ser
785                 790                 795                 800

Tyr Phe Val Ile Gly Ala Val Leu Tyr Arg Thr Leu Gly Leu Ile Leu
                805                 810                 815

Pro Pro Pro Arg Pro Pro Leu Ala Val Thr Ser Arg Val Met Thr Val
            820                 825                 830

Thr Val Arg Pro Pro Thr Gln Pro Pro Ala Glu Pro Leu Ile Thr Val
        835                 840                 845

Glu Leu Ser Tyr Ile Ile Asn Gly Thr Thr Asp Pro His Cys Ala Ser
    850                 855                 860

Trp Asp Tyr Ser Arg Ala Asp Ala Ser Ser Gly Asp Trp Asp Thr Glu
865                 870                 875                 880

Asn Cys Gln Thr Leu Glu Thr Gln Ala Ala His Thr Arg Cys Gln Cys

885 890 895

Gln His Leu Ser Thr Phe Ala Val Leu Ala Gln Pro Pro Lys Asp Leu
900 905 910

Thr Leu Glu Leu Ala Gly Ser Pro Ser Val Pro Leu Val Ile Gly Cys
915 920 925

Ala Val Ser Cys Met Ala Leu Leu Thr Leu Leu Ala Ile Tyr Ala Ala
930 935 940

Phe Trp Arg Phe Ile Lys Ser Glu Arg Ser Ile Ile Leu Leu Asn Phe
945 950 955 960

Cys Leu Ser Ile Leu Ala Ser Asn Ile Leu Ile Leu Val Gly Gln Ser
965 970 975

Arg Val Leu Ser Lys Gly Val Cys Thr Met Thr Ala Ala Phe Leu His
980 985 990

Phe Phe Phe Leu Ser Ser Phe Cys Trp Val Leu Thr Glu Ala Trp Gln
995 1000 1005

Ser Tyr Leu Ala Val Ile Gly Arg Met Arg Thr Arg Leu Val Arg
1010 1015 1020

Lys Arg Phe Leu Cys Leu Gly Trp Gly Leu Pro Ala Leu Val Val
1025 1030 1035

Ala Val Ser Val Gly Phe Thr Arg Thr Lys Gly Tyr Gly Thr Ser
1040 1045 1050

Ser Tyr Cys Trp Leu Ser Leu Glu Gly Gly Leu Leu Tyr Ala Phe
1055 1060 1065

Val Gly Pro Ala Ala Val Ile Val Leu Val Asn Met Leu Ile Gly
1070 1075 1080

Ile Ile Val Phe Asn Lys Leu Met Ala Arg Asp Gly Ile Ser Asp
1085 1090 1095

Lys Ser Lys Lys Gln Arg Ala Gly Ser Glu Arg Cys Pro Trp Ala
1100 1105 1110

Ser Leu Leu Leu Pro Cys Ser Ala Cys Gly Ala Val Pro Ser Pro
1115 1120 1125

Leu Leu Ser Ser Ala Ser Ala Arg Asn Ala Met Ala Ser Leu Trp
1130 1135 1140

Ser Ser Cys Val Val Leu Pro Leu Leu Ala Leu Thr Trp Met Ser

1145                    1150                    1155

Ala Val Leu Ala Met Thr Asp Arg Arg Ser Val Leu Phe Gln Ala
    1160                    1165                    1170

Leu Phe Ala Val Phe Asn Ser Ala Gln Gly Phe Val Ile Thr Ala
    1175                    1180                    1185

Val His Cys Phe Leu Arg Arg Glu Val Gln Asp Val Val Lys Cys
    1190                    1195                    1200

Gln Met Gly Val Cys Arg Ala Asp Glu Ser Glu Asp Ser Pro Asp
    1205                    1210                    1215

Ser Cys Lys Asn Gly Gln Leu Gln Ile Leu Ser Asp Phe Glu Lys
    1220                    1225                    1230

Asp Val Asp Leu Ala Cys Gln Thr Val Leu Phe Lys Glu Val Asn
    1235                    1240                    1245

Thr Cys Asn Pro Ser Thr Ile Thr Gly Thr Leu Ser Arg Leu Ser
    1250                    1255                    1260

Leu Asp Glu Asp Glu Glu Pro Lys Ser Cys Leu Val Gly Pro Glu
    1265                    1270                    1275

Gly Ser Leu Ser Phe Ser Pro Leu Pro Gly Asn Ile Leu Val Pro
    1280                    1285                    1290

Met Ala Ala Ser Pro Gly Leu Gly Glu Pro Pro Pro Pro Gln Glu
    1295                    1300                    1305

Ala Asn Pro Val Tyr Met Cys Gly Glu Gly Gly Leu Arg Gln Leu
    1310                    1315                    1320

Asp Leu Thr Trp Leu Arg Pro Thr Glu Pro Gly Ser Glu Gly Asp
    1325                    1330                    1335

Tyr Met Val Leu Pro Arg Arg Thr Leu Ser Leu Gln Pro Gly Gly
    1340                    1345                    1350

Gly Gly Gly Gly Gly Glu Asp Ala Pro Arg Ala Arg Pro Glu Gly
    1355                    1360                    1365

Thr Pro Arg Arg Ala Ala Lys Thr Val Ala His Thr Glu Gly Tyr
    1370                    1375                    1380

Pro Ser Phe Leu Ser Val Asp His Ser Gly Leu Gly Leu Gly Pro
    1385                    1390                    1395

Ala Tyr Gly Ser Leu Gln Asn Pro Tyr Gly Met Thr Phe Gln Pro

1400                    1405                    1410

Pro Pro Pro Thr Pro Ser Ala Arg Gln Val Pro Glu Pro Gly Glu
    1415                    1420                    1425

Arg Ser Arg Thr Met Pro Arg Thr Val Pro Gly Ser Thr Met Lys
    1430                    1435                    1440

Met Gly Ser Leu Glu Arg Lys Lys Leu Arg Tyr Ser Asp Leu Asp
    1445                    1450                    1455

Phe Glu Val Met His Thr Arg Lys Arg His Ser Glu Leu Tyr His
    1460                    1465                    1470

Glu Leu Asn Gln Lys Phe His Thr Phe Asp Arg Tyr Arg Ser Gln
    1475                    1480                    1485

Ser Thr Ala Lys Arg Glu Lys Arg Trp Ser Val Ser Ser Gly Gly
    1490                    1495                    1500

Ala Ala Glu Arg Ser Val Cys Thr Asp Lys Pro Ser Pro Gly Glu
    1505                    1510                    1515

Arg Pro Ser Leu Ser Gln His Arg Arg His Gln Ser Trp Ser Thr
    1520                    1525                    1530

Phe Lys Ser Met Thr Leu Gly Ser Leu Pro Pro Lys Pro Arg Glu
    1535                    1540                    1545

Arg Leu Thr Leu His Arg Ala Ala Ala Trp Glu Pro Thr Glu Pro
    1550                    1555                    1560

Pro Asp Gly Asp Phe Gln Thr Glu Val
    1565                    1570

<210>  23
<211>  21
<212>  DNA
<213>  Artificial

<220>
<223>  Designed oligonucleotide for using as a primer

<400>  23
gacctctttc ctaccatcgc c                                                21

<210>  24
<211>  19
<212>  DNA
<213>  Artificial

<220>
<223>  Designed oligonucleotide for using as a primer

<400>  24

cacaggctcc acgcattcc 19